# EUROPEAN PATENT SPECIFICATION

(11) **EP 1 940 849 B1**
(45) Date of publication and mention of the grant of the patent: **27.01.2010**
(21) Application number: 06807174.5
(22) Date of filing: 12.10.2006
(51) Int. Cl.: C07D 513/04, A01N 43/90

(54) **ISOTHIAZOLOPYRIDIN-3-YLENAMINES FOR COMBATING ANIMAL PESTS**
ISOTHIAZOLOPYRIDIN-3-YLENAMINE ZUR BEKÄMPFUNG TIERISCHER SCHÄDLINGE
COMPOSES D'ISOTHIAZOLOPYRIDINE-3-YLENAMINES POUR COMBATTRE LES PARASITES D'ANIMAUX

(30) Priority: 21.10.2005 US 729076 P
(43) Date of publication of application: 09.07.2008
(73) Proprietor: BASF SE, 67056 Ludwigshafen (DE)
(72) Inventor: KAISER, Florian, 68167 Mannheim (DE); VON DEYN, Wolfgang, 67435 Neustadt (DE); POHLMAN, Matthias, 69115 Heidelberg (DE); BASTIAANS, Henricus Maria Martinus, 61250 Usingen (DE); ANSPAUGH, Douglas D., Apex, NC 27502 (US); CULBERTSON, Deborah L., Fuquay Varina, NC 27526 (US); COTTER, Henry Van Tuyl, Raleigh, NC 27613 (US)
(86) International application number: PCT/EP2006/067306
(87) International publication number: WO 2007/045588

(56) References cited:
- EP-A1- 0 033 984
- EP-A2- 0 133 418
- WO-A-2006/091858
- WO-A-2006/114706
- WO-A1-2005/085216
- DE-A1- 2 365 176
- BORGNA, PIETRO ET AL: "On the reaction between 3H-1,2-dithiolo[3,4-b]pyridine-3-thione and primary alkyl and arylalkylamines" JOURNAL OF HETEROCYCLIC CHEMISTRY , 30(4), 1079-84 CODEN: JHTCAD; ISSN: 0022-152X, 1993, XP002416019
- GREENWOOD, D. ET AL: "Search for a veterinary insecticide . I. Sulfonamides and disulfonamides active against sheep blowfly" JOURNAL OF THE SCIENCE OF FOOD AND AGRICULTURE , 16(6), 293-9 CODEN: JSFAAE; ISSN: 0022-5142, 1965, XP002416018

## Description

The present invention relates to Isothiazolopyridin-3-ylenamine compounds and to the agriculturally useful salts thereof and to compositions comprising such compounds.
The invention also relates to the use of the Isothiazolopyridin-3-ylenamine compounds, of their salts or of compositions comprising them for combating animal pests.

Animal pests destroy growing and harvested crops and attack wooden dwelling and commercial structures, causing large economic loss to the food supply and to property. While a large number of pesticidal agents are known, due to the ability of target pests to develop resistance to said agents, there is an ongoing need for new agents for combating animal pests. In particular, animal pests such as insects and acaridae are difficult to be effectively controlled.

EP-A 33984 discloses iminosaccharines having aphicidal activity. Their activity, however, is not satisfactory. Similar compounds are described in BR 8004671.

It is therefore an object of the present invention to provide compounds having a good pesticidal activity, especially against difficult to control insects and acaridae.

It has been found that these objects are solved by Isothiazolopyridin-3-ylenamine compounds of the general isomeric formulas I. 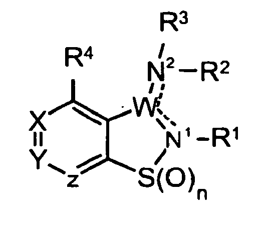 wherein
- n: is 0, 1, or 2
- X: is N, NO or C-R⁵
- Y: is N, NO or C-R⁶
- Z: is N, NO or C-R⁷

With the proviso that one of the variables X, Y, Z at least one and not more than one is an N or NO and the other two variables are optionally substituted carbons.
- W: is C, which is either connected to N¹ with a double bond and to N² with a single bond or connected to N² with a double bond and to N¹ with a single bond.

Isothiazolopyridin-3-ylenamine of the present invention are represented by the following two isomeric formula IA and formula IB:

Isomeric formula IA is represented, if W is connected to N¹ with a single bond and to N² with a double bond, then N² is carrying only the substituent R² and not the substituent R³. In this case, R² is hydrogen, and R¹ is selected from the group consisting of hydrogen, C(=O)-R⁸, C₁-C₁₀-alkyl, C₂-C₆-alkenyl, C₂-C₁₀-alkinyl, C₁-C₁₀-alkoxy or C₃-C₁₀-cycloalkyl, wherein the five last-mentioned radicals may be unsubstituted, partially or fully halogenated and/or may carry 1, 2 or 3 radicals, independently of one another each selected from the group consisting of cyano, nitro, amino, C₁-C₁₀-alkoxy, C₁-C₁₀-alkylthio, C₁-C₁₀-alkylsulfinyl, C₁-C₁₀-alkylsulfonyl, C₁-C₁₀-haloalkoxy, C₁-C₁₀-haloalkylthio, C₁-C₁₀-alkoxycarbonyl, (C₁-C₁₀-alkyl)amino, di-(C₁-C₁₀-alkyl)amino, C₃-C₁₀-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy.

Isomeric formula IB is represented, if W is connected to N² with a single bond and to N¹ with a double bond, then N¹ is not carrying the substituent R¹. In this case, R² and R³ are, independently of one another, selected from the group consisting of hydrogen, C(=O)-R⁸, C₁-C₁₀-alkyl, C₂-C₆-alkenyl, C₂-C₁₀-alkinyl, C₁-C₁₀-alkoxy or C₃-C₁₀-cycloalkyl,
wherein the five last-mentioned radicals may be unsubstituted, partially or fully halogenated and/or may carry 1, 2 or 3 radicals, independently of one another each selected from the group consisting of cyano, nitro, amino, C₁-C₁₀-alkoxy, C₁-C₁₀-alkylthio, C₁-C₁₀-alkylsulfinyl, C₁-C₁₀-alkylsulfonyl, C₁-C₁₀-haloalkoxy, C₁-C₁₀-haloalkylthio, C₁-C₁₀-alkoxycarbonyl, (C₁-C₁₀-alkyl)amino, di-(C₁-C₁₀-alkyl)amino, C₃-C₁₀-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy; or
R² and R³ together with the adjacent nitrogen form a 3 to 10-membered ring, preferably a 3 to 6-membered ring, optionally substituted by 1, 2 or 3 C₁-C₅-alkyl radicals, wherein the ring may contain, in addition to the nitrogen and carbon ring members, 1, 2 or 3 heteroatoms as ring members selected from the group consisting of nitrogen, oxygen, sulfur, a group SO, SO₂ or N-R⁹;
R⁴ is hydrogen, nitro, cyano, azido, amino, halogen, sulfonylamino, sulfenylamino, sulfinylamino, C(=O)R¹⁰, C₁-C₆-alkyl, C₂-C₆-alkenyl, C₂-C₆-alkinyl, C₃-C₈-cycloalkyl, C₁-C₆-alkoxy, C₁-C₆-alkylthio, (C₁-C₆-alkyl)amino, di(C₁-C₆-alkyl)amino, C₁-C₆-alkylsulfinyl, C₁-C₆-alkylsulfenyl or C₁-C₆-alkylsulfonyl, wherein the eleven last-mentioned radicals may be unsubstituted, partially or fully halogenated and/or may carry 1, 2 or 3 radicals, selected from the group consisting of cyano, nitro, amino, C₁-C₄-alkoxy, C₁-C₄-alkylthio, C₁-C₄-alkylsulfinyl, C₁-C₄-alkylsulfonyl, C₁-C₄-haloalkoxy, C₁-C₄-haloalkylthio, (C₁-C₄-alkoxy)carbonyl, (C₁-C₄-alkyl)amino, di(C₁-C₄-alkyl)amino, C₃-C₈-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy;
R⁵, R⁶ and R⁷ are independently of one another selected from the group consisting of hydrogen, halogen, cyano, azido, nitro, C₁-C₆-alkyl, C₃-C₈-cycloalkyl, C₁-C₄-haloalkyl, C₁-C₄-alkoxy, C₁-C₄-alkylthio, C₁-C₄-alkylsulfinyl, C₁-C₄-alkylsulfonyl, C₁-C₄-haloalkoxy, C₁-C₄-haloalkylthio, C₂-C₆-alkenyl, C₂-C₆-alkinyl, (C₁-C₄-alkoxy)carbonyl, amino, (C₁-C₄-alkyl)amino, di(C₁-C₄-alkyl)amino, aminocarbonyl, (C₁-C₄-alkyl)aminocarbonyl, di(C₁-C₄-alkyl)aminocarbonyl, sulfonyl, sulfonylamino, sulfenylamino, sulfanylamino and C(=O)-R¹¹;
R⁸ is C₁-C₆-alkyl, aryl, aryl-C₁-C₄-alkyl,
3- to 7-membered heteroaryl or heteroaryl-C₁-C₄-alkyl, wherein the heteroaryl ring contains as ring members 1,2 or 3 heteroatoms, selected from the group consisting of nitrogen, oxygen, sulfur, a group SO, SO₂ or N-R¹², wherein R¹² is hydrogen or C₁-C₄-alkyl;
3- to 7-membered heterocyclyl or heterocyclyl-C₁-C₄-alkyl, wherein the heterocyclic ring contains as ring members 1, 2 or 3 heteroatoms, selected from the group consisting of nitrogen, oxygen, sulfur, a group SO, SO₂ or N-R¹³, wherein R¹³ is hydrogen or C₁-C₄-alkyl;
and wherein the carbon atoms of the heterocyclic rings may by unsubstituted or substituted by 1 or 2 C₁-C₄-alkyl groups;
R⁹ is hydrogen or C₁-C₄-alkyl
R¹⁰ and R¹¹, independently of one another, are hydrogen, hydroxy, C₁-C₆-alkoxy, amino, C₁-C₄-alkyl, aryl, aryl-C₁-C₄-alkyl,
3- to 7-membered heteroaryl or heteroaryl-C₁-C₄-alkyl, wherein the heteroaryl ring contains as ring members 1, 2 or 3 heteroatoms, selected from the group consisting of nitrogen, oxygen, sulfur, a group SO, SO₂ or N-R¹⁴, wherein R¹⁴ is hydrogen or C₁-C₄-alkyl;
3- to 7-membered heterocyclyl or heterocyclyl C₁-C₄-alkyl, wherein the heterocyclic ring contains 1, 2 or 3 heteroatoms, selected from the group consisting of nitrogen, oxygen, sulfur, a group SO, SO₂ or N-R¹⁵, wherein R¹⁵ is hydrogen or C₁-C₄-alkyl;
and wherein the carbon atoms of the heterocyclic rings may by unsubstituted or substituted by 1 or 2 C₁-C₄-alkyl groups;
and/or the agriculturally acceptable salts thereof.

The compounds of the formula I and their agriculturally acceptable salts are highly active against animal pest, i.e. harmful arthropodes and nematodes, especially against difficult to control insects and acaridae.

Accordingly, the present invention relates to Isothiazolopyridin-3-ylenamine compounds of the general formula I and to their agriculturally useful salts.

Moreover, the present invention relates to:
- Agricultural compositions comprising such an amount of at least one Isothiazolopyridin-3-yienamine compounds derivative of the formula I and/or at least one agriculturally useful salt of I and at least one inert liquid and/or solid agronomically acceptable carrier that it has a pesticidal action and, if desired, at least one surfactant;
- The use of compounds I and/or their salts for combating animal pests; and
- A method of combating animal pests which comprises contacting the animal pests, their habit, breeding ground, food supply, plant, seed, soil, area, material or environment in which the animal pests are growing or may grow, or the materials, plants, seeds, soils, surfaces or spaces to be protected from animal attack or infestation with a pesticidally effective amount of at least one compound of the formula I and/or at least one agriculturally acceptable salt thereof, as defined herein, and to
- A method for protecting crops from attack or infestation by animal pests, which comprises contacting a crop with a pesticidally effective amount of at least one compound of the formula I and/or at least one salt thereof.
- A method for the protection of seeds from soil insects and of the seedlings' roots and shoots from insects comprising contacting the seeds before sowing and/or after pregermination with a compound of the formula I and/or at least one agriculturally acceptable salt thereof.

In the substituents R¹ to R¹⁵ the compounds of the general formula I may have one or more centers of chirality, in which case they are present as mixtures of enantiomers or diastereomers. The present invention provides both the pure enantiomes or diastereomers or mixtures thereof. The compounds of the general formula I may also exist in the form of different tautomers if R⁵, R⁶ or R⁷ are amino. The invention comprises the single tautomers, if seperable, as well as the tautomer mixtures.

Salts of the compounds of the formula I are preferably agriculturally and/or veterinarily acceptable salts. They can be formed in a customary method, e.g. by reacting the compound with an acid of the anion in question if the compound of formula I has a basic functionality or by reacting an acidic compound of formula I with a suitable base.

Suitable agriculturally useful salts are especially the salts of those cations or the acid addition salts of those acids whose cations and anions, respectively, do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethyl-ammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting the compounds of the formulae Ia and Ib with an acid of the corresponding anion, preferably of hydrochloric acid, hydrobromic acid, sulfuric acid, phosphoric acid or nitric acid.

By the term "veterinarily acceptable salts" is meant salts the anions of which are known and accepted in the art for the formation of salts for veterinary use. Suitable acid addition salts, e.g. formed by compounds of formula I containing a basic nitrogen atom, e.g. an amino group, include salts with inorganic acids, for example hydrochlorids, sulphates, phosphates, and nitrates and salts of organic acids for example acetic acid, maleic acid, dimaleic acid, fumaric acid, difumaric acid, methane sulfenic acid, methane sulfonic acid, and succinic acid.

The organic moieties mentioned in the above definitions of the variables are - like the term halogen - collective terms for individual listings of the individual group members.

The prefix Cₙ-Cₘ indicates in each case the possible number of carbon atoms in the group.

"Halogen" will be taken to mean fluoro, chloro, bromo and iodo, in particular fluoro and chloro.

The term "partially or fully halogenated" will be taken to mean that 1 or more, e.g. 1, 2, 3, 4 or 5 or all of the hydrogen atoms of a given radical have been replaced by a halogen atom, in particular by fluorine or chlorine.

The term "Cₙ-Cₘ-alkyl" as used herein (and also in Cₙ-Cₘ-alkylamino, di-Cₙ-Cₘ-alkylamino, Cₙ-Cₘ-alkylaminocarbonyl, di-(Cₙ-Cₘ-alkylamino)carbonyl, Cₙ-Cₘ-alkylthio, Cₙ-Cₘ-alkylsulfinyl and Cₙ-Cₘ-alkylsulfonyl) refers to a branched or unbranched saturated hydrocarbon group having n to m, e.g. 1 to 10 carbon atoms, preferably 1 to 6 carbon atoms, for example methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

The term "Cₙ-Cₘ-haloalkyl" as used herein (and also in Cₙ-Cₘ-haloalkylsulfinyl and Cₙ-Cₘ-haloalkylsulfonyl) refers to a straight-chain or branched alkyl group having n to m carbon atoms, e.g. 1 to 10 in particular 1 to 6 carbon atoms (as mentioned above), where some or all of the hydrogen atoms in these groups may be replaced by halogen atoms as mentioned above, for example C₁-C₄-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like. The term C₁-C₁₀-haloalkyl in particular comprises C₁-C₂-fluoroalkyl, which is synonym with methyl or ethyl,
wherein 1, 2, 3, 4 or 5 hydrogen atoms are substituted by fluorine atoms, such as fluoromethyl, difluoromethyl, trifluoromethyl, 1-fluoroethyl, 2-fluoroethyl, 2,2-difluoroethyl, 2,2,2-trifluoroethyl and pentafluoromethyl.

Similarly, "Cₙ-Cₘ-alkoxy" and "Cₙ-Cₘ-alkylthio" (or Cₙ-Cₘ-alkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen or sulfur linkages, respectively, at any bond in the alkyl group. Examples include C₁-C₄-alkoxy such as methoxy, ethoxy, propoxy, isopropoxy, butoxy, sec-butoxy, isobutoxy and tert-butoxy, futher C₁-C₄-alkylthio such as methylthio, ethylthio, propylthio, isopropylthio, and n-butylthio.

Accordingly, the terms "Cₙ-Cₘ-haloalkoxy" and "Cₙ-Cₘ-haloalkylthio" (or Cₙ-Cₘ-haloalkylsulfenyl, respectively) refer to straight-chain or branched alkyl groups having n to m carbon atoms, e.g. 1 to 10, in particular 1 to 6 or 1 to 4 carbon atoms (as mentioned above) bonded through oxygen or sulfur linkages, respectively, at any bond in the alkyl group, where some or all of the hydrogen atoms in these groups may be replaced by halogen atoms as mentioned above, for example C₁-C₂-haloalkoxy, such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy and pentafluoroethoxy, further C₁-C₂-haloalkylthio, such as chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like. Similarly the terms C₁-C₂-fluoroalkoxy and C₁-C₂-fluoroalkylthio refer to C₁-C₂-fluoroalkyl which is bound to the remainder of the molecule via an oxygen atom or a sulfur atom, respectively.

The term "C₂-Cₘ-alkenyl" as used herein intends a branched or unbranched unsaturated hydrocarbon group having 2 to m, e.g. 2 to 10 or 2 to 6 carbon atoms and a double bond in any position, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "C₂-Cₘ-alkynyl" as used herein refers to a branched or unbranched unsaturated hydrocarbon group having 2 to m, e.g. 2 to 10 or 2 to 6 carbon atoms and containing at least one triple bond, such as ethynyl, propynyl, 1-butynyl, 2-butynyl, and the like.

The term "C₁-C₄-alkoxy-C₁-C₄-alkyl" as used herein refers to alkyl having 1 to 4 carbon atoms, wherein a hydrogen atom of the alkyl radical is replaced by a C₁-C₄-alkoxy group.

The term "C₁-C₄-alkylthio-C₁-C₄-alkyl" as used herein refers to alkyl having 1 to 4 carbon atoms, wherein a hydrogen atom of the alkyl radical is replaced by a C₁-C₄-alkylthio group.

The term "C₃-Cₘ-cycloalkyl" as used herein refers to a monocyclic 3- to m-membered saturated cycloaliphatic radicals, e.g. cyclopropyl, cyclobutyl, cyclopentyl, cyclohexyl, cycloheptyl, cyclooctyl and cyclodecyl.

The term "aryl" as used herein refers to an aromatic hydrocarbon radical such as naphthyl or in particular phenyl.

The term "aryl-C₁-C₄-alkyl" as used herein refers to an aromatic hydrocarbon radical, which is bound to the remainder of the molecule via a C₁-C₄-alkylene group, examples comprise benzyl, 1-phenylethyl or 2-phenylethyl.

The term "3- to 7-membered heterocyclyl" as used herein (and also in "heterocyclyl-C₁-C₄-alkyl") refers to a saturated or partially unsaturated non-aromatic heterocyclic radical havin 3 to 7 ring members, wherein 1, 2 or 3 ring members are heteroatoms selected from O, N and S or heteroatom groups, selected from S=O, S(O)₂ or N-R with R being H or alkyl. Examples for non-aromatic rings include azetidiyl, pyrrolidinyl, pyrazolinyl, imidazolinyl, pyrrolinyl, pyrazolinyl, imidazolinyl, tetrahydrofuranyl, dihydrofuranyl, 1,3-dioxolanyl, dioxolenyl, thiolanyl, dihydrothienyl, oxazolidinyl, isoxazolidinyl, oxazolinyl, isoxazolinyl, thiazolinyl, isothiazolinyl, thiazolidinyl, isothiazolidinyl, oxathiolanyl, piperidinyl, piperazinyl, pyranyl, dihydropyranyl, tetrahydropyranyl, dioxanyl, thiopyranyl, dihydrothiopyranyl, tetrahydrothiopyranyl, morpholinyl, thiazinyl and the like.

The term "3- to 7-membered heteroaryl" as used herein (and also in "heteroaryl-C₁-C₄-alkyl") refers to an aromatic heterocyclic radical having 3 to 7 ring members, wherein 1, 2 or 3 ring members are heteroatoms selected from O, N and S or heteroatom groups, selected from S=O, S(O)₂ or N-R with R being H or alkyl. Examples for monocyclic 3-to 7-membered heteroaromatic rings include triazinyl, pyrazinyl, pyrimidyl, pyridazinyl, pyridyl, thienyl, furyl, pyrrolyl, pyrazolyl, imidazolyl, triazolyl, tetrazolyl, thiazolyl, oxazolyl, thiadiazolyl, oxadiazolyl, isothiazolyl and isoxazolyl.

The terms "heterocyclyl-C₁-C₄-alkyl" and "heteroaryl-C₁-C₄-alkyl" as used herein refer to an aromatic or non-aromatic heterocyclic radical, which is bound to the remainder of the molecule via a C₁-C₄-alkylene group.

With respect to the use according to the invention of the compounds of formula I, particular preference is given to the following meanings of the substituents and variables, in each case on their own or in combination:
A preferred embodiment of the invention relates to isomeric form IA of 4-Isothiazolopyridin-3-ylenamine compounds of the formula I wherein W is connected to N¹ with a single bond and to N² with a double bond. Then N² is carrying only the substituent R² and not the substituent R³. In this case, R² is hydrogen, and R¹ is preferably selected from the group consisting of hydrogen, C₁-C₆-alkyl, C₁-C₆-haloalkyl, C₂-C₆-alkenyl, C₂-C₆-haloalkenyl, C₂-C₆-alkinyl, C₂-C₆-haloalkinyl, C₃-C₆-cycloalkyl, C₁-C₄-alkoxy-C₁-C₄-alkyl and C₁-C₄-alkylthio-C₁-C₄-alkyl. In particular R¹ is radical having 1 to 6 and more preferably 1 to 4 carbon atoms. However R¹ may also form a radical C(=O)-R⁸, wherein R⁸ is as defined herein, in particular R⁸ being C₁-C₄-alkyl or C₁-haloalkyl.
More preferably R¹ is selected from the group consisting of hydrogen, C₁-C₄-alkyl, C₁-C₄-haloalkyl, C₂-C₄-alkinyl, cyclopropyl, C₁-C₃-alkoxy-C₁-C₃-alkyl, in particular 2-methoxyethyl, C₁-C₃-alkylthio-C₁-C₃-alkyl, in particular 2-methylthioethyl, and C(=O)-R⁸, wherein R⁸ is selected from C₁-C₄-alkyl, in particular methyl ethyl, 1-methylethyl or 2-methylpropan-2-yl, and C₁-haloalkyl, in particular trifluoromethyl or trichlormethyl. More preferably R¹ is selected from hydrogen, C₁-C₄-alkyl, in particular methyl, ethyl, or 1-methylethyl, and C₂-C₄-alkinyl, in particular prop-3-yn-1-yl.

A preferred embodiment of the invention relates to isomeric form IB of 4-Isothiazolopyridin-3-ylenamine compounds of the formula I wherein W is connected to N² with a single bond and to N¹ with a double bond. Then N¹ is not carrying the substituent R¹. In this case, R² and R³ are, independently of one another, selected from the group consisting of hydrogen, C₁-C₆-alkyl, C₁-C₆-haloalkyl, C₂-C₆-alkenyl, C₂-C₆-haloalkenyl, C₂-C₆-alkinyl, C₂-C₆-haloalkinyl, C₃-C₆-cycloalkyl, C₁-C₄-alkoxy-C₁-C₄-alkyl and C₁-C₄-alkylthio-C₁-C₄-alkyl. However R² may also form a radical C(=O)-R⁸, wherein R⁸ is as defined herein, in particular R⁸ being C₁-C₄-alkyl or C₁-haloalkyl.

More preferably R² is selected from the group consisting of hydrogen, C₁-C₄-alkyl, in particular methyl, ethyl, or 1-methylethyl, cyclopropyl, C₂-C₄-alkinyl, in particular prop-3-yn-1-yl, and C(=O)-R⁸, wherein R⁸ is selected from C₁-C₄-alkyl, in particular methyl, ethyl, 1-methylethyl or 2-methylpropan-2-yl, C₁-C₃-alkoxy-C₁-C₃-alkyl, in particular 2-methoxyethyl, C₁-C₃-alkylthio-C₁-C₃-alkyl, in particular 2-methylthioethyl, and C₁-C₄-haloalkyl, in particular trifluoromethyl or trichlormethyl.

More preferably R² and R³ are, independently of one another, selected from the group consisting of hydrogen, C₁-C₄-alkyl, in particular methyl, ethyl, or 1-methylethyl, cyclopropyl, C₂-C₄-alkinyl, in particular prop-3-yn-1-yl, and C(=O)-R⁸, wherein R⁸ is selected from C₁-C₄-alkyl, in particular methyl or ethyl,

R⁴ is preferably selected from halogen, C₁-C₄-alkyl, C₁-C₄-haloalkyl, C₁-C₄-alkoxy or C₁-C₄-haloalkoxy. More preferably R⁴ is selected from C₁-C₂-alkyl, C₁-haloalkyl, C₁-C₂-alkoxy or C₁-haloalkoxy. More paticularly preferably R⁴ is selected from methyl, trifluoromethyl, trichloromethyl, methoxy, trifluoromethoxy, difluoromethoxy, fluoromethoxy or chlorodifluoromethoxy. In another embodiment of the invention R⁴ is halogen, in particular chlorine and fluorine.

R⁵, R⁶, and R⁷ are preferably selected, independently from each other, from hydrogen C₁-C₄-alkyl, C₁-C₄-haloalkyl, C₁-C₄-alkoxy, C₁-C₄-haloalkoxy or halogen. More preferably R⁵, R⁶, and R⁷ are selected, independently from each other, from hydrogen, halogen, C₁-C₄-alkyl, or C₁-C₄-haloalkyl, especially from hydrogen, halogen, C₁-C₂-alkyl, or C₁-haloalkyl, most preferably, from H, Cl, Br, I, CH₃ or CF₃. In a preferred embodiment of the invention one of the radicals R⁵, R⁶ or R⁷ being present, is different from hydrogen, while the other one being present is hydrogen, or both of the radicals R⁵, R⁶ or R⁷ being present are hydrogen.

In formula I the variable n is preferably 2. These compounds are also referred to as compounds Ia:

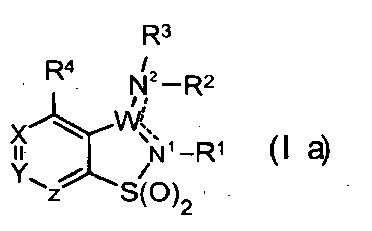

Compounds I, where n is 0 are referred to as compounds Ib, while compounds I, where n is 1 are referred to as compounds Ic,

A preferred embodiment of the invention relates to 4-Isothiazolopyridin-3-ylenamine compounds of the formula I, i.e. compounds of the formula I wherein X is C-R⁵, Y is N or NO and Z is C-R⁷. In this embodiment, preference is given to those compounds,
wherein X is C-R⁵, Y is N, Z is C-R⁷ and n is 2. In this embodiment preference is given to compounds of the formula I, wherein either R⁵ or R⁷ is hydrogen and the other radical R⁵ or R⁷ is selected from halogen, C₁-C₄-alkyl, or C₁-C₄-haloalkyl, in particular form halogen, C₁-C₂-alkyl, or C₁-haloalkyl, most preferably, from Cl, Br, I, CH₃ or CF₃. Likewise preferred are compounds of this embodiment, wherein both R⁵ and R⁷ are hydrogen.

Another embodiment of the invention relates to 3-Isothiazolopyridin-3-ylenamine compounds of the formula I, i.e. compounds of the formula I wherein X is C-R⁵, Y is C-R⁶ and Z is N or NO. In this embodiment, preference is given to those compounds,
wherein X is C-R⁵, Y is C-R⁶ and Z is N and n is 2. In this embodiment preference is given to compounds of the formula I, wherein either R⁵ or R⁶ is hydrogen and the other radical R⁵ or R⁶ is selected from halogen, C₁-C₄-alkyl, or C₁-C₄-haloalkyl, in particular form halogen, C₁-C₂-alkyl, or C₁-haloalkyl, most preferably, from Cl, Br, I, CH₃ or CF₃. Likewise preferred are compounds of this embodiment, wherein both R⁵ and R⁶ are hydrogen.

Examples of preferred compounds of the formula I are given in the following tables 1 to 11268.
The more perferred examples within table 1-11268 are those referring to formula la, wherein n is 2.

Compounds of the formulae Ia, Ib and Ic, wherein each of X and Z are CH, Y is N, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCCIF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X arid Z are CH, Y is N, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCH₂-OCH³ and where according to isomeric form IA R¹ is as defined in one row of table A and R² is hydrogen or where according to isomeric form IB R² and R³ are as defined in one row of table B;
Compounds of the formulae Ia, Ib and Ic, wherein each of X and Z are CH, Y is N, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is CF₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-Cl, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is OCCIF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is OCHCl₂ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-Cl, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Cl, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-Cl, R⁴ is OCH₂CH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-F, R⁴ is CH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-F, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is OCF₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-F, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is OCHF₂ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-F, R⁴ is OCCIF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-F, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is CF³ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is OCClF₂ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-Br, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-Br, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-Br, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is F and
where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-I, R⁴ is Cl and
where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-I, R⁴ is Br and
where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is I and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-I, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is OCClF₂ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-I, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is OCHCl₂ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-I, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-I, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is I and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-NH₂, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCF₃ and where according to isomeric form IA R¹ is as defined in one row of table.A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-NH₂, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is Cl and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-N(CH₃)₂, R⁴ is Br and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-N(CH₃)₂, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is OCCl₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-N(CH₃)₂, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is OCH₂F and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-N(CH₃)₂, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂I, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-N(CH₃)₂, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-N(CH₃)₂, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is I and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CH₃, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is OCF₃ and where according to isomeric form IA R¹ is as defined in one row of table A and R² is hydrogen or where according to isomeric form IB R² and R³ are as defined in one row ,of table B;
Compounds of the formulae Ia, Ib and Ic, wherein X is CH, Y is N, Z is C-CH₃, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is OCClF₂ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CH₃, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CH₃, R⁴ is OCH₂Cl and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CH₃, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CH₃, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is CH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CF₃, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is CCl₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CF₃, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is I and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CF₃, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is . OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CF₃, R⁴ is OCH₂Cl and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CF₃, R⁴ is . OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CF₃, R⁴ is OCH₂CH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-OCH₃, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is C₂H₅ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-OCH₃, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is Cl and where according to isomeric form IA R¹ is as defined in one row of table A an R² is hydrogen or where according to isomeric form IB R² and R³ are as defined in one row of table B;
Compounds of the formulae Ia, Ib and Ic, wherein X is CH, Y is N, Z is C-OCH₃, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is OCHF₂ and where according to isomeric form IA R¹ is as defined in one row of table A and R² is hydrogen or where according to isomeric form IB R² and R₃ are as defined in one row of table B;
Compounds of the formulae Ia, Ib and Ic, wherein X is CH, Y is N, Z is C-OCH₃, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OCH₃, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, **I**b and Ic, wherein X is CH, Y is N, Z is C-OCH₃, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CCl₃, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CCl₃, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CCl₃, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CCl₃, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CCl₃, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N; Z is C-CCl₃, R⁴ is Cl and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-CCl₃, R⁴ is Br and where according to isomeric form IA R¹ is as defined in one row of table A and R² is hydrogen or where according to isomeric form IB R² and R³ are as defined in one row of table B;
Compounds of the formulae la, lb and Ic, wherein X is CH, Y is N, Z is C-CCl₃, R⁴ is I and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-CCl₃, R⁴ is OCH₃ and where according to isomeric form IA R¹ is as defined in one row of table A and R² is hydrogen or where according to isomeric form IB R² and R³ are as defined in one row of table B;
Compounds of the formulae la, lb and Ic, wherein X is CH, Y is N, Z is C-CCl₃, R⁴ is OCF₃ and where according to isomeric form IA R¹ is as defined in one row of table. A and R² is hydrogen or where according to isomeric form IB R² and R³ are as defined in one row of table B;
Compounds of the formulae la, lb and Ic, wherein X is CH, Y is N, Z is C-CCl₃, R⁴ is OCCl₃ and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-CCl₃, R⁴ is OCHF₂ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-CCl₃, R⁴ is OCCIF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CCl₃, R⁴ is OCH₂F and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-CCl₃, R⁴ is OCHCl₂ and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-CCl₃, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-CCl₃, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH; Y is N, Z is C-CCl₃, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and lc, wherein X is CH, Y is N, Z is C-C₂H₅, R⁴ is CH₃ and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-C₂H₅, R⁴ is C₂H₅ and where according to isomeric form IA R¹ is as defined in one row of table A and R² is hydrogen or where according to isomeric form IB R² and R³ are as defined in one row of table B;
Compounds of the formulae la, Ib and lc, wherein X is CH, Y is N, Z is C-C₂H₅; R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is F and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-C₂H₅, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is I and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-C₂H₅, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is OCCl₃ and where according to isomeric form IA R¹ is as defined in one row of table A and R² is hydrogen or where according to isomeric form IB R² and R³ are as defined in one row of table B;
Compounds of the formulae la, lb and Ic, wherein X is CH, Y is N, Z is C-C₂H₅, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is OCCIF₂ and where according to isomeric form IA R¹ is as defined in one row of table A and R² is hydrogen or where according to isomeric form IB R² and R³ are as defined in one row of table B;
Compounds of the formulae la, Ib and Ic, wherein X is CH, Y is N, Z is C-C₂H₅, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴. is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is OCH₂-OCH³ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-C₂H₅, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is CH₃ and where according to isomeric form IA R¹ is as defined in one row of table A and R² is hydrogen or where according to isomeric form IB R² and R³ are as defined in one row of table B;
Compounds of the formulae la, Ib and lc, wherein X is CH, Y is N, Z is C-OC₂H₅, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is CCl₃ and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-OC₂H₅, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is Cl and where according to isomeric form IA R¹ is as defined in one row of table A and R² is hydrogen or where according to isomeric form IB R² and R³ are as defined in one row of table B;
Compounds of the formulae la, Ib and lc, wherein X is CH, Y is N, Z is C-OC₂H₅, R⁴ is Br and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-OC₂H₅, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is OCHF₂ and where according to isomeric form IA R¹ is as defined in one row of table A and R² is hydrogen or where according to isomeric form IB R² and R³ are as defined in one row of table B;
Compounds of the formulae la, lb and lc, wherein X is CH, Y is N, Z is C-OC₂H₅, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is OCH₂F and where according to isomeric form IA R¹ is as defined in one row of table A and R² is hydrogen or where according to isomeric form IB R² and R³ are as defined in one row of table B;
Compounds of the formulae Ia, lb and lc, wherein X is CH, Y is N, Z is C-OC₂H₅, R⁴ is OCHCl₂ and where according to isomeric form IA R¹ is as defined in one row of table A and R² is hydrogen or where according to isomeric form IB R² and R³ are as defined in one row of table B;
Compounds of the formulae la, lb and Ic, wherein X is CH, Y is N, Z is C-OC₂H₅, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein X is CH, Y is N, Z is C-OC₂H₅, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and lc, wherein X is CH, Y is N, Z is OC-C₂H₅, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is F and where according to isomeric form IA R¹ is as defined in one row of table A and R² is hydrogen or where according to isomeric form IB R² and R³ are as defined in one row of table B;
Compounds of the formulae Ia, lb and Ic, wherein Z is CH, Y is N, X is C-Cl, R⁴ is Cl and where according to isomeric form IA R¹ is as defined in one row of table A and R² is hydrogen or where according to isomeric form IB R² and R³ are as defined in one row of table B;
Compounds of the formulae la, lb and lc, wherein Z is CH, Y is N, X is C-Cl, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is I and where according to isomeric form IA R¹ is as defined in one row of table A and R² is hydrogen or where according to isomeric form IB R² and R³ are as defined in one row of table B;
Compounds of the formulae la, Ib and lc, wherein Z is CH, Y is N, X is C-Cl, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Cl, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and lc, wherein Z is CH, Y is N, X is C-Cl, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is CH₃ and where according to isomeric form IA R¹ is as defined in one row of table A and R² is hydrogen or where according to isomeric form IB R² and R³ are as defined in one row of table B;
Compounds of the formulae Ia, lb and lc, wherein Z is CH, Y is N, X is C-F, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is. Cl and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-F, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-F, R⁴ is OCH₂CH₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-Br, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCCl₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-Br, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCH₂-OCH³ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-Br, R⁴ is OCH₂CH₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-I, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is CF³ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is CCl³ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-I, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is Br and
where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-I, R⁴ is I and
where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-I, R⁴ is OCH³ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-I, R⁴ is OCH₂Cl and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-I, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-I, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is C₂H₅ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-NH₂, R⁴ is CF₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-NH₂, R⁴ is CCl₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-NH₂, R⁴ is F and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-NH₂, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is OCHF₂ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-NH₂, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is OCH₂F and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-NH₂, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-NH₂, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-NH₂, R⁴ is OCH₂CH³ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is C₂H₅ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-N(CH₃)₂, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is Cl and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-N(CH₃)₂, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is I and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-N(CH₃)₂, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is.N, X is C-N(CH₃)₂, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂l, R⁴ is OCH₂Cl and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-N(CH₃)₂, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-N(CH₃)₂, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is Cl and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-CH₃, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z ,is CH, Y is N, X is C-CH₃, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CH₃, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is CH₃ and where according to isomeric form IA R¹ is as defined in one row of table A and
R² is hydrogen or where according to isomeric form IB R² and R³ are as defined in one row of table B;
Compounds of the formulae Ia, Ib and Ic, wherein Z is CH, Y is N, X is C-CF₃, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is F and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-CF₃, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is Br and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-CF₃, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CF₃, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is CH₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OCH₃, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is CF₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OCH₃, R⁴ is CCl₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OCH₃, R⁴ is F and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OCH₃, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is Br and where according to isomeric form IA R¹ is as defined in one row of table A and R² is hydrogen or where according to isomeric form IB R² and R³ are as defined in one row of table B; .
Compounds of the formulae Ia, Ib and Ic, wherein Z is CH, Y is N, X is C-OCH₃, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCF₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OCH₃, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OCH₃, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is CF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is I and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCF₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCH₂F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-CCl₃, R⁴ is OCH₂CH₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-C₂H₅, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is C₂H₅ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is CF₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-C₂H₅, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is Cl and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-C₂H₅, R⁴ is Br and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-C₂H₅, R⁴ is I and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-C₂H₅, R⁴ is OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-C₂H₅, R⁴ is OCF₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-C₂H₅, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is OCH₂F and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-C₂H₅, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is OCH₂Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-C₂H₅, R⁴ is OCH₂CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is CH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is C₂H₅ and where according to isomeric form IA R¹ is as defined in one row of table A and R² is hydrogen or where according to isomeric form IB R² and R³ are as defined in one row of table B; Compounds of the formulae Ia, Ib and Ic, wherein Z is CH, Y is N, X is C-OC₂H₅, R⁴ is CF₃ and where according to isomeric form IA R¹ is as defined in one row of table A and R² is hydrogen or where according to isomeric form IB R^{2'} and R³ are as defined in one row of table B;
Compounds of the formulae Ia, Ib and Ic, wherein Z is CH, Y is N, X is C-OC₂H₅, R⁴ is CCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is F and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is Cl and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is Br and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is I and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OC₂H₅, R⁴ is OCH₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OC₂H₅, R⁴ is OCF₃ and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OC₂H₅, R⁴ is OCCl₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is OCHF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is OCClF₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is OCH₂F and where according to isomeric form IA R¹ is as defined in one row of table A and R² is hydrogen or where according to isomeric form IB R² and R³ are as defined in one row of table B;
Compounds of the formulae la, Ib and Ic, wherein Z is CH, Y is N, X is C-OC₂H₅, R⁴ is OCHCl₂ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is OCH₂Cl and where according to isomeric form IA R¹ is as defined in one.row of table A and R² is hydrogen or where according to isomeric form IB R² and R³ are as defined in one row of table B;
Compounds of the formulae Ia, Ib and Ic, wherein Z is CH, Y is N, X is C-OC₂H₅, R⁴ is OCH₂-OCH₃ and where according to isomeric form IA R¹ is as defined in one row of table A and R² is hydrogen or where according to isomeric form IB R² and R³ are as defined in one row of table B;
Compounds of the formulae Ia, Ib and Ic, wherein Z is CH, Y is N, X is OC-C₂H₅, R⁴ is OCH₂CH₃ and where according to isomeric form IA R¹ is as defined in one row of table A and R² is hydrogen or where according to isomeric form IB R² and R³ are as defined in one row of table B;

Tables 451 to 666: Compounds of the formulae Ia, Ib and Ic, wherein X is C-Cl, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 667 to 882: Compounds of the formulae Ia, Ib and Ic, wherein X is C-Br, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 883 to 1098: Compounds of the formulae Ia, Ib and Ic, wherein X is C-F, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 1099 to 1314: Compounds of the formulae Ia, Ib and Ic, wherein X is C-I, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 1315 to 1530: Compounds of the formulae Ia, Ib and Ic, wherein X is C-NH₂, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 1531 to 1746: Compounds of the formulae Ia, Ib and Ic, wherein X is C-N(CH₃)₂, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 1747 to 1962: Compounds of the formulae Ia, Ib and Ic, wherein X is C-CH₃, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 1963 to 2178: Compounds of the formulae Ia, Ib and Ic, wherein X is C-C₂H₅, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 2179 to 2394: Compounds of the formulae Ia, Ib and Ic, wherein X is C-OCH₃, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 2395 to 2610: Compounds of the formulae la, Ib and Ic, wherein X is C-OC₂H₅, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 2611 to 2826: Compounds of the formulae Ia, Ib and Ic, wherein X is C-CF₃, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 2827 to 3042: Compounds of the formulae Ia, Ib and Ic, wherein X is C-CCl₃, where according to isomeric form IA R¹ is as defined in one row of table A and R² is hydrogen or where according to isomeric form IB R² and R³ are as defined in one row of table B and wherein Y, Z and R⁴ correspond to those given in tables 19 to 234.

Tables 3043 to 3258: Compounds of the formulae Ia, Ib and Ic, wherein Z is C-Cl, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 3259 to 3474: Compounds of the formulae la, Ib and Ic, wherein Z is C-Br, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 3475 to 3690: Compounds of the formulae la, Ib and Ic, wherein Z is C-F, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 3691 to 3906: Compounds of the formulae Ia, Ib and Ic, wherein Z is C-I, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 3907 to 4122: Compounds of the formulae Ia, Ib and Ic, wherein Z is C-NH₂, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 4123 to 4338: Compounds of the formulae la, Ib and Ic, wherein Z is C-N(CH₃)₂, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 4339 to 4554: Compounds of the formulae Ia, Ib and Ic, wherein Z is C-CH₃, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 4555 to 4770: Compounds of the formulae Ia, Ib and Ic, wherein Z is C-C₂H₅, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 4771 to 4986: Compounds of the formulae Ia, Ib and Ic, wherein Z is C-OCH₃, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 4987 to 5202: Compounds of the formulae la, Ib and Ic, wherein Z is C-OC₂H₅, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 5203 to 5418: Compounds of the formulae la, Ib and Ic, wherein Z is C-CF₃, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 5419 to 5634: Compounds of the formulae la, Ib and Ic, wherein Z is C-CCl₃, where according to isomeric form IA R¹ is as defined in one row of table A and R² is hydrogen or where according to isomeric form IB R² and R³ are as defined in one row of table B and wherein X, Y and R⁴ correspond to those given in tables 235 to 450.

Tables 5635 to 11268: Compounds of the formulae Ia, Ib and Ic, wherein Y is N-O, where according to isomeric form IA R¹ is as defined in one row of table A and R² is hydrogen or where according to isomeric form IB R² and R³ are as defined in one row of table B and wherein X, Z and R⁴ correspond to those given in tables 1 to 5634.

Isomeric formula IA is represented, if W is connected to N¹ with a single bond and to N² with a double bond, then N² is carrying only the substituent R² and not the substituent R³. In this case:

**Table A referring to isomeric form IA of formula I:**

| N° | R¹ | R² |
|---|---|---|
| C.IA.1. | H | H |
| C.IA.2. | CH₃ | H |
| C.IA.3. | CH₃CH₂- | H |
| C.IA.4. | (CH₃)₂CH- | H |
| C.IA.5. | CH₃CH₂CH₂- | H |
| C.IA.6. | n-C₄H₉ | H |
| C.IA.7. | (CH₃)₃C- | H |
| C.IA.8. | (CH₃)₂CH-CH₂- | H |
| C.IA.9. | n-C₅H₁₁ | H |
| C.IA.10. | (CH₃)₂CH-CH₂-CH₂- | H |
| C.IA.11. | (C₂H₅)₂-CH- | H |
| C.IA.12. | (CH₃)₃C-CH₂- | H |
| C.IA.13. | (CH₃)₃C-CH₂-CH₂- | H |
| C.IA.14. | C₂H₅CH(CH₃)-CH₂- | H |
| C.IA.15. | CH₃-CH₂-C(CH₃)₂- | H |
| C.IA.16. | (CH₃)₂CH-CH(CH₃)- | H |
| C.IA.17. | (CH₃)₃C-CH(CH₃)- | H |
| C.IA.18. | (CH₃)₂CH-CH₂-CH(CH₃)- | H |
| C.IA.19. | CH₃-CH₂-C(CH₃)(C₂H₅)- | H |
| C.IA.20. | CH₃-CH₂-CH₂-C(CH₃)₂- | H |
| C.IA.21. | C₂H₅-CH₂-CH(CH₃)-CH₂- | H |
| C.IA.22. | Cyclopropyl | H |
| C.IA.23. | cyclopropyl-CH₂- | H |
| C.IA.24. | cyclopropyl-CH(CH₃)- | H |
| C.IA.25. | Cyclobutyl | H |
| C.IA.26. | Cyclopentyl | H |
| C.IA.27. | Cyclohexyl | H |
| C.IA.28. | HC≡ C-CH₂- | H |
| C.IA.29. | HC≡ C-CH(CH₃)- | H |
| C.IA.30. | HC≡ C-C(CH₃)₂- | H |
| C.IA.31. | HC≡ C-C(CH₃)(C₂H₅)- | H |
| C.IA.32. | HC≡ C-C(CH₃)(C₃H₇)- | H |
| C.IA.33. | CH₂=CH-CH₂- | H |
| C.IA.34. | H₂C=CH-CH(CH₃)- | H |
| C.IA.35. | H₂C=CH-C(CH₃)₂- | H |
| C.IA.36. | H₂C=CH-C(C₂H₅)(CH₃)- | H |
| C.IA.37. | C₆H₅-CH₂- | H |
| C.IA.38. | 4-(CH₃)₃C-C₆H₄-CH₂- | H |
| C.IA.39. | C₆H₅-CH₂- | H |
| C.IA.40. | 4-(CH₃)₃C-C₆H₄-CH₂- | H |
| C.IA.41. | 4-Cl-C₆H₄-CH₂- | H |
| C.IA.42. | 3-(CH₃O)-C₆H₄-CH₂- | H |
| C.IA.43. | 4-(CH₃O)-C₆H₄-CH₂- | H |
| C.IA.44. | 2-(CH₃O)-C₆H₄-CH₂- | H |
| C.IA.45. | 3-Cl-C₆H₄-CH₂- | H |
| C.IA.46. | 2-Cl-C₆H₄-CH₂- | H |
| C.IA.47. | 4-(F₃C)-C₆H₄-CH₂- | H |
| C.IA.48. | NC-CH₂- | H |
| C.IA.49. | NC-CH₂-CH₂- | H |
| C.IA.50. | NC-CH₂-CH(CH₃)- | H |
| C.IA.51. | NC-CH₂-C(CH₃)₂- | H |
| C.IA.52. | NC-CH₂-CH₂-CH₂- | H |
| C.IA.53. | FH₂C-CH₂- | H |
| C.IA.54. | ClH₂C-CH₂- | H |
| C.IA.55. | BrH₂C-CH₂- | H |
| C.IA.56. | FH₂C-CH(CH₃)- | H |
| C.IA.57. | ClH₂C-CH(CH₃)- | H |
| C.IA.58. | BrH₂C-CH(CH₃)- CH₃ | H |
| C.IA.59. | F₂HC-CH₂- | H |
| C.IA.60. | F₃C-CH₂- | H |
| C.IA.61. | FH₂C-CH₂-CH₂- | H |
| C.IA.62. | ClH₂C-CH₂-CH₂- | H |
| C.IA.63. | BrH₂C-CH₂-CH₂- | H |
| C.IA.64. | F₂HC-CH₂-CH₂- | H |
| C.IA.65. | F₃C-CH₂-CH₂- | H |
| C.IA.66. | CH₃-O-CH₂-CH₂- | H |
| C.IA.67. | CH₃-S-CH₂-CH₂- | H |
| C.IA.68. | CH₃-SO₂-CH₂-CH₂- | H |
| C.IA.69. | C₂H₅-O-CH₂-CH₂- | H |
| C.IA.70. | (CH₃)₂CH-O-CH₂-CH₂- | H |
| C.IA.71. | C₂H₅-S-CH₂-CH₂- | H |
| C.IA.72. | C₂H₅-SO₂-CH₂-CH₂- | H |
| C.IA.73. | (CH₃)₂N-CH₂-CH₂- | H |
| C.IA.74. | (C₂H₅)₂N-CH₂-CH₂- | H |
| C.IA.75. | [(CH₃)₂CH]₂N-CH₂-CH₂- | H |
| C.IA.76. | CH₃-O-CH₂-CH(CH₃)- | H |
| C.IA.77. | CH₃-S-CH₂-CH(CH₃)- | H |
| C.IA.78. | CH₃-SO₂-CH₂-CH(CH₃)- | H |
| C.IA.79. | C₂H₅-O-CH₂-CH(CH₃)- | H |
| C.IA.80. | C₂H₅-S-CH₂-CH(CH₃)- | H |
| C.IA.81. | C₂H₅-SO₂-CH₂-CH(CH₃)- | H |
| C.IA.82. | (CH₃)₂N-CH₂-CH(CH₃)- | H |
| C.IA.83. | (C₂H₅)₂N-CH₂-CH(CH₃)- | H |
| C.IA.84. | [(CH₃)₂CH]₂N-CH₂-CH(CH₃)- | H |
| C.IA.85. | CH₃-O-CH(CH₃)-CH₂- | H |
| C.IA.86. | CH₃-S-CH(CH₃)-CH₂- | H |
| C.IA.87. | CH₃-SO₂-CH(CH₃)-CH₂- | H |
| C.IA.88. | C₂H₅-O-CH(CH₃)-CH₂- | H |
| C.IA.89. | C₂H₅-S-CH(CH₃)-CH₂- | H |
| C.IA.90. | C₂H₅-SO₂-CH(CH₃)-CH₂- | H |
| C.IA.91. | (CH₃)₂N-CH(CH₃)-CH₂- | H |
| C.IA.92. | (C₂H₅)₂N-CH(CH₃)-CH₂- | H |
| C.IA.93. | [(CH₃)₂CH]₂N-CH(CH₃)-CH₂- | H |
| C.IA.94. | CH₃-O-CH₂-CH₂-CH₂- | H |
| C.IA.95. | CH₃-S-CH₂-CH₂-CH₂- | H |
| C.IA.96. | CH₃-SO₂-CH₂-CH₂-CH₂- | H |
| C.IA.97. | C₂H₅-O-CH₂-CH₂-CH₂- | H |
| C.IA.98. | C₂H₅-S-CH₂-CH₂-CH₂- | H |
| C.IA.99. | C₂H₅-SO₂-CH₂-CH₂-CH₂- | H |
| C.IA.100. | (CH₃)₂N-CH₂-CH₂-CH₂- | H |
| C.IA.101. | (C₂H₅)₂N-CH₂-CH₂-CH₂- | H |
| C.IA.102. | CH₃-O-CH₂-C(CH₃)₂- | H |
| C.IA.103. | CH₃-S-CH₂-C(CH₃)₂- | H |
| C.IA.104. | CH₃-SO₂-CH₂-C(CH₃)₂- | H |
| C.IA.105. | C₂H₅-O-CH₂-C(CH₃)₂- | H |
| C.IA.106. | C₂H₅-S-CH₂-C(CH₃)₂- | H |
| C.IA.107. | C₂H₅-SO₂-CH₂-C(CH₃)₂- | H |
| C.IA.108. | (CH₃)₂N-CH₂-C(CH₃)₂- | H |
| C.IA.109. | (C₂H₅)₂N-CH₂-C(CH₃)₂- | H |
| C.IA.110. | [(CH₃)₂CH]₂N-CH₂-C(CH₃)₂- | H |
| C.IA.111. | Cl-CH₂-C≡ C-CH₂- | H |
| C.IA.112. | CH₃-O-C(O)-CH₂ | H |
| C.IA.113. | C₂H₅-O-C(O)-CH₂ | H |
| C.IA.114. | CH₃-O-C(O)-CH(CH₃)- | H |
| C.IA.115. | C₂H₅-O-C(O)-CH(CH₃)- | H |
| C.IA.116. | (CH₃O)₂CH-CH₂- | H |
| C.IA.117. | (C₂H₅O)₂CH-CH₂- | H |
| C.IA.118. | C(=O)CH₃ | H |
| C.IA.119. | C(=O)CH₂-CH₃ | H |
| C.IA.120. | C(=O)CF₃ | H |
| C.IA.121. | C(=O)CCl₃ | H |
| C.IA.122. | C(=O)CH₂-CH₂-CH₃ | H |
| C.IA.123. | C(=O)C-(CH₃)₃ | H |
| C.IA.124. | C(=O)CH₂-C₆H₅ | H |
| C.IA.125. | C(=O)CH₂-CH₂-CH₃ | H |

Isomeric formula IB is represented, if W is connected to N² with a single bond and to N¹ with a double bond, then N¹ is not carrying the substituent R¹. In this case:

**Table B referring to isomeric form IB of formula I:**

| N° | R² | R³ |
|---|---|---|
| C.IB.1. | H | H |
| C.IB.2. | CH₃ | H |
| C.IB.3. | CH₃CH₂- | H |
| C.IB.4. | (CH₃)₂CH- | H |
| C.IB.5. | CH₃CH₂CH₂- | H |
| C.IB.6. | n-C₄H₉ | H |
| C.IB.7. | (CH₃)₃C- | H |
| C.IB.8. | (CH₃)₂CH-CH₂- | H |
| C.IB.9. | n-C₅H₁₁ | H |
| C.IB.10. | (CH₃)₂CH-CH₂-CH₂- | H |
| C.IB.11. | (C₂H₅)₂-CH- | H |
| C.IB.12. | (CH₃)₃C-CH₂- | H |
| C.IB.13. | (CH₃)₃C-CH₂-CH₂- | H |
| C.IB.14. | C₂H₅CH(CH₃)-CH₂- | H |
| C.IB.15. | CH₃-CH₂-C(CH₃)₂- | H |
| C.IB.16. | (CH₃)₂CH-CH(CH₃)- | H |
| C.IB.17. | (CH₃)₃C-CH(CH₃)- | H |
| C.IB.18. | (CH₃)₂CH-CH₂-CH(CH₃)- | H |
| C.IB.19. | CH₃-CH₂-C(CH₃)(C₂H₅)- | H |
| C.IB.20. | CH₃-CH₂-CH₂-C(CH₃)₂- | H |
| C.IB.21. | C₂H₅-CH₂-CH(CH₃)-CH₂- | H |
| C.IB.22. | cyclopropyl | H |
| C.IB.23. | cyclopropyl-CH₂- | H |
| C.IB.24. | cyclopropyl-CH(CH₃)- | H |
| C.IB.25. | Cyclobutyl | H |
| C.IB.26. | cyclopentyl | H |
| C.IB.27. | cyclohexyl | H |
| C.IB.28. | HC≡ C-CH₂- | H |
| C.IB.29. | HC≡ C-CH(CH₃)- | H |
| C.IB.30. | HC≡ C-C(CH₃)₂- | H |
| C.IB.31. | HC≡ C-C(CH₃)(C₂H₅)- | H |
| C.IB.32. | HC≡ C-C(CH₃)(C₃H₇)- | H |
| C.IB.33. | CH₂=CH-CH₂- | H |
| C.IB.34. | H₂C=CH-CH(CH₃)- | H |
| C.IB.35. | H₂C=CH-C(CH₃)₂- | H |
| C.IB.36. | H₂C=CH-C(C₂H₅)(CH₃)- | H |
| C.IB.37. | C₆H₅-CH₂- | H |
| C.IB.38. | 4-(CH₃)₃C-C₆H₄-CH₂- | H |
| C.IB.39. | C₆H₅-CH₂- | H |
| C.IB.40. | 4-(CH₃)₃C-C₆H₄-CH₂- | H |
| C.IB.41. | 4-Cl-C₆H₄-CH₂- | H |
| C.IB.42. | 3-(CH₃O)-C₆H₄-CH₂- | H |
| C.IB.43. | 4-(CH₃O)-C₆H₄-CH₂- | H |
| C.IB.44. | 2-(CH₃O)-C₆H₄-CH₂- | H |
| C.IB.45. | 3-Cl-C₆H₄-CH₂- | H |
| C.IB.46. | 2-Cl-C₆H₄-CH₂- | H |
| C.IB.47. | 4-(F₃C)-C₆H₄-CH₂- | H |
| C.IB.48. | NC-CH₂- | H |
| C.IB.49. | NC-CH₂-CH₂- | H |
| C.IB.50. | NC-CH₂-CH(CH₃)- | H |
| C.IB.51. | NC-CH₂-C(CH₃)₂- | H |
| C.IB.52. | NC-CH₂-CH₂-CH₂- | H |
| C.IB.53. | FH₂C-CH₂- | H |
| C.IB.54. | ClH₂C-CH₂- | H |
| C.IB.55. | BrH₂C-CH₂- | H |
| C.IB.56. | FH₂C-CH(CH₃)- | H |
| C.IB.57. | ClH₂C-CH(CH₃)- | H |
| C.IB.58. | BrH₂C-CH(CH₃)- CH₃ | H |
| C.IB.59. | F₂HC-CH₂- | H |
| C.IB.60. | F₃C-CH₂- | H |
| C.IB.61. | FH₂C-CH₂-CH₂- | H |
| C.IB.62. | ClH₂C-CH₂-CH₂- | H |
| C.IB.63. | BrH₂C-CH₂-CH₂- | H |
| C.IB.64. | F₂HC-CH₂-CH₂- | H |
| C.IB.65. | F₃C-CH₂-CH₂- | H |
| C.IB.66. | CH₃-O-CH₂-CH₂- | H |
| C.IB.67. | CH₃-S-CH₂-CH₂- | H |
| C.IB.68. | CH₃-SO₂-CH₂-CH₂- | H |
| C.IB.69. | C₂H₅-O-CH₂-CH₂- | H |
| C.IB.70. | (CH₃)₂CH-O-CH₂-CH₂- | H |
| C.IB.71. | C₂H₅-S-CH₂-CH₂- | H |
| C.IB.72. | C₂H₅-SO₂-CH₂-CH₂- | H |
| C.IB.73. | (CH₃)₂N-CH₂-CH₂- | H |
| C.IB.74. | (C₂H₅)₂N-CH₂-CH₂- | H |
| C.IB.75. | [(CH₃)₂CH]₂N-CH₂-CH₂- | H |
| C.IB.76. | CH₃-O-CH₂-CH(CH₃)- | H |
| C.IB.77. | CH₃-S-CH₂-CH(CH₃)- | H |
| C.IB.78. | CH₃-SO₂-CH₂-CH(CH₃)- | H |
| C.IB.79. | C₂H₅-O-CH₂-CH(CH₃)- | H |
| C.IB.80. | C₂H₅-S-CH₂-CH(CH₃)- | H |
| C.IB.81. | C₂H₅-SO₂-CH₂-CH(CH₃)- | H |
| C.IB.82. | (CH₃)₂N-CH₂-CH(CH₃)- | H |
| C.IB.83. | (C₂H₅)₂N-CH₂-CH(CH₃)- | H |
| C.IB.84. | [(CH₃)₂CH]₂N-CH₂-CH(CH₃)- | H |
| C.IB.85. | CH₃-O-CH(CH₃)-CH₂- | H |
| C.IB.86. | CH₃-S-CH(CH₃)-CH₂- | H |
| C.IB.87. | CH₃-SO₂-CH(CH₃)-CH₂- | H |
| C.IB.88. | C₂H₅-O-CH(CH₃)-CH₂- | H |
| C.IB.89. | C₂H₅-S-CH(CH₃)-CH₂- | H |
| C.IB.90. | C₂H₅-SO₂-CH(CH₃)-CH₂- | H |
| C.IB.91. | (CH₃)₂N-CH(CH₃)-CH₂- | H |
| C.IB.92. | (C₂H₅)₂N-CH(CH₃)-CH₂- | H |
| C.IB.93. | [(CH₃)₂CH]₂N-CH(CH₃)-CH₂- | H |
| C.IB.94. | CH₃-O-CH₂-CH₂-CH₂- | H |
| C.IB.95. | CH₃-S-CH₂-CH₂-CH₂- | H |
| C.IB.96. | CH₃-SO₂-CH₂-CH₂-CH₂- | H |
| C.IB.97. | C₂H₅-O-CH₂-CH₂-CH₂- | H |
| C.IB.98. | C₂H₅-S-CH₂-CH₂-CH₂- | H |
| C.IB.99. | C₂H₅-SO₂-CH₂-CH₂-CH₂- | H |
| C.IB.100. | (CH₃)₂N-CH₂-CH₂-CH₂- | H |
| C.IB.101. | (C₂H₅)₂N-CH₂-CH₂-CH₂- | H |
| C.IB.102. | CH₃-O-CH₂-C(CH₃)₂- | H |
| C.IB.103. | CH₃-S-CH₂-C(CH₃)₂- | H |
| C.IB.104. | CH₃-SO₂-CH₂-C(CH₃)₂- | H |
| C.IB.105. | C₂H₅-O-CH₂-C(CH₃)₂- | H |
| C.IB.106. | C₂H₅-S-CH₂-C(CH₃)₂- | H |
| C.IB.107. | C₂H₅-SO₂-CH₂-C(CH₃)₂- | H |
| C.IB.108. | (CH₃)₂N-CH₂-C(CH₃)₂- | H |
| C.IB.109. | (C₂H₅)₂N-CH₂-C(CH₃)₂- | H |
| C.IB.110. | [(CH₃)₂CH]₂N-CH₂-C(CH₃)₂- | H |
| C.IB.111. | Cl-CH₂-C≡ C-CH₂- | H |
| C.IB.112. | CH₃-O-C(O)-CH₂ | H |
| C.IB.113. | C₂H₅-O-C(O)-CH₂ | H |
| C.IB.114. | CH₃-O-C(O)-CH(CH₃)- | H |
| C.IB.115. | C₂H₅-O-C(O)-CH(CH₃)- | H |
| C.IB.116. | (CH₃O)₂CH-CH₂- | H |
| C.IB.117. | (C₂H₅O)₂CH-CH₂- | H |
| C.IB.118. | C(=O)CH₃ | H |
| C.IB.119. | C(=O)CH₂-CH₃ | H |
| C.IB.120. | C(=O)CF₃ | H |
| C.IB.121. | C(=O)CCl₃ | H |
| C.IB.122. | C(=O)CH₂-CH₂-CH₃ | H |
| C.IB.123. | C(=O)C-(CH₃)₃ | H |
| C.IB.124. | C(=O)CH₂-C₆H₅ | H |
| C.IB.125. | C(=O)CH₂-CH₂-CH₃ | H |
| C.IB.126. | H | CH₃ |
| C.IB.127. | CH₃ | CH₃ |
| C.IB.128. | CH₃CH₂- | CH₃ |
| C.IB.129. | (CH₃)₂CH- | CH₃ |
| C.IB.130. | CH₃CH₂CH₂- | CH₃ |
| C.IB.131. | n-C₄H₉ | CH₃ |
| C.IB.132. | (CH₃)₃C- | CH₃ |
| C.IB.133. | (CH₃)₂CH-CH₂- | CH₃ |
| C.IB.134. | n-C₅H₁₁ | CH₃ |
| C.IB.135. | (CH₃)₂CH-CH₂-CH₂- | CH₃ |
| C.IB.136. | (C₂H₅)₂-CH- | CH₃ |
| C.IB.137. | (CH₃)₃C-CH₂- | CH₃ |
| C.IB.138. | (CH₃)₃C-CH₂-CH₂- | CH₃ |
| C.IB.139. | C₂H₅CH(CH₃)-CH₂- | CH₃ |
| C.IB.140. | CH₃-CH₂-C(CH₃)₂- | CH₃ |
| C.IB.141. | (CH₃)₂CH-CH(CH₃)- | CH₃ |
| C.IB.142. | (CH₃)₃C-CH(CH₃)- | CH₃ |
| C.IB.143. | (CH₃)₂CH-CH₂-CH(CH₃)- | CH₃ |
| C.IB.144. | CH₃-CH₂-C(CH₃)(C₂H₅)- | CH₃ |
| C.IB.145. | CH₃-CH₂-CH₂-C(CH₃)₂- | CH₃ |
| C.IB.146. | C₂H₅-CH₂-CH(CH₃)-CH₂- | CH₃ |
| C.IB.147. | cyclopropyl | CH₃ |
| C.IB.148. | cyclopropyl-CH₂- | CH₃ |
| C.IB.149. | cyclopropyl-CH(CH₃)- | CH₃ |
| C.IB.150. | cyclobutyl | CH₃ |
| C.IB.151. | cyclopentyl | CH₃ |
| C.IB.152. | cyclohexyl | CH₃ |
| C.IB.153. | HC≡ C-CH₂- | CH₃ |
| C.IB.154. | HC≡ C-CH(CH₃)- | CH₃ |
| C.IB.155. | HC≡ C-C(CH₃)₂- | CH₃ |
| C.IB.156. | HC≡ C-C(CH₃)(C₂H₅)- | CH₃ |
| C.IB.157. | HC≡ C-C(CH₃)(C₃H₇)- | CH₃ |
| C.IB.158. | CH₂=CH-CH₂- | CH₃ |
| C.IB.159. | H₂C=CH-CH(CH₃)- | CH₃ |
| C.IB.160. | H₂C=CH-C(CH₃)₂- | CH₃ |
| C.IB.161. | H₂C=CH-C(C₂H₅)(CH₃)- | CH₃ |
| C.IB.162. | C₆H₅-CH₂- | CH₃ |
| C.IB.163. | 4-(CH₃)₃C-C₆H₄-CH₂- | CH₃ |
| C.IB.164. | C₆H₅-CH₂- | CH₃ |
| C.IB.165. | 4-(CH₃)₃C-C₆H₄-CH₂- | CH₃ |
| C.IB.166. | 4-Cl-C₆H₄-CH₂- | CH₃ |
| C.IB.167. | 3-(CH₃O)-C₆H₄-CH₂- | CH₃ |
| C.IB.168. | 4-(CH₃O)-C₆H₄-CH₂- | CH₃ |
| C.IB.169. | 2-(CH₃O)-C₆H₄-CH₂- | CH₃ |
| C.IB.170. | 3-Cl-C₆H₄-CH₂- | CH₃ |
| C.IB.171. | 2-Cl-C₆H₄-CH₂- | CH₃ |
| C.IB.172. | 4-(F₃C)-C₆H₄-CH₂- | CH₃ |
| C.IB.173. | NC-CH₂- | CH₃ |
| C.IB.174. | NC-CH₂-CH₂- | CH₃ |
| C.IB.175. | NC-CH₂-CH(CH₃)- | CH₃ |
| C.IB.176. | NC-CH₂-C(CH₃)₂- | CH₃ |
| C.IB.177. | NC-CH₂-CH₂-CH₂- | CH₃ |
| C.IB.178. | FH₂C-CH₂- | CH₃ |
| C.IB.179. | ClH₂C-CH₂- | CH₃ |
| C.IB.180. | BrH₂C-CH₂- | CH₃ |
| C.IB.181. | FH₂C-CH(CH₃)- | CH₃ |
| C.IB.182. | ClH₂C-CH(CH₃)- | CH₃ |
| C.IB.183. | BrH₂C-CH(CH₃)- CH₃ | CH₃ |
| C.IB.184. | F₂HC-CH₂- | CH₃ |
| C.IB.185. | F₃C-CH₂- | CH₃ |
| C.IB.186. | FH₂C-CH₂-CH₂- | CH₃ |
| C.IB.187. | ClH₂C-CH₂-CH₂- | CH₃ |
| C.IB.188. | BrH₂C-CH₂-CH₂- | CH₃ |
| C.IB.189. | F₂HC-CH₂-CH₂- | CH₃ |
| C.IB.190. | F₃C-CH₂-CH₂- | CH₃ |
| C.IB.191. | CH₃-O-CH₂-CH₂- | CH₃ |
| C.IB.192. | CH₃-S-CH₂-CH₂- | CH₃ |
| C.IB.193. | CH₃-SO₂-CH₂-CH₂- | CH₃ |
| C.IB.194. | C₂H₅-O-CH₂-CH₂- | CH₃ |
| C.IB.195. | (CH₃)₂CH-O-CH₂-CH₂- | CH₃ |
| C.IB.196. | C₂H₅-S-CH₂-CH₂- | CH₃ |
| C.IB.197. | C₂H₅-SO₂-CH₂-CH₂- | CH₃ |
| C.IB.198. | (CH₃)₂N-CH₂-CH₂- | CH₃ |
| C.IB.199. | (C₂H₅)₂N-CH₂-CH₂- | CH₃ |
| C.IB.200. | [(CH₃)₂CH]₂N-CH₂-CH₂- | CH₃ |
| C.IB.201. | CH₃-O-CH₂-CH(CH₃)- | CH₃ |
| C.IB.202. | CH₃-S-CH₂-CH(CH₃)- | CH₃ |
| C.IB.203. | CH₃-SO₂-CH₂-CH(CH₃)- | CH₃ |
| C.IB.204. | C₂H₅-O-CH₂-CH(CH₃)- | CH₃ |
| C.IB.205. | C₂H₅-S-CH₂-CH(CH₃)- | CH₃ |
| C.IB.206. | C₂H₅-SO₂-CH₂-CH(CH₃)- | CH₃ |
| C.IB.207. | (CH₃)₂N-CH₂-CH(CH₃)- | CH₃ |
| C.IB.208. | (C₂H₅)₂N-CH₂-CH(CH₃)- | CH₃ |
| C.IB.209. | [(CH₃)₂CH]₂N-CH₂-CH(CH₃)- | CH₃ |
| C.IB.210. | CH₃-O-CH(CH₃)-CH₂- | CH₃ |
| C.IB.211. | CH₃-S-CH(CH₃)-CH₂- | CH₃ |
| C.IB.212. | CH₃-SO₂-CH(CH₃)-CH₂- | CH₃ |
| C.IB.213. | C₂H₅-O-CH(CH₃)-CH₂- | CH₃ |
| C.IB.214. | C₂H₅-S-CH(CH₃)-CH₂- | CH₃ |
| C.IB.215. | C₂H₅-SO₂-CH(CH₃)-CH₂- | CH₃ |
| C.IB.216. | (CH₃)₂N-CH(CH₃)-CH₂- | CH₃ |
| C.IB.217. | (C₂H₅)₂N-CH(CH₃)-CH₂- | CH₃ |
| C.IB.218. | [(CH₃)₂CH]₂N-CH(CH₃)-CH₂- | CH₃ |
| C.IB.219. | CH₃-O-CH₂-CH₂-CH₂- | CH₃ |
| C.IB.220. | CH₃-S-CH₂-CH₂-CH₂- | CH₃ |
| C.IB.221. | CH₃-SO₂-CH₂-CH₂-CH₂- | CH₃ |
| C.IB.222. | C₂H₅-O-CH₂-CH₂-CH₂- | CH₃ |
| C.IB.223. | C₂H₅-S-CH₂-CH₂-CH₂- | CH₃ |
| C.IB.224. | C₂H₅-SO₂-CH₂-CH₂-CH₂- | CH₃ |
| C.IB.225. | (CH₃)₂N-CH₂-CH₂-CH₂- | CH₃ |
| C.IB.226. | (C₂H₅)₂N-CH₂-CH₂-CH₂- | CH₃ |
| C.IB.227. | CH₃-O-CH₂-C(CH₃)₂- | CH₃ |
| C.IB.228. | CH₃-S-CH₂-C(CH₃)₂- | CH₃ |
| C.IB.229. | CH₃-SO₂-CH₂-C(CH₃)₂- | CH₃ |
| C.IB.230. | C₂H₅-O-CH₂-C(CH₃)₂- | CH₃ |
| C.IB.231. | C₂H₅-S-CH₂-C(CH₃)₂- | CH₃ |
| C.IB.232. | C₂H₅-SO₂-CH₂-C(CH₃)₂- | CH₃ |
| C.IB.233. | (CH₃)₂N-CH₂-C(CH₃)₂- | CH₃ |
| C.IB.234. | (C₂H₅)₂N-CH₂-C(CH₃)₂- | CH₃ |
| C.IB.235. | [(CH₃)₂CH]₂N-CH₂-C(CH₃)₂- | CH₃ |
| C.IB.236. | Cl-CH₂-C≡ C-CH₂- | CH₃ |
| C.IB.237. | CH₃-O-C(O)-CH₂ | CH₃ |
| C.IB.238. | C₂H₅-O-C(O)-CH₂ | CH₃ |
| C.IB.239. | CH₃-O-C(O)-CH(CH₃)- | CH₃ |
| C.IB.240. | C₂H₅-O-C(O)-CH(CH₃)- | CH₃ |
| C.IB.241. | (CH₃O)₂CH-CH₂- | CH₃ |
| C.IB.242. | (C₂H₅O)₂CH-CH₂- | CH₃ |
| C.IB.243. | C(=O)CH₃ | CH₃ |
| C.IB.244. | C(=O)CH₂-CH₃ | CH₃ |
| C.IB.245. | C(=O)CF₃ | CH₃ |
| C.IB.246. | C(=O)CCl₃ | CH₃ |
| C.IB.247. | C(=O)CH₂-CH₂-CH₃ | CH₃ |
| C.IB.248. | C(=O)C-(CH₃)₃ | CH₃ |
| C.IB.249. | C(=O)CH₂-C₆H₅ | CH₃ |
| C.IB.250. | C(=O)CH₂-CH₂-CH₃ | CH₃ |
| C.IB.251. | H | C₂H₅ |
| C.IB.252. | CH₃ | C₂H₅ |
| C.IB.253. | CH₃CH₂- | C₂H₅ |
| C.IB.254. | (CH₃)₂CH- | C₂H₅ |
| C.IB.255. | CH₃CH₂CH₂- | C₂H₅ |
| C.IB.256. | n-C₄H₉ | C₂H₅ |
| C.IB.257. | (CH₃)₃C- | C₂H₅ |
| C.IB.258. | (CH₃)₂CH-CH₂- | C₂H₅ |
| C.IB.259. | n-C₅H₁₁ | C₂H₅ |
| C.IB.260. | (CH₃)₂CH-CH₂-CH₂- | C₂H₅ |
| C.IB.261. | (C₂H₅)₂-CH- | C₂H₅ |
| C.IB.262. | (CH₃)₃C-CH₂- | C₂H₅ |
| C.IB.263. | (CH₃)₃C-CH₂-CH₂- | C₂H₅ |
| C.IB.264. | C₂H₅CH(CH₃)-CH₂- | C₂H₅ |
| C.IB.265. | CH₃-CH₂-C(CH₃)₂- | C₂H₅ |
| C.IB.266. | (CH₃)₂CH-CH(CH₃)- | C₂H₅ |
| C.IB.267. | (CH₃)₃C-CH(CH₃)- | C₂H₅ |
| C.IB.268. | (CH₃)₂CH-CH₂-CH(CH₃)- | C₂H₅ |
| C.IB.269. | CH₃-CH₂-C(CH₃)(C₂H₅)- | C₂H₅ |
| C.IB.270. | CH₃-CH₂-CH₂-C(CH₃)₂- | C₂H₅ |
| C.IB.271. | C₂H₅-CH₂-CH(CH₃)-CH₂- | C₂H₅ |
| C.IB.272. | cyclopropyl | C₂H₅ |
| C.IB.273. | cyclopropyl-CH₂- | C₂H₅ |
| C.IB.274. | cyclopropyl-CH(CH₃)- | C₂H₅ |
| C.IB.275. | cyclobutyl | C₂H₅ |
| C.IB.276. | cyclopentyl | C₂H₅ |
| C.IB.277. | cyclohexyl | C₂H₅ |
| C.IB.278. | HC≡ C-CH₂- | C₂H₅ |
| C.IB.279. | HC≡ C-CH(CH₃)- | C₂H₅ |
| C.IB.280. | HC≡ C-C(CH₃)₂- | C₂H₅ |
| C.IB.281. | HC≡ C-C(CH₃)(C₂H₅)- | C₂H₅ |
| C.IB.282. | HC≡ C-C(CH₃)(C₃H₇)- | C₂H₅ |
| C.IB.283. | CH₂=CH-CH₂- | C₂H₅ |
| C.IB.284. | H₂C=CH-CH(CH₃)- | C₂H₅ |
| C.IB.285. | H₂C=CH-C(CH₃)₂- | C₂H₅ |
| C.IB.286. | H₂C=CH-C(C₂H₅)(CH₃)- | C₂H₅ |
| C.IB.287. | C₆H₅-CH₂- | C₂H₅ |
| C.IB.288. | 4-(CH₃)₃C-C₆H₄-CH₂- | C₂H₅ |
| C.IB.289. | C₆H₅-CH₂- | C₂H₅ |
| C.IB.290. | 4-(CH₃)₃C-C₆H₄-CH₂- | C₂H₅ |
| C.IB.291. | 4-Cl-C₆H₄-CH₂- | C₂H₅ |
| C.IB.292. | 3-(CH₃O)-C₆H₄-CH₂- | C₂H₅ |
| C.IB.293. | 4-(CH₃O)-C₆H₄-CH₂- | C₂H₅ |
| C.IB.294. | 2-(CH₃O)-C₆H₄-CH₂- | C₂H₅ |
| C.IB.295. | 3-Cl-C₆H₄-CH₂- | C₂H₅ |
| C.IB.296. | 2-Cl-C₆H₄-CH₂- | C₂H₅ |
| C.IB.297. | 4-(F₃C)-C₆H₄-CH₂- | C₂H₅ |
| C.IB.298. | NC-CH₂- | C₂H₅ |
| C.IB.299. | NC-CH₂-CH₂- | C₂H₅ |
| C.IB.300. | NC-CH₂-CH(CH₃)- | C₂H₅ |
| C.IB.301. | NC-CH₂-C(CH₃)₂- | C₂H₅ |
| C.IB.302. | NC-CH₂-CH₂-CH₂- | C₂H₅ |
| C.IB.303. | FH₂C-CH₂- | C₂H₅ |
| C.IB.304. | ClH₂C-CH₂- | C₂H₅ |
| C.IB.305. | BrH₂C-CH₂- | C₂H₅ |
| C.IB.306. | FH₂C-CH(CH₃)- | C₂H₅ |
| C.IB.307. | ClH₂C-CH(CH₃)- | C₂H₅ |
| C.IB.308. | BrH₂C-CH(CH₃)- CH₃ | C₂H₅ |
| C.IB.309. | F₂HC-CH₂- | C₂H₅ |
| C.IB.310. | F₃C-CH₂- | C₂H₅ |
| C.IB.311. | FH₂C-CH₂-CH₂- | C₂H₅ |
| C.IB.312. | ClH₂C-CH₂-CH₂- | C₂H₅ |
| C.IB.313. | BrH₂C-CH₂-CH₂- | C₂H₅ |
| C.IB.314. | F₂HC-CH₂-CH₂- | C₂H₅ |
| C.IB.315. | F₃C-CH₂-CH₂- | C₂H₅ |
| C.IB.316. | CH₃-O-CH₂-CH₂- | C₂H₅ |
| C.IB.317. | CH₃-S-CH₂-CH₂- | C₂H₅ |
| C.IB.318. | CH₃-SO₂-CH₂-CH₂- | C₂H₅ |
| C.IB.319. | C₂H₅-O-CH₂-CH₂- | C₂H₅ |
| C.IB.320. | (CH₃)₂CH-O-CH₂-CH₂- | C₂H₅ |
| C.IB.321. | C₂H₅-S-CH₂-CH₂- | C₂H₅ |
| C.IB.322. | C₂H₅-SO₂-CH₂-CH₂- | C₂H₅ |
| C.IB.323. | (CH₃)₂N-CH₂-CH₂- | C₂H₅ |
| C.IB.324. | (C₂H₅)₂N-CH₂-CH₂- | C₂H₅ |
| C.IB.325. | [(CH₃)₂CH]₂N-CH₂-CH₂- | C₂H₅ |
| C.IB.326. | CH₃-O-CH₂-CH(CH₃)- | C₂H₅ |
| C.IB.327. | CH₃-S-CH₂-CH(CH₃)- | C₂H₅ |
| C.IB.328. | CH₃-SO₂-CH₂-CH(CH₃)- | C₂H₅ |
| C.IB.329. | C₂H₅-O-CH₂-CH(CH₃)- | C₂H₅ |
| C.IB.330. | C₂H₅-S-CH₂-CH(CH₃)- | C₂H₅ |
| C.IB.331. | C₂H₅-SO₂-CH₂-CH(CH₃)- | C₂H₅ |
| C.IB.332. | (CH₃)₂N-CH₂-CH(CH₃)- | C₂H₅ |
| C.IB.333. | (C₂H₅)₂N-CH₂-CH(CH₃)- | C₂H₅ |
| C.IB.334. | [(CH₃)₂CH]₂N-CH₂-CH(CH₃)- | C₂H₅ |
| C.IB.335. | CH₃-O-CH(CH₃)-CH₂- | C₂H₅ |
| C.IB.336. | CH₃-S-CH(CH₃)-CH₂- | C₂H₅ |
| C.IB.337. | CH₃-SO₂-CH(CH₃)-CH₂- | C₂H₅ |
| C.IB.338. | C₂H₅-O-CH(CH₃)-CH₂- | C₂H₅ |
| C.IB.339. | C₂H₅-S-CH(CH₃)-CH₂- | C₂H₅ |
| C.IB.340. | C₂H₅-SO₂-CH(CH₃)-CH₂- | C₂H₅ |
| C.IB.341. | (CH₃)₂N-CH(CH₃)-CH₂- | C₂H₅ |
| C.IB.342. | (C₂H₅)₂N-CH(CH₃)-CH₂- | C₂H₅ |
| C.IB.343. | [(CH₃)₂CH]₂N-CH(CH₃)-CH₂- | C₂H₅ |
| C.IB.344. | CH₃-O-CH₂-CH₂-CH₂- | C₂H₅ |
| C.IB.345. | CH₃-S-CH₂-CH₂-CH₂- | C₂H₅ |
| C.IB.346. | CH₃-SO₂-CH₂-CH₂-CH₂- | C₂H₅ |
| C.IB.347. | C₂H₅-O-CH₂-CH₂-CH₂- | C₂H₅ |
| C.IB.348. | C₂H₅-S-CH₂-CH₂-CH₂- | C₂H₅ |
| C.IB.349. | C₂H₅-SO₂-CH₂-CH₂-CH₂- | C₂H₅ |
| C.IB.350. | (CH₃)₂N-CH₂-CH₂-CH₂- | C₂H₅ |
| C.IB.351. | (C₂H₅)₂N-CH₂-CH₂-CH₂- | C₂H₅ |
| C.IB.352. | CH₃-O-CH₂-C(CH₃)₂- | C₂H₅ |
| C.IB.353. | CH₃-S-CH₂-C(CH₃)₂- | C₂H₅ |
| C.IB.354. | CH₃-SO₂-CH₂-C(CH₃)₂- | C₂H₅ |
| C.IB.355. | C₂H₅-O-CH₂-C(CH₃)₂- | C₂H₅ |
| C.IB.356. | C₂H₅-S-CH₂-C(CH₃)₂- | C₂H₅ |
| C.IB.357. | C₂H₅-SO₂-CH₂-C(CH₃)₂- | C₂H₅ |
| C.IB.358. | (CH₃)₂N-CH₂-C(CH₃)₂- | C₂H₅ |
| C.IB.359. | (C₂H₅)₂N-CH₂-C(CH₃)₂- | C₂H₅ |
| C.IB.360. | [(CH₃)₂CH]₂N-CH₂-C(CH₃)₂- | C₂H₅ |
| C.IB.361. | Cl-CH₂-C≡ C-CH₂- | C₂H₅ |
| C.IB.362. | CH₃-O-C(O)-CH₂ | C₂H₅ |
| C.IB.363. | C₂H₅-O-C(O)-CH₂ | C₂H₅ |
| C.IB.364. | CH₃-O-C(O)-CH(CH₃)- | C₂H₅ |
| C.IB.365. | C₂H₅-O-C(O)-CH(CH₃)- | C₂H₅ |
| C.IB.366. | (CH₃O)₂CH-CH₂- | C₂H₅ |
| C.IB.367. | (C₂H₅O)₂CH-CH₂- | C₂H₅ |
| C.IB.368. | C(=O)CH₃ | C₂H₅ |
| C.IB.369. | C(=O)CH₂-CH₃ | C₂H₅ |
| C.IB.370. | C(=O)CF₃ | C₂H₅ |
| C.IB.371. | C(=O)CCl₃ | C₂H₅ |
| C.IB.372. | C(=O)CH₂-CH₂-CH₃ | C₂H₅ |
| C.IB.373. | C(=O)C-(CH₃)₃ | C₂H₅ |
| C.IB.374. | C(=O)CH₂-C₆H₅ | C₂H₅ |
| C.IB.375. | C(=O)CH₂-CH₂-CH₃ | C₂H₅ |
| C.IB.376. | H | C(=O)CH₃ |
| C.IB.377. | CH₃ | C(=O)CH₃ |
| C.IB.378. | CH₃CH₂- | C(=O)CH₃ |
| C.IB.379. | (CH₃)₂CH- | C(=O)CH₃ |
| C.IB.380. | CH₃CH₂CH₂- | C(=O)CH₃ |
| C.IB.381. | n-C₄H₉ | C(=O)CH₃ |
| C.IB.382. | (CH₃)₃C- | C(=O)CH₃ |
| C.IB.383. | (CH₃)₂CH-CH₂- | C(=O)CH₃ |
| C.IB.384. | n-C₅H₁₁ | C(=O)CH₃ |
| C.IB.385. | (CH₃)₂CH-CH₂-CH₂- | C(=O)CH₃ |
| C.IB.386. | (C₂H₅)₂-CH- | C(=O)CH₃ |
| C.IB.387. | (CH₃)₃C-CH₂- | C(=O)CH₃ |
| C.IB.388. | (CH₃)₃C-CH₂-CH₂- | C(=O)CH₃ |
| C.IB.389. | C₂H₅CH(CH₃)-CH₂- | C(=O)CH₃ |
| C.IB.390. | CH₃-CH₂-C(CH₃)₂- | C(=O)CH₃ |
| C.IB.391. | (CH₃)₂CH-CH(CH₃)- | C(=O)CH₃ |
| C.IB.392. | (CH₃)₃C-CH(CH₃)- | C(=O)CH₃ |
| C.IB.393. | (CH₃)₂CH-CH₂-CH(CH₃)- | C(=O)CH₃ |
| C.IB.394. | CH₃-CH₂-C(CH₃)(C₂H₅)- | C(=O)CH₃ |
| C.IB.395. | CH₃-CH₂-CH₂-C(CH₃)₂- | C(=O)CH₃ |
| C.IB.396. | C₂H₅-CH₂-CH(CH₃)-CH₂- | C(=O)CH₃ |
| C.IB.397. | cyclopropyl | C(=O)CH₃ |
| C.IB.398. | cyclopropyl-CH₂- | C(=O)CH₃ |
| C.IB.399. | cyclopropyl-CH(CH₃)- | C(=O)CH₃ |
| C.IB.400. | cyclobutyl | C(=O)CH₃ |
| C.IB.401. | cyclopentyl | C(=O)CH₃ |
| C.IB.402. | cyclohexyl | C(=O)CH₃ |
| C.IB.403. | HC≡ C-CH₂- | C(=O)CH₃ |
| C.IB.404. | HC≡ C-CH(CH₃)- | C(=O)CH₃ |
| C.IB.405. | HC≡ C-C(CH₃)₂- | C(=O)CH₃ |
| C.IB.406. | HC≡ C-C(CH₃)(C₂H₅)- | C(=O)CH₃ |
| C.IB.407. | HC≡ C-C(CH₃)(C₃H₇)- | C(=O)CH₃ |
| C.IB.408. | CH₂=CH-CH₂- | C(=O)CH₃ |
| C.IB.409. | H₂C=CH-CH(CH₃)- | C(=O)CH₃ |
| C.IB.410. | H₂C=CH-C(CH₃)₂- | C(=O)CH₃ |
| C.IB.411. | H₂C=CH-C(C₂H₅)(CH₃)- | C(=O)CH₃ |
| C.IB.412. | C₆H₅-CH₂- | C(=O)CH₃ |
| C.IB.413. | 4-(CH₃)₃C-C₆H₄-CH₂- | C(=O)CH₃ |
| C.IB.414. | C₆H₅-CH₂- | C(=O)CH₃ |
| C.IB.415. | 4-(CH₃)₃C-C₆H₄-CH₂- | C(=O)CH₃ |
| C.IB.416. | 4-Cl-C₆H₄-CH₂- | C(=O)CH₃ |
| C.IB.417. | 3-(CH₃O)-C₆H₄-CH₂- | C(=O)CH₃ |
| C.IB.418. | 4-(CH₃O)-C₆H₄-CH₂- | C(=O)CH₃ |
| C.IB.419. | 2-(CH₃O)-C₆H₄-CH₂- | C(=O)CH₃ |
| C.IB.420. | 3-Cl-C₆H₄-CH₂- | C(=O)CH₃ |
| C.IB.421. | 2-Cl-C₆H₄-CH₂- | C(=O)CH₃ |
| C.IB.422. | 4-(F₃C)-C₆H₄-CH₂- | C(=O)CH₃ |
| C.IB.423. | NO-CH₂- | C(=O)CH₃ |
| C.IB.424. | NC-CH₂-CH₂- | C(=O)CH₃ |
| C.IB.425. | NC-CH₂-CH(CH₃)- | C(=O)CH₃ |
| C.IB.426. | NC-CH₂-C(CH₃)₂- | C(=O)CH₃ |
| C.IB.427. | NC-CH₂-CH₂-CH₂- | C(=O)CH₃ |
| C.IB.428. | FH₂C-CH₂- | C(=O)CH₃ |
| C.IB.429. | CIH₂C-CH₂- | C(=O)CH₃ |
| C.IB.430. | BrH₂C-CH₂- | C(=O)CH₃ |
| C.IB.431. | FH₂C-CH(CH₃)- | C(=O)CH₃ |
| C.IB.432. | ClH₂C-CH(CH₃)- | C(=O)CH₃ |
| C.IB.433. | BrH₂C-CH(CH₃)- CH₃ | C(=O)CH₃ |
| C.IB.434. | F₂HC-CH₂- | C(=O)CH₃ |
| C.IB.435. | F₃C-CH₂- | C(=O)CH₃ |
| C.IB.436. | FH₂C-CH₂-CH₂- | C(=O)CH₃ |
| C.IB.437. | ClH₂C-CH₂-CH₂- | C(=O)CH₃ |
| C.IB.438. | BrH₂C-CH₂-CH₂- | C(=O)CH₃ |
| C.IB.439. | F₂HC-CH₂-CH₂- | C(=O)CH₃ |
| C.IB.440. | F₃C-CH₂-CH₂- | C(=O)CH₃ |
| C.IB.441. | CH₃-O-CH₂-CH₂- | C(=O)CH₃ |
| C.IB.442. | CH₃-S-CH₂-CH₂- | C(=O)CH₃ |
| C.IB.443. | CH₃-SO₂-CH₂-CH₂- | C(=O)CH₃ |
| C.IB.444. | C₂H₅-O-CH₂-CH₂- | C(=O)CH₃ |
| C.IB.445. | (CH₃)₂CH-O-CH₂-CH₂- | C(=O)CH₃ |
| C.IB.446. | C₂H₅-S-CH₂-CH₂- | C(=O)CH₃ |
| C.IB.447. | C₂H₅-SO₂-CH₂-CH₂- | C(=O)CH₃ |
| C.IB.448. | (CH₃)₂N-CH₂-CH₂- | C(=O)CH₃ |
| C.IB.449. | (C₂H₅)₂N-CH₂-CH₂- | C(=O)CH₃ |
| C.IB.450. | [(CH₃)₂CH]₂N-CH₂-CH₂- | C(=O)CH₃ |
| C.IB.451. | CH₃-O-CH₂-CH(CH₃)- | C(=O)CH₃ |
| C.IB.452. | CH₃-S-CH₂-CH(CH₃)- | C(=O)CH₃ |
| C.IB.453. | CH₃-SO₂-CH₂-CH(CH₃)- | C(=O)CH₃ |
| C.IB.454. | C₂H₅-O-CH₂-CH(CH₃)- | C(=O)CH₃ |
| C.IB.455. | C₂H₅-S-CH₂-CH(CH₃)- | C(=O)CH₃ |
| C.IB.456. | C₂H₅-SO₂-CH₂-CH(CH₃)- | C(=O)CH₃ |
| C.IB.457. | (CH₃)₂N-CH₂-CH(CH₃)- | C(=O)CH₃ |
| C.IB.458. | (C₂H₅)₂N-CH₂-CH(CH₃)- | C(=O)CH₃ |
| C.IB.459. | [(CH₃)₂CH]₂N-CH₂-CH(CH₃)- | C(=O)CH₃ |
| C.IB.460. | CH₃-O-CH(CH₃)-CH₂- | C(=O)CH₃ |
| C.IB.461. | CH₃-S-CH(CH₃)-CH₂- | C(=O)CH₃ |
| C.IB.462. | CH₃-SO₂-CH(CH₃)-CH₂- | C(=O)CH₃ |
| C.IB.463. | C₂H₅-O-CH(CH₃)-CH₂- | C(=O)CH₃ |
| C.IB.464. | C₂H₅-S-CH(CH₃)-CH₂- | C(=O)CH₃ |
| C.IB.465. | C₂H₅-SO₂-CH(CH₃)-CH₂- | C(=O)CH₃ |
| C.IB.466. | (CH₃)₂N-CH(CH₃)-CH₂- | C(=O)CH₃ |
| C.IB.467. | (C₂H₅)₂N-CH(CH₃)-CH₂- | C(=O)CH₃ |
| C.IB.468. | [(CH₃)₂CH]₂N-CH(CH₃)-CH₂- | C(=O)CH₃ |
| C.IB.469. | CH₃-O-CH₂-CH₂-CH₂- | C(=O)CH₃ |
| C.IB.470. | CH₃-S-CH₂-CH₂-CH₂- | C(=O)CH₃ |
| C.IB.471. | CH₃-SO₂-CH₂-CH₂-CH₂- | C(=O)CH₃ |
| C.IB.472. | C₂H₅-O-CH₂-CH₂-CH₂- | C(=O)CH₃ |
| C.IB.473. | C₂H₅-S-CH₂-CH₂-CH₂- | C(=O)CH₃ |
| C.IB.474. | C₂H₅-SO₂-CH₂-CH₂-CH₂- | C(=O)CH₃ |
| C.IB.475. | (CH₃)₂N-CH₂-CH₂-CH₂- | C(=O)CH₃ |
| C.IB.476. | (C₂H₅)₂N-CH₂-CH₂-CH₂- | C(=O)CH₃ |
| C.IB.477. | CH₃-O-CH₂-C(CH₃)₂- | C(=O)CH₃ |
| C.IB.478. | CH₃-S-CH₂-C(CH₃)₂- | C(=O)CH₃ |
| C.IB.479. | CH₃-SO₂-CH₂-C(CH₃)₂- | C(=O)CH₃ |
| C.IB.480. | C₂H₅-O-CH₂-C(CH₃)₂- | C(=O)CH₃ |
| C.IB.481. | C₂H₅-S-CH₂-C(CH₃)₂- | C(=O)CH₃ |
| C.IB.482. | C₂H₅-SO₂-CH₂-C(CH₃)₂- | C(=O)CH₃ |
| C.IB.483: | (CH₃)₂N-CH₂-C(CH₃)₂- | C(=O)CH₃ |
| C.IB.484. | (C₂H₅)₂N-CH₂-C(CH₃)₂- | C(=O)CH₃ |
| C.IB.485. | [(CH₃)₂CH]₂N-CH₂-C(CH₃)₂- | C(=O)CH₃ |
| C.IB.486. | Cl-CH₂-C≡ C-CH₂- | C(=O)CH₃ |
| C.IB.487. | CH₃-O-C(O)-CH₂ | C(=O)CH₃ |
| C.IB.488. | C₂H₅-O-C(O)-CH₂ | C(=O)CH₃ |
| C.IB.489. | CH₃-O-C(O)-CH(CH₃)- | C(=O)CH₃ |
| C.IB.490. | C₂H₅-O-C(O)-CH(CH₃)- | C(=O)CH₃ |
| C.IB.491. | (CH₃O)₂CH-CH₂- | C(=O)CH₃ |
| C.IB.492. | (C₂H₅O)₂CH-CH₂- | C(=O)CH₃ |
| C.IB.493. | C(=O)CH₃ | C(=O)CH₃ |
| C.IB.494. | C(=O)CH₂-CH₃ | C(=O)CH₃ |
| C.IB.495. | C(=O)CF₃ | C(=O)CH₃ |
| C.IB.496. | C(=O)CCl₃ | C(=O)CH₃ |
| C.IB.497. | C(=O)CH₂-CH₂-CH₃ | C(=O)CH₃ |
| C.IB.498. | C(=O)C-(CH₃)₃ | C(=O)CH₃ |
| C.IB.499. | C(=O)CH₂-C₆H₅ | C(=O)CH₃ |
| C.IB.500. | C(=O)CH₂-CH₂-CH₃ | C(=O)CH₃ |
| C.IB.501. | H | C(=O)C₂H₅ |
| C.IB.502. | CH₃ | C(=O)C₂H₅ |
| C.IB.503. | CH₃CH₂- | C(=O)C₂H₅ |
| C.IB.504. | (CH₃)₂CH- | C(=O)C₂H₅ |
| C.IB.505. | CH₃CH₂CH₂- | C(=O)C₂H₅ |
| C.IB.506. | n-C₄H₉ | C(=O)C₂H₅ |
| C.IB.507. | (CH₃)₃C- | C(=O)C₂H₅ |
| C.IB.508. | (CH₃)₂CH-CH₂- | C(=O)C₂H₅ |
| C.IB.509. | n-C₅H₁₁ | C(=O)C₂H₅ |
| C.IB.510. | (CH₃)₂CH-CH₂-CH₂- | C(=O)C₂H₅ |
| C.IB.511. | (C₂H₅)₂-CH- | C(=O)C₂H₅ |
| C.IB.512. | (CH₃)₃C-CH₂- | C(=O)C₂H₅ |
| C.IB.513. | (CH₃)₃C-CH₂-CH₂- | C(=O)C₂H₅ |
| C.IB.514. | C₂H₅CH(CH₃)-CH₂- | C(=O)C₂H₅ |
| C.IB.515. | CH₃-CH₂-C(CH₃)₂- | C(=O)C₂H₅ |
| C.IB.516. | (CH₃)₂CH-CH(CH₃)- | C(=O)C₂H₅ |
| C.IB.517. | (CH₃)₃C-CH(CH₃)- | C(=O)C₂H₅ |
| C.IB.518. | (CH₃)₂CH-CH₂-CH(CH₃)- | C(=O)C₂H₅ |
| C.IB.519. | CH₃-CH₂-C(CH₃)(C₂H₅)- | C(=O)C₂H₅ |
| C.IB.520. | CH₃-CH₂-CH₂-C(CH₃)₂- | C(=O)C₂H₅ |
| C.IB.521. | C₂H₅-CH₂-CH(CH₃)-CH₂- | C(=O)C₂H₅ |
| C.IB.522. | cyclopropyl | C(=O)C₂H₅ |
| C.IB.523. | cyclopropyl-CH₂- | C(=O)C₂H₅ |
| C.IB.524. | cyclopropyl-CH(CH₃)- | C(=O)C₂H₅ |
| C.IB.525. | cyclobutyl | C(=O)C₂H₅ |
| C.IB.526. | cyclopentyl | C(=O)C₂H₅ |
| C.IB.527. | cyclohexyl | C(=O)C₂H₅ |
| C.IB.528. | HC≡ C-CH₂- | C(=O)C₂H₅ |
| C.IB.529. | HC≡ C-CH(CH₃)- | C(=O)C₂H₅ |
| C.IB.530. | HC≡ C-C(CH₃)₂- | C(=O)C₂H₅ |
| C.IB.531. | HC≡ C-C(CH₃)(C₂H₅)- | C(=O)C₂H₅ |
| C.IB.532. | HC≡ C-C(CH₃)(C₃H₇)- | C(=O)C₂H₅ |
| C.IB.533. | CH₂=CH-CH₂- | C(=O)C₂H₅ |
| C.IB.534. | H₂C=CH-CH(CH₃)- | C(=O)C₂H₅ |
| C.IB.535. | H₂C=CH-C(CH₃)₂- | C(=O)C₂H₅ |
| C.IB.536. | H₂C=CH-C(C₂H₅)(CH₃)- | C(=O)C₂H₅ |
| C.IB.537. | C₆H₅-CH₂- | C(=O)C₂H₅ |
| C.IB.538. | 4-(CH₃)₃C-C₆H₄-CH₂- | C(=O)C₂H₅ |
| C.IB.539. | C₆H₅-CH₂- | C(=O)C₂H₅ |
| C.IB.540. | 4-(CH₃)₃C-C₆H₄-CH₂- | C(=O)C₂H₅ |
| C.IB.541. | 4-Cl-C₆H₄-CH₂- | C(=O)C₂H₅ |
| C.IB.542. | 3-(CH₃O)-C₆H₄-CH₂- | C(=O)C₂H₅ |
| C.IB.543. | 4-(CH₃O)-C₆H₄-CH₂- | C(=O)C₂H₅ |
| C.IB.544. | 2-(CH₃O)-C₆H₄-CH₂- | C(=O)C₂H₅ |
| C.IB.545. | 3-Cl-C₆H₄-CH₂- | C(=O)C₂H₅ |
| C.IB.546. | 2-Cl-C₆H₄-CH₂- | C(=O)C₂H₅ |
| C.IB.547. | 4-(F₃C)-C₆H₄-CH₂- | C(=O)C₂H₅ |
| C.IB.548. | NC-CH₂- | C(=O)C₂H₅ |
| C.IB.549. | NC-CH₂-CH₂- | C(=O)C₂H₅ |
| C.IB.550. | NC-CH₂-CH(CH₃)- | C(=O)C₂H₅ |
| C.IB.551. | NC-CH₂-C(CH₃)₂- | C(=O)C₂H₅ |
| C.IB.552. | NC-CH₂-CH₂-CH₂- | C(=O)C₂H₅ |
| C.IB.553. | FH₂C-CH₂- | C(=O)C₂H₅ |
| C.IB.554. | ClH₂C-CH₂- | C(=O)C₂H₅ |
| C.IB.555. | BrH₂C-CH₂- | C(=O)C₂H₅ |
| C.IB.556. | FH₂C-CH(CH₃)- | C(=O)C₂H₅ |
| C.IB.557. | ClH₂C-CH(CH₃)- | C(=O)C₂H₅ |
| C.IB.558. | BrH₂C-CH(CH₃)- CH₃ | C(=O)C₂H₅ |
| C.IB.559. | F₂HC-CH₂- | C(=O)C₂H₅ |
| C.IB.560. | F₃C-CH₂- | C(=O)C₂H₅ |
| C.IB.561. | FH₂C-CH₂-CH₂- | C(=O)C₂H₅ |
| C.IB.562. | ClH₂C-CH₂-CH₂- | C(=O)C₂H₅ |
| C.IB.563. | BrH₂C-CH₂-CH₂- | C(=O)C₂H₅ |
| C.IB.564. | F₂HC-CH₂-CH₂- | C(=O)C₂H₅ |
| C.IB.565. | F₃C-CH₂-CH₂- | C(=O)C₂H₅ |
| C.IB.566. | CH₃-O-CH₂-CH₂- | C(=O)C₂H₅ |
| C.IB.567. | CH₃-S-CH₂-CH₂- | C(=O)C₂H₅ |
| C.IB.568. | CH₃-SO₂-CH₂-CH₂- | C(=O)C₂H₅ |
| C.IB.569. | C₂H₅-O-CH₂-CH₂- | C(=O)C₂H₅ |
| C.IB.570. | (CH₃)₂CH-O-CH₂-CH₂- | C(=O)C₂H₅ |
| C.IB.571. | C₂H₅-S-CH₂-CH₂- | C(=O)C₂H₅ |
| C.IB.572. | C₂H₅-SO₂-CH₂-CH₂- | C(=O)C₂H₅ |
| C.IB.573. | (CH₃)₂N-CH₂-CH₂- | C(=O)C₂H₅ |
| C.IB.574. | (C₂H₅)₂N-CH₂-CH₂- | C(=O)C₂H₅ |
| C.IB.575. | [(CH₃)₂CH]₂N-CH₂-CH₂- | C(=O)C₂H₅ |
| C.IB.576. | CH₃-O-CH₂-CH(CH₃)- | C(=O)C₂H₅ |
| C.IB.577. | CH₃-S-CH₂-CH(CH₃)- | C(=O)C₂H₅ |
| C.IB.578. | CH₃-SO₂-CH₂-CH(CH₃)- | C(=O)C₂H₅ |
| C.IB.579. | C₂H₅-O-CH₂-CH(CH₃)- | C(=O)C₂H₅ |
| C.IB.580. | C₂H₅-S-CH₂-CH(CH₃)- | C(=O)C₂H₅ |
| C.IB.581. | C₂H₅-SO₂-CH₂-CH(CH₃)- | C(=O)C₂H₅ |
| C.IB.582. | (CH₃)₂N-CH₂-CH(CH₃)- | C(=O)C₂H₅ |
| C.IB.583. | (C₂H₅)₂N-CH₂-CH(CH₃)- | C(=O)C₂H₅ |
| C.IB.584. | [(CH₃)₂CH]₂N-CH₂-CH(CH₃)- | C(=O)C₂H₅ |
| C.IB.585. | CH₃-O-CH(CH₃)-CH₂- | C(=O)C₂H₅ |
| C.IB.586. | CH₃-S-CH(CH₃)-CH₂- | C(=O)C₂H₅ |
| C.IB.587. | CH₃-SO₂-CH(CH₃)-CH₂- | C(=O)C₂H₅ |
| C.IB.588. | C₂H₅-O-CH(CH₃)-CH₂- | C(=O)C₂H₅ |
| C.IB.589. | C₂H₅-S-CH(CH₃)-CH₂- | C(=O)C₂H₅ |
| C.IB.590. | C₂H₅-SO₂-CH(CH₃)-CH₂- | C(=O)C₂H₅ |
| C.IB.591. | (CH₃)₂N-CH(CH₃)-CH₂- | C(=O)C₂H₅ |
| C.IB.592. | (C₂H₅)₂N-CH(CH₃)-CH₂- | C(=O)C₂H₅ |
| C.IB.593. | [(CH₃)₂CH]₂N-CH(CH₃)-CH₂- | C(=O)C₂H₅ |
| C.IB.594. | CH₃-O-CH₂-CH₂-CH₂- | C(=O)C₂H₅ |
| C.IB.595. | CH₃-S-CH₂-CH₂-CH₂- | C(=O)C₂H₅ |
| C.IB.596. | CH₃-SO₂-CH₂-CH₂-CH₂- | C(=O)C₂H₅ |
| C.IB.597. | C₂H₅-O-CH₂-CH₂-CH₂- | C(=O)C₂H₅ |
| C.IB.598. | C₂H₅-S-CH₂-CH₂-CH₂- | C(=O)C₂H₅ |
| C.IB.599. | C₂H₅-SO₂-CH₂-CH₂-CH₂- | C(=O)C₂H₅ |
| C.IB.600. | (CH₃)₂N-CH₂-CH₂-CH₂- | C(=O)C₂H₅ |
| C.IB.601. | (C₂H₅)₂N-CH₂-CH₂-CH₂- | C(=O)C₂H₅ |
| C.IB.602. | CH₃-O-CH₂-C(CH₃)₂- | C(=O)C₂H₅ |
| C.IB.603. | CH₃-S-CH₂-C(CH₃)₂- | C(=O)C₂H₅ |
| C.IB.604. | CH₃-SO₂-CH₂-C(CH₃)₂- | C(=O)C₂H₅ |
| C.IB.605. | C₂H₅-O-CH₂-C(CH₃)₂- | C(=O)C₂H₅ |
| C.IB.606. | C₂H₅-S-CH₂-C(CH₃)₂- | C(=O)C₂H₅ |
| C.IB.607. | C₂H₅-SO₂-CH₂-C(CH₃)₂- | C(=O)C₂H₅ |
| C.IB.608. | (CH₃)₂N-CH₂-C(CH₃)₂- | C(=O)C₂H₅ |
| C.IB.609. | (C₂H₅)₂N-CH₂-C(CH₃)₂- | C(=O)C₂H₅ |
| C.IB.610. | [(CH₃)₂CH]₂N-CH₂-C(CH₃)₂- | C(=O)C₂H₅ |
| C.IB.611. | Cl-CH₂-C≡ C-CH₂- | C(=O)C₂H₅ |
| C.IB.612. | CH₃-O-C(O)-CH₂ | C(=O)C₂H₅ |
| C.IB.613. | C₂H₅-O-C(O)-CH₂ | C(=O)C₂H₅ |
| C.IB.614. | CH₃-O-C(O)-CH(CH₃)- | C(=O)C₂H₅ |
| C.IB.615. | C₂H₅-O-C(O)-CH(CH₃)- | C(=O)C₂H₅ |
| C.IB.616. | (CH₃O)₂CH-CH₂- | C(=O)C₂H₅ |
| C.IB.617. | (C₂H₅O)₂CH-CH₂- | C(=O)C₂H₅ |
| C.IB.618. | C(=O)CH₃ | C(=O)C₂H₅ |
| C.IB.619. | C(=O)CH₂-CH₃ | C(=O)C₂H₅ |
| C.IB.620. | C(=O)CF₃ | C(=O)C₂H₅ |
| C.IB.621. | C(=O)CCl₃ | C(=O)C₂H₅ |
| C.IB.622. | C(=O)CH₂-CH₂-CH₃ | C(=O)C₂H₅ |
| C.IB.623. | C(=O)C-(CH₃)₃ | C(=O)C₂H₅ |
| C.IB.624. | C(=O)CH₂-C₆H₅ | C(=O)C₂H₅ |
| C.IB.625. | C(=O)CH₂-CH₂-CH₃ | C(=O)C₂H₅ |
| C.IB.626. | H | C(=O)C(CH₃)₃ |
| C.IB.627. | CH₃ | C(=O)C(CH₃)₃ |
| C.IB.628. | CH₃CH₂- | C(=O)C(CH₃)₃ |
| C.IB.629. | (CH₃)₂CH- | C(=O)C(CH₃)₃ |
| C.IB.630. | CH₃CH₂CH₂- | C(=O)C(CH₃)₃ |
| C.IB.631. | n-C₄H₉ | C(=O)C(CH₃)₃ |
| C.IB.632. | (CH₃)₃C- | C(=O)C(CH₃)₃ |
| C.IB.633. | (CH₃)₂CH-CH₂- | C(=O)C(CH₃)₃ |
| C.IB.634. | n-C₅H₁₁ | C(=O)C(CH₃)₃ |
| C.IB.635. | (CH₃)₂CH-CH₂-CH₂- | C(=O)C(CH₃)₃ |
| C.IB.636. | (C₂H₅)₂-CH- | C(=O)C(CH₃)₃ |
| C.IB.637. | (CH₃)₃C-CH₂- | C(=O)C(CH₃)₃ |
| C.IB.638. | (CH₃)₃C-CH₂-CH₂- | C(=O)C(CH₃)₃ |
| C.IB.639. | C₂H₅CH(CH₃)-CH₂- | C(=O)C(CH₃)₃ |
| C.IB.640. | CH₃-CH₂-C(CH₃)₂- | C(=O)C(CH₃)₃ |
| C.IB.641. | (CH₃)₂CH-CH(CH₃)- | C(=O)C(CH₃)₃ |
| C.IB.642. | (CH₃)₃C-CH(CH₃)- | C(=O)C(CH₃)₃ |
| C.IB.643. | (CH₃)₂CH-CH₂-CH(CH₃)- | C(=O)C(CH₃)₃ |
| C.IB.644. | CH₃-CH₂-C(CH₃)(C₂H₅)- | C(=O)C(CH₃)₃ |
| C.IB.645. | CH₃-CH₂-CH₂-C(CH₃)₂- | C(=O)C(CH₃)₃ |
| C.IB.646. | C₂H₅-CH₂-CH(CH₃)-CH₂- | C(=O)C(CH₃)₃ |
| C.IB.647. | cyclopropyl | C(=O)C(CH₃)₃ |
| C.IB.648. | cyclopropyl-CH₂- | C(=O)C(CH₃)₃ |
| C.IB.649. | cyclopropyl-CH(CH₃)- | C(=O)C(CH₃)₃ |
| C.IB.650. | cyclobutyl | C(=O)C(CH₃)₃ |
| C.IB.651. | cyclopentyl | C(=O)C(CH₃)₃ |
| C.IB.652. | cyclohexyl | C(=O)C(CH₃)₃ |
| C.IB.653. | HC≡ C-CH₂- | C(=O)C(CH₃)₃ |
| C.IB.654. | HC≡ C-CH(CH₃)- | C(=O)C(CH₃)₃ |
| C.IB.655. | HC≡ C-C(CH₃)₂- | C(=O)C(CH₃)₃ |
| C.IB.656. | HC≡ C-C(CH₃)(C₂H₅)- | C(=O)C(CH₃)₃ |
| C.IB.657. | HC= C-C(CH₃)(C₃H₇)- | C(=O)C(CH₃)₃ |
| C.IB.658. | CH₂=CH-CH₂- | C(=O)C(CH₃)₃ |
| C.IB.659. | H₂C=CH-CH(CH₃)- | C(=O)C(CH₃)₃ |
| C.IB.660. | H₂C=CH-C(CH₃)₂- | C(=O)C(CH₃)₃ |
| C.IB.661. | H₂C=CH-C(C₂H₅)(CH₃)- | C(=O)C(CH₃)₃ |
| C.IB.662. | C₆H₅-CH₂- | C(=O)C(CH₃)₃ |
| C.IB.663. | 4-(CH₃)₃C-C₆H₄-CH₂- | C(=O)C(CH₃)₃ |
| C.IB.664. | C₆H₅-CH₂- | C(=O)C(CH₃)₃ |
| C.IB.665. | 4-(CH₃)₃C-C₆H₄-CH₂- | C(=O)C(CH₃)₃ |
| C.IB.666. | 4-Cl-C₆H₄-CH₂- | C(=O)C(CH₃)₃ |
| C.IB.667. | 3-(CH₃O)-C₆H₄-CH₂- | C(=O)C(CH₃)₃ |
| C.IB.668. | 4-(CH₃O)-C₆H₄-CH₂- | C(=O)C(CH₃)₃ |
| C.IB.669. | 2-(CH₃O)-C₆H₄-CH₂- | C(=O)C(CH₃)₃ |
| C.IB.670. | 3-Cl-C₆H₄-CH₂- | C(=O)C(CH₃)₃ |
| C.IB.671. | 2-Cl-C₆H₄-CH₂- | C(=O)C(CH₃)₃ |
| C.IB.672. | 4-(F₃C)-C₆H₄-CH₂- | C(=O)C(CH₃)₃ |
| C.IB.673. | NC-CH₂- | C(=O)C(CH₃)₃ |
| C.IB.674. | NC-CH₂-CH₂- | C(=O)C(CH₃)₃ |
| C.IB.675. | NC-CH₂-CH(CH₃)- | C(=O)C(CH₃)₃ |
| C.IB.676. | NC-CH₂-C(CH₃)₂- | C(=O)C(CH₃)₃ |
| C.IB.677. | NC-CH₂-CH₂-CH₂- | C(=O)C(CH₃)₃ |
| C.IB.678. | FH₂C-CH₂- | C(=O)C(CH₃)₃ |
| C.IB.679. | ClH₂C-CH₂- | C(=O)C(CH₃)₃ |
| C.IB.680. | BrH₂C-CH₂- | C(=O)C(CH₃)₃ |
| C.IB.681. | FH₂C-CH(CH₃)- | C(=O)C(CH₃)₃ |
| C.IB.682. | ClH₂C-CH(CH₃)- | C(=O)C(CH₃)₃ |
| C.IB.683. | BrH₂C-CH(CH₃)- CH₃ | C(=O)C(CH₃)₃ |
| C.IB.684. | F₂HC-CH₂- | C(=O)C(CH₃)₃ |
| C.IB.685. | F₃C-CH₂- | C(=O)C(CH₃)₃ |
| C.IB.686. | FH₂C-CH₂-CH₂- | C(=O)C(CH₃)₃ |
| C.IB.687. | ClH₂C-CH₂-CH₂- | C(=O)C(CH₃)₃ |
| C.IB.688. | BrH₂C-CH₂-CH₂- | C(=O)C(CH₃)₃ |
| C.IB.689. | F₂HC-CH₂-CH₂- | C(=O)C(CH₃)₃ |
| C.IB.690. | F₃C-CH₂-CH₂- | C(=O)C(CH₃)₃ |
| C.IB.691. | CH₃-O-CH₂-CH₂- | C(=O)C(CH₃)₃ |
| C.IB.692. | CH₃-S-CH₂-CH₂- | C(=O)C(CH₃)₃ |
| C.IB.693. | CH₃-SO₂-CH₂-CH₂- | C(=O)C(CH₃)₃ |
| C.IB.694. | C₂H₅-O-CH₂-CH₂- | C(=O)C(CH₃)₃ |
| C.IB.695. | (CH₃)₂CH-O-CH₂-CH₂- | C(=O)C(CH₃)₃ |
| C.IB.696. | C₂H₅-S-CH₂-CH₂- | C(=O)C(CH₃)₃ |
| C.IB.697. | C₂H₅-SO₂-CH₂-CH₂- | C(=O)C(CH₃)₃ |
| C.IB.698. | (CH₃)₂N-CH₂-CH₂- | C(=O)C(CH₃)₃ |
| C.IB.699. | (C₂H₅)₂N-CH₂-CH₂- | C(=O)C(CH₃)₃ |
| C.IB.700. | [(CH₃)₂CH]₂N-CH₂-CH₂- | C(=O)C(CH₃)₃ |
| C.IB.701. | CH₃-O-CH₂-CH(CH₃)- | C(=O)C(CH₃)₃ |
| C.IB.702. | CH₃-S-CH₂-CH(CH₃)- | C(=O)C(CH₃)₃ |
| C.IB.703. | CH₃-SO₂-CH₂-CH(CH₃)- | C(=O)C(CH₃)₃ |
| C.IB.704. | C₂H₅-O-CH₂-CH(CH₃)- | C(=O)C(CH₃)₃ |
| C.IB.705. | C₂H₅-S-CH₂-CH(CH₃)- | C(=O)C(CH₃)₃ |
| C.IB.706. | C₂H₅-SO₂-CH₂-CH(CH₃)- | C(=O)C(CH₃)₃ |
| C.IB.707. | (CH₃)₂N-CH₂-CH(CH₃)- | C(=O)C(CH₃)₃ |
| C.IB.708. | (C₂H₅)₂N-CH₂-CH(CH₃)- | C(=O)C(CH₃)₃ |
| C.IB.709. | [(CH₃)₂CH]₂N-CH₂-CH(CH₃)- | C(=O)C(CH₃)₃ |
| C.IB.710. | CH₃-O-CH(CH₃)-CH | C(=O)C(CH₃)₃ |
| C.IB.711. | CH₃-S-CH(CH₃)-CH₂- | C(=O)C(CH₃)₃ |
| C.IB.712. | CH₃-SO₂-CH(CH₃)-CH₂- | C(=O)C(CH₃)₃ |
| C.IB.713. | C₂H₅-O-CH(CH₃)-CH₂- | C(=O)C(CH₃)₃ |
| C.IB.714. | C₂H₅-S-CH(CH₃)-CH₂- | C(=O)C(CH₃)₃ |
| C.IB.715. | C₂H₅-SO₂-CH(CH₃)-CH₂- | C(=O)C(CH₃)₃ |
| C.IB.716. | (CH₃)₂N-CH(CH₃)-CH₂- | C(=O)C(CH₃)₃ |
| C.IB.717. | (C₂H₅)₂N-CH(CH₃)-CH₂- | C(=O)C(CH₃)₃ |
| C.IB.718. | [(CH₃)₂CH]₂N-CH(CH₃)-CH₂- | C(=O)C(CH₃)₃ |
| C.IB.719. | CH₃-O-CH₂-CH₂-CH₂- | C(=O)C(CH₃)₃ |
| C.IB.720. | CH₃-S-CH₂-CH₂-CH₂- | C(=O)C(CH₃)₃ |
| C.IB.721. | CH₃-SO₂-CH₂-CH₂-CH₂- | C(=O)C(CH₃)₃ |
| C.IB.722. | C₂H₅-O-CH₂-CH₂-CH₂- | C(=O)C(CH₃)₃ |
| C.IB.723. | C₂H₅-S-CH₂-CH₂-CH₂- | C(=O)C(CH₃)₃ |
| C.IB.724. | C₂H₅-SO₂-CH₂-CH₂-CH₂- | C(=O)C(CH₃)₃ |
| C.IB.725. | (CH₃)₂N-CH₂-CH₂-CH₂- | C(=O)C(CH₃)₃ |
| C.IB.726. | (C₂H₅)₂N-CH₂-CH₂-CH₂- | C(=O)C(CH₃)₃ |
| C.IB.727. | CH₃-O-CH₂-C(CH₃)₂- | C(=O)C(CH₃)₃ |
| C.IB.728. | CH₃-S-CH₂-C(CH₃)₂- | C(=O)C(CH₃)₃ |
| C.IB.729. | CH₃-SO₂-CH₂-C(CH₃)₂- | C(=O)C(CH₃)₃ |
| C.IB.730. | C₂H₅-O-CH₂-C(CH₃)₂- | C(=O)C(CH₃)₃ |
| C.IB.731. | C₂H₅-S-CH₂-C(CH₃)₂- | C(=O)C(CH₃)₃ |
| C.IB.732. | C₂H₅-SO₂-CH₂-C(CH₃)₂- | C(=O)C(CH₃)₃ |
| C.IB.733. | (CH₃)₂N-CH₂-C(CH₃)₂- | C(=O)C(CH₃)₃ |
| C.IB.734. | (C₂H₅)₂N-CH₂-C(CH₃)₂- | C(=O)C(CH₃)₃ |
| C.IB.735. | [(CH₃)₂CH]₂N-CH₂-C(CH₃)₂- | C(=O)C(CH₃)₃ |
| C.IB.736. | Cl-CH₂-C≡ C-CH₂- | C(=O)C(CH₃)₃ |
| C.IB.737. | CH₃-O-C(O)-CH₂ | C(=O)C(CH₃)₃ |
| C.IB.738. | C₂H₅-O-C(O)-CH₂ | C(=O)C(CH₃)₃ |
| C.IB.739. | CH₃-O-C(O)-CH(CH₃)- | C(=O)C(CH₃)₃ |
| C.IB.740. | C₂H₅-O-C(O)-CH(CH₃)- | C(=O)C(CH₃)₃ |
| C.IB.741. | (CH₃O)₂CH-CH₂- | C(=O)C(CH₃)₃ |
| C.IB.742. | (C₂H₅O)₂CH-CH₂- | C(=O)C(CH₃)₃ |
| C.IB.743. | C(=O)CH₃ | C(=O)C(CH₃)₃ |
| C.IB.744. | C(=O)CH₂-CH₃ | C(=O)C(CH₃)₃ |
| C.IB.745. | C(=O)CF₃ | C(=O)C(CH₃)₃ |
| C.IB.746. | C(=O)CCl₃ | C(=O)C(CH₃)₃ |
| C.IB.747. | C(=O)CH₂-CH₂-CH₃ | C(=O)C(CH₃)₃ |
| C.IB.748. | C(=O)C-(CH₃)₃ | C(=O)C(CH₃)₃ |
| C.IB.749. | C(=O)CH₂-C₆H₅ | C(=O)C(CH₃)₃ |
| C.IB.750. | C(=O)CH₂-CH₂-CH₃ | C(=O)C(CH₃)₃ |
| C.IB.751. | H | C(=O)CF₃ |
| C.IB.752. | CH₃ | C(=O)CF₃ |
| C.IB.753. | CH₃CH₂- | C(=O)CF₃ |
| C.IB.754. | (CH₃)₂CH- | C(=O)CF₃ |
| C.IB.755. | CH₃CH₂CH₂- | C(=O)CF₃ |
| C.IB.756. | n-C₄H₉ | C(=O)CF₃ |
| C.IB.757. | (CH₃)₃C- | C(=O)CF₃ |
| C.IB.758. | (CH₃)₂CH-CH₂- | C(=O)CF₃ |
| C.IB.759. | n-C₅H₁₁ | C(=O)CF₃ |
| C.IB.760. | (CH₃)₂CH-CH₂-CH₂- | C(=O)CF₃ |
| C.IB.761. | (C₂H₅)₂-CH- | C(=O)CF₃ |
| C.IB.762. | (CH₃)₃C-CH₂- | C(=O)CF₃ |
| C.IB.763. | (CH₃)₃C-CH₂-CH₂- | C(=O)CF₃ |
| C.IB.764. | C₂H₅CH(CH₃)-CH₂- | C(=O)CF₃ |
| C.IB.765. | CH₃-CH₂-C(CH₃)₂- | C(=O)CF₃ |
| C.IB.766. | (CH₃)₂CH-CH(CH₃)- | C(=O)CF₃ |
| C.IB.767. | (CH₃)₃C-CH(CH₃)- | C(=O)CF₃ |
| C.IB.768. | (CH₃)₂CH-CH₂-CH(CH₃)- | C(=O)CF₃ |
| C.IB.769. | CH₃-CH₂-C(CH₃)(C₂H₅)- | C(=O)CF₃ |
| C.IB.770. | CH₃-CH₂-CH₂-C(CH₃)₂- | C(=O)CF₃ |
| C.IB.771. | C₂H₅-CH₂-CH(CH₃)-CH₂- | C(=O)CF₃ |
| C.IB.772. | cyclopropyl | C(=O)CF₃ |
| C.IB.773. | cyclopropyl-CH₂- | C(=O)CF₃ |
| C.IB.774. | cyclopropyl-CH(CH₃)- | C(=O)CF₃ |
| C.IB.775. | cyclobutyl | C(=O)CF₃ |
| C.IB.776. | cyclopentyl | C(=O)CF₃ |
| C.IB.777. | cyclohexyl | C(=O)CF₃ |
| C.IB.778. | HC≡ C-CH₂- | C(=O)CF₃ |
| C.IB.779. | HC≡ C-CH(CH₃)- | C(=O)CF₃ |
| C.IB.780. | HC≡ C-C(CH₃)₂- | C(=O)CF₃ |
| C.IB.781. | HC≡ C-C(CH₃)(C₂H₅)- | C(=O)CF₃ |
| C.IB.782. | HC≡ C-C(CH₃)(C₃H₇)- | C(=O)CF₃ |
| C.IB.783. | CH₂=CH-CH₂- | C(=O)CF₃ |
| C.IB.784. | H₂C=CH-CH(CH₃)- | C(=O)CF₃ |
| C.IB.785. | H₂C=CH-C(CH₃)₂- | C(=O)CF₃ |
| C.IB.786. | H₂C=CH-C(C₂H₅)(CH₃)- | C(=O)CF₃ |
| C.IB.787. | C₆H₅-CH₂- | C(=O)CF₃ |
| C.IB.788. | 4-(CH₃)₃C-C₆H₄-CH₂- | C(=O)CF₃ |
| C.IB.789. | C₆H₅-CH₂- | C(=O)CF₃ |
| C.IB.790. | 4-(CH₃)₃C-C₆H₄-CH₂- | C(=O)CF₃ |
| C.IB.791. | 4-Cl-C₆H₄=CH₂- | C(=O)CF₃ |
| C.IB.792. | 3-(CH₃O)-C₆H₄-CH₂- | C(=O)CF₃ |
| C.IB.793. | 4-(CH₃O)-C₆H₄-CH₂- | C(=O)CF₃ |
| C.IB.794. | 2-(CH₃O)-C₆H₄-CH₂- | C(=O)CF₃ |
| C.IB.795. | 3-Cl-C₆H₄-CH₂- | C(=O)CF₃ |
| C.IB.796. | 2-Cl-C₆H₄-CH₂- | C(=O)CF₃ |
| C.IB.797. | 4-(F₃C)-C₆H₄-CH₂- | C(=O)CF₃ |
| C.IB.798. | NC-CH₂- | C(=O)CF₃ |
| C.IB.799. | NC-CH₂-CH₂- | C(=O)CF₃ |
| C.IB.800. | NC-CH₂-CH(CH₃)- | C(=O)CF₃ |
| C.IB.801. | NC-CH₂-C(CH₃)₂- | C(=O)CF₃ |
| C.IB.802. | NC-CH₂-CH₂-CH₂- | C(=O)CF₃ |
| C.IB.803. | FH₂C-CH₂- | C(=O)CF₃ |
| C.IB.804. | ClH₂C-CH₂- | C(=O)CF₃ |
| C.IB.805. | BrH₂C-CH₂- | C(=O)CF₃ |
| C.IB.806. | FH₂C-CH(CH₃)- | C(=O)CF₃ |
| C.IB.807. | ClH₂C-CH(CH₃)- | C(=O)CF₃ |
| C.IB.808. | BrH₂C-CH(CH₃)- CH₃ | C(=O)CF₃ |
| C.IB.809. | F₂HC-CH₂- | C(=O)CF₃ |
| C.IB.810. | F₃C-CH₂- | C(=O)CF₃ |
| C.IB.811. | FH₂C-CH₂-CH₂- | C(=O)CF₃ |
| C.IB.812. | ClH₂C-CH₂-CH₂- | C(=O)CF₃ |
| C.IB.813. | BrH₂C-CH₂-CH₂- | C(=O)CF₃ |
| C.IB.814. | F₂HC-CH₂-CH₂- | C(=O)CF₃ |
| C.IB.815. | F₃C-CH₂-CH₂- | C(=O)CF₃ |
| C.IB.816. | CH₃-O-CH₂-CH₂- | C(=O)CF₃ |
| C.IB.817. | CH₃-S-CH₂-CH₂- | C(=O)CF₃ |
| C.IB.818. | CH₃-SO₂-CH₂-CH₂- | C(=O)CF₃ |
| C.IB.819. | C₂H₅-O-CH₂-CH₂- | C(=O)CF₃ |
| C.IB.820. | (CH₃)₂CH-O-CH₂-CH₂- | C(=O)CF₃ |
| C.IB.821. | C₂H₅-S-CH₂-CH₂- | C(=O)CF₃ |
| C.IB.822. | C₂H₅-SO₂-CH₂-CH₂- | C(=O)CF₃ |
| C.IB.823. | (CH₃)₂N-CH₂-CH₂- | C(=O)CF₃ |
| C.IB.824. | (C₂H₅)₂N-CH₂-CH₂- | C(=O)CF₃ |
| C.IB.825. | [(CH₃)₂CH]₂N-CH₂-CH₂- | C(=O)CF₃ |
| C.IB.826. | CH₃-O-CH₂-CH(CH₃)- | C(=O)CF₃ |
| C.IB.827. | CH₃-S-CH₂-CH(CH₃)- | C(=O)CF₃ |
| C.IB.828. | CH₃-SO₂-CH₂-CH(CH₃)- | C(=O)CF₃ |
| C.IB.829. | C₂H₅-O-CH₂-CH(CH₃)- | C(=O)CF₃ |
| C.IB.830. | C₂H₅-S-CH₂-CH(CH₃)- | C(=O)CF₃ |
| C.IB.831. | C₂H₅-SO₂-CH₂-CH(CH₃)- | C(=O)CF₃ |
| C.IB.832. | (CH₃)₂N-CH₂-CH(CH₃)- | C(=O)CF₃ |
| C.IB.833. | (C₂H₅)₂N-CH₂-CH(CH₃)- | C(=O)CF₃ |
| C.IB.834. | [(CH₃)₂CH]₂N-CH₂-CH(CH₃)- | C(=O)CF₃ |
| C.IB.835. | CH₃-O-CH(CH₃)-CH₂- | C(=O)CF₃ |
| C.IB.836. | CH₃-S-CH(CH₃)-CH₂- | C(=O)CF₃ |
| C.IB.837. | CH₃-SO₂-CH(CH₃)-CH₂- | C(=O)CF₃ |
| C.IB.838. | C₂H₅-O-CH(CH₃)-CH₂- | C(=O)CF₃ |
| C.IB.839. | C₂H₅-S-CH(CH₃)-CH₂- | C(=O)CF₃ |
| C.IB.840. | C₂H₅-SO₂-CH(CH₃)-CH₂- | C(=O)CF₃ |
| C.IB.841. | (CH₃)₂N-CH(CH₃)-CH₂- | C(=O)CF₃ |
| C.IB.842. | (C₂H₅)₂N-CH(CH₃)-CH₂- | C(=O)CF₃ |
| C.IB.843. | [(CH₃)₂CH]₂N-CH(CH₃)-CH₂- | C(=O)CF₃ |
| C.IB.844. | CH₃-O-CH₂-CH₂-CH₂- | C(=O)CF₃ |
| C.IB.845. | CH₃-S-CH₂-CH₂-CH₂- | C(=O)CF₃ |
| C.IB.846. | CH₃-SO₂-CH₂-CH₂-CH₂- | C(=O)CF₃ |
| C.IB.847. | C₂H₅-O-CH₂-CH₂-CH₂- | C(=O)CF₃ |
| C.IB.848. | C₂H₅-S-CH₂-CH₂-CH₂- | C(=O)CF₃ |
| C.IB.849. | C₂H₅-SO₂-CH₂-CH₂-CH₂- | C(=O)CF₃ |
| C.IB.850. | (CH₃)₂N-CH₂-CH₂-CH₂- | C(=O)CF₃ |
| C.IB.851. | (C₂H₅)₂N-CH₂-CH₂-CH₂- | C(=O)CF₃ |
| C.IB.852. | CH₃-O-CH₂-C(CH₃)₂- | C(=O)CF₃ |
| C.IB.853. | CH₃-S-CH₂-C(CH₃)₂- | C(=O)CF₃ |
| C.IB.854. | CH₃-SO₂-CH₂-C(CH₃)₂- | C(=O)CF₃ |
| C.IB.855. | C₂H₅-O-CH₂-C(CH₃)₂- | C(=O)CF₃ |
| C.IB.856. | C₂H₅-S-CH₂-C(CH₃)₂- | C(=O)CF₃ |
| C.IB.857. | C₂H₅-SO₂-CH₂-C(CH₃)₂- | C(=O)CF₃ |
| C.IB.858. | (CH₃)₂N-CH₂-C(CH₃)₂- | C(=O)CF₃ |
| C.IB.859. | (C₂H₅)₂N-CH₂-C(CH₃)₂- | C(=O)CF₃ |
| C.IB.860. | [(CH₃)₂CH]₂N-CH₂-C(CH₃)₂- | C(=O)CF₃ |
| C.IB.861. | Cl-CH₂-C≡ C-CH₂- | C(=O)CF₃ |
| C.IB.862. | CH₃-O-C(O)-CH₂ | C(=O)CF₃ |
| C.IB.863. | C₂H₅-O-C(O)-CH₂ | C(=O)CF₃ |
| C.IB.864. | CH₃-O-C(O)-CH(CH₃)- | C(=O)CF₃ |
| C.IB.865. | C₂H₅-O-C(O)-CH(CH₃)- | C(=O)CF₃ |
| C.IB.866. | (CH₃O)₂CH-CH₂- | C(=O)CF₃ |
| C.IB.867. | (C₂H₅O)₂CH-CH₂- | C(=O)CF₃ |
| C.IB.868. | C(=O)CH₃ | C(=O)CF₃ |
| C.IB.869. | C(=O)CH₂-CH₃ | C(=O)CF₃ |
| C.IB.870. | C(=O)CF₃ | C(=O)CF₃ |
| C.IB.871. | C(=O)CCl₃ | C(=O)CF₃ |
| C.IB.872. | C(=O)CH₂-CH₂-CH₃ | C(=O)CF₃ |
| C.IB.873. | C(=O)C-(CH₃)₃ | C(=O)CF₃ |
| C.IB.874. | C(=O)CH₂-C₆H₅ | C(=O)CF₃ |
| C.IB.875. | C(=O)CH₂-CH₂-CH₃ | C(=O)CF₃ |
| C.IB.876. | H | C(=O)CCl₃ |
| C.IB.877. | CH₃ | C(=O)CCl₃ |
| C.IB.878. | CH₃CH₂- | C(=O)CCl₃ |
| C.IB.879. | (CH₃)₂CH- | C(=O)CCl₃ |
| C.IB.880. | CH₃CH₂CH₂- | C(=O)CCl₃ |
| C.IB.881. | n-C₄H₉ | C(=O)CCl₃ |
| C.IB.882. | (CH₃)₃C- | C(=O)CCl₃ |
| C.IB.883. | (CH₃)₂CH-CH₂- | C(=O)CCl₃ |
| C.IB.884. | n-C₅H₁₁ | C(=O)CCl₃ |
| C.IB.885. | (CH₃)₂CH-CH₂-CH₂- | C(=O)CCl₃ |
| C.IB.886. | (C₂H₅)₂-CH- | C(=O)CCl₃ |
| C.IB.887. | (CH₃)₃C-CH₂- | C(=O)CCl₃ |
| C.IB.888. | (CH₃)₃C-CH₂-CH₂- | C(=O)CCl₃ |
| C.IB.889. | C₂H₅CH(CH₃)-CH₂- | C(=O)CCl₃ |
| C.IB.890. | CH₃-CH₂-C(CH₃)₂- | C(=O)CCl₃ |
| C.IB.891. | (CH₃)₂CH-CH(CH₃)- | C(=O)CCl₃ |
| C.IB.892. | (CH₃)₃C-CH(CH₃)- | C(=O)CCl₃ |
| C.IB.893. | (CH₃)₂CH-CH₂-CH(CH₃)- | C(=O)CCl₃ |
| C.IB.894. | CH₃-CH₂-C(CH₃)(C₂H₅)- | C(=O)CCl₃ |
| C.IB.895. | CH₃-CH₂-CH₂-C(CH₃)₂- | C(=O)CCl₃ |
| C.IB.896. | C₂H₅-CH₂-CH(CH₃)-CH₂- | C(=O)CCl₃ |
| C.IB.897. | cyclopropyl | C(=O)CCl₃ |
| C.IB.898. | cyclopropyl-CH₂- | C(=O)CCl₃ |
| C.IB.899. | cyclopropyl-CH(CH₃)- | C(=O)CCl₃ |
| C.IB.900. | cyclobutyl | C(=O)CCl₃ |
| C.IB.901. | cyclopentyl | C(=O)CCl₃ |
| C.IB.902. | cyclohexyl | C(=O)CCl₃ |
| C.IB.903. | HC≡ C-CH₂- | C(=O)CCl₃ |
| C.IB.904. | HC≡ C-CH(CH₃)- | C(=O)CCl₃ |
| C.IB.905. | HC≡ C-C(CH₃)₂- | C(=O)CCl₃ |
| C.IB.906. | HC≡ C-C(CH₃)(C₂H₅)- | C(=O)CCl₃ |
| C.IB.907. | HC≡ C-C(CH₃)(C₃H₇)- | C(=O)CCl₃ |
| C.IB.908. | CH₂=CH-CH₂- | C(=O)CCl₃ |
| C.IB.909. | H₂C=CH-CH(CH₃)- | C(=O)CCl₃ |
| C.IB.910. | H₂C=CH-C(CH₃)₂- | C(=O)CCl₃ |
| C.IB.911. | H₂C=CH-C(C₂H₅)(CH₃)- | C(=O)CCl₃ |
| C.IB.912. | C₆H₅-CH₂- | C(=O)CCl₃ |
| C.IB.913. | 4-(CH₃)₃C-C₆H₄-CH₂- | C(=O)CCl₃ |
| C.IB.914. | C₆H₅-CH₂- | C(=O)CCl₃ |
| C.IB.915. | 4-(CH₃)₃C-C₆H₄-CH₂- | C(=O)CCl₃ |
| C.IB.916. | 4-Cl-C₆H₄-CH₂- | C(=O)CCl₃ |
| C.IB.917. | 3-(CH₃O)-C₆H₄-CH₂- | C(=O)CCl₃ |
| C.IB.918. | 4-(CH₃O)-C₆H₄-CH₂- | C(=O)CCl₃ |
| C.IB.919. | 2-(CH₃O)-C₆H₄-CH₂- | C(=O)CCl₃ |
| C.IB.920. | 3-Cl-C₆H₄-CH₂- | C(=O)CCl₃ |
| C.IB.921. | 2-Cl-C₆H₄-CH₂- | C(=O)CCl₃ |
| C.IB.922. | 4-(F₃C)-C₆H₄-CH₂- | C(=O)CCl₃ |
| C.IB.923. | NC-CH₂- | C(=O)CCl₃ |
| C.IB.924. | NC-CH₂-CH₂- | C(=O)CCl₃ |
| C.IB.925. | NC-CH₂-CH(CH₃)- | C(=O)CCl₃ |
| C.IB.926. | NC-CH₂-C(CH₃)₂- | C(=O)CCl₃ |
| C.IB.927. | NC-CH₂-CH₂-CH₂- | C(=O)CCl₃ |
| C.IB.928. | FH₂C-CH₂- | C(=O)CCl₃ |
| C.IB.929. | ClH₂C-CH₂- | C(=O)CCl₃ |
| C.IB.930. | BrH₂C-CH₂- | C(=O)CCl₃ |
| C.IB.931. | FH₂C-CH(CH₃)- | C(=O)CCl₃ |
| C.IB.932. | ClH₂C-CH(CH₃)- | C(=O)CCl₃ |
| C.IB.933. | BrH₂C-CH(CH₃)-CH₃ | C(=O)CCl₃ |
| C.IB.934. | F₂HC-CH₂- | C(=O)CCl₃ |
| C.IB.935. | F₃C-CH₂- | C(=O)CCl₃ |
| C.IB.936. | FH₂C-CH₂-CH₂- | C(=O)CCl₃ |
| C.IB.937. | ClH₂C-CH₂-CH₂- | C(=O)CCl₃ |
| C.IB.938. | BrH₂C-CH₂-CH₂- | C(=O)CCl₃ |
| C.IB.939. | F₂HC-CH₂-CH₂- | C(=O)CCl₃ |
| C.IB.940. | F₃C-CH₂-CH₂- | C(=O)CCl₃ |
| C.IB.941. | CH₃-O-CH₂-CH₂- | C(=O)CCl₃ |
| C.IB.942. | CH₃-S-CH₂-CH₂- | C(=O)CCl₃ |
| C.IB.943. | CH₃-SO₂-CH₂-CH₂- | C(=O)CCl₃ |
| C.IB.944. | C₂H₅-O-CH₂-CH₂- | C(=O)CCl₃ |
| C.IB.945. | (CH₃)₂CH-O-CH₂-CH₂- | C(=O)CCl₃ |
| C.IB.946. | C₂H₅-S-CH₂-CH₂- | C(=O)CCl₃ |
| C.IB.947. | C₂H₅-SO₂-CH₂-CH₂- | C(=O)CCl₃ |
| C.IB.948. | (CH₃)₂N-CH₂-CH₂- | C(=O)CCl₃ |
| C.IB.949. | (C₂H₅)₂N-CH₂-CH₂- | C(=O)CCl₃ |
| C.IB.950. | [(CH₃)₂CH]₂N-CH₂-CH₂- | C(=O)CCl₃ |
| C.IB.951. | CH₃-O-CH₂-CH(CH₃)- | C(=O)CCl₃ |
| C.IB.952. | CH₃-S-CH₂-CH(CH₃)- | C(=O)CCl₃ |
| C.IB.953. | CH₃-SO₂-CH₂-CH(CH₃)- | C(=O)CCl₃ |
| C.IB.954. | C₂H₅-O-CH₂-CH(CH₃)- | C(=O)CCl₃ |
| C.IB.955. | C₂H₅-S-CH₂-CH(CH₃)- | C(=O)CCl₃ |
| C.IB.956. | C₂H₅-SO₂-CH₂-CH(CH₃)- | C(=O)CCl₃ |
| C.IB.957. | (CH₃)₂N-CH₂-CH(CH₃)- | C(=O)CCl₃ |
| C.IB.958. | (C₂H₅)₂N-CH₂-CH(CH₃)- | C(=O)CCl₃ |
| C.IB.959. | [(CH₃)₂CH]₂N-CH₂-CH(CH₃)- | C(=O)CCl₃ |
| C.IB.960. | CH₃-O-CH(CH₃)-CH₂- | C(=O)CCl₃ |
| C.IB.961. | CH₃-S-CH(CH₃)-CH₂- | C(=O)CCl₃ |
| C.IB.962. | CH₃-SO₂-CH(CH₃)-CH₂- | C(=O)CCl₃ |
| C.IB.963. | C₂H₅-O-CH(CH₃)-CH₂- | C(=O)CCl₃ |
| C.IB.964. | C₂H₅-S-CH(CH₃)-CH₂- | C(=O)CCl₃ |
| C.IB.965. | C₂H₅-SO₂-CH(CH₃)-CH₂- | C(=O)CCl₃ |
| C.IB.966. | (CH₃)₂N-CH(CH₃)-CH₂- | C(=O)CCl₃ |
| C.IB.967. | (C₂H₅)₂N-CH(CH₃)-CH₂- | C(=O)CCl₃ |
| C.IB.968. | [(CH₃)₂CH]₂N-CH(CH₃)-CH₂- | C(=O)CCl₃ |
| C.IB.969. | CH₃-O-CH₂-CH₂-CH₂- | C(=O)CCl₃ |
| C.IB.970. | CH₃-S-CH₂-CH₂-CH₂- | C(=O)CCl₃ |
| C.IB.971. | CH₃-SO₂-CH₂-CH₂-CH₂- | C(=O)CCl₃ |
| C.IB.972. | C₂H₅-O-CH₂-CH₂-CH₂- | C(=O)CCl₃ |
| C.IB.973. | C₂H₅-S-CH₂-CH₂-CH₂- | C(=O)CCl₃ |
| C.IB.974. | C₂H₅-SO₂-CH₂-CH₂-CH₂- | C(=O)CCl₃ |
| C.IB.975. | (CH₃)₂N-CH₂-CH₂-CH₂- | C(=O)CCl₃ |
| C.IB.976. | (C₂H₅)₂N-CH₂-Ch₂-CH₂- | C(=O)CCl₃ |
| C.IB.977. | CH₃-O-CH₂-C(CH₃)₂- | C(=O)CCl₃ |
| C.IB.978. | CH₃-S-CH₂-C(CH₃)₂- | C(=O)CCl₃ |
| C.IB.979. | CH₃-SO₂-CH₂-C(CH₃)₂- | C(=O)CCl₃ |
| C.IB.980. | C₂H₅-O-CH₂-C(CH₃)₂- | C(=O)CCl₃ |
| C.IB.981. | C₂H₅-S-CH₂-C(CH₃)₂- | C(=O)CCl₃ |
| C.IB.982. | C₂H₅-SO₂-CH₂-C(CH₃)₂- | C(=O)CCl₃ |
| C.IB.983. | (CH₃)₂N-CH₂-C(CH₃)₂- | C(=O)CCl₃ |
| C.IB.984. | (C₂H₅)₂N-CH₂-C(CH₃)₂- | C(=O)CCl₃ |
| C.IB.985. | [(CH₃)₂CH]₂N-CH₂-C(CH₃)₂- | C(=O)CCl₃ |
| C.IB.986. | Cl-CH₂-C≡ C-CH₂- | C(=O)CCl₃ |
| C.IB.987. | CH₃-O-C(O)-CH₂ | C(=O)CCl₃ |
| C.IB.988. | C₂H₅-O-C(O)-CH₂ | C(=O)CCl₃ |
| C.IB.989. | CH₃-O-C(O)-CH(CH₃)- | C(=O)CCl₃ |
| C.IB.990. | C₂H₅-O-C(O)-CH(CH₃)- | C(=O)CCl₃ |
| C.IB.991. | (CH₃O)₂CH-CH₂- | C(=O)CCl₃ |
| C.IB.992. | (C₂H₅O)₂CH-CH₂- | C(=O)CCl₃ |
| C.IB.993. | C(=O)CH₃ | C(=O)CCl₃ |
| C.IB.994. | C(=O)CH₂-CH₃ | C(=O)CCl₃ |
| C.IB.995. | C(=O)CF₃ | C(=O)CCl₃ |
| C.IB.996. | C(=O)CCl₃ | C(=O)CCl₃ |
| C.IB.997. | C(=O)CH₂-CH₂-CH₃ | C(=O)CCl₃ |
| C.IB.998. | C(=O)C-(CH₃)₃ | C(=O)CCl₃ |
| C.IB.999. | C(=O)CH₂-C₆H₅ | C(=O)CCl₃ |
| C.IB.1000 | C(=O)CH₂-CH₂-CH₃ | C(=O)CCl₃ |
| C.IB.1001 | -CH₂CH₂- | |
| C.IB.1002 | -CH₂CH₂CH₂- | |
| C.IB.1003 | -CH₂CH₂CH₂- | |
| C.IB.1004 | -CH₂CH₂CH₂CH₂- | |
| C.IB.1005 | -CH₂CH₂CH₂CH₂CH₂- | |
| C.IB.1006 | -CH₂CH₂CH₂CH₂CH₂CH₂- | |
| C.IB.1007 | -CH₂CH₂OCH₂CH₂- | |
| C.IB.1008 | -CH₂CH₂-NH-CH₂CH₂- | |
| C.IB.1009 | -CH=CH-CH=CH- | |
| C.IB.1010 | -N=CH-CH=CH- | |
| C.IB.1011 | -CH=N-CH=CH- | |
| C.IB.1012 | -C(CH₃)=CH-CH=CH- | |
| C.IB.1013 | -C(CH₃)=CH-CH=C(CH₃)- | |
| C.IB.1014 | -CH=C(CH₃)-C(CH₃)=CH- | |
| C.IB.1015. | -CH=C(Cl)-C(Cl)=CH- | |
| C.IB.1016. | -CH=C(Br)-C(Br)=CH- | |

Isothiazolopyridin-3-ylenamine compounds of the formula Ia, i.e. compounds I, where, n = 2, W is carbon, which is connected to N² with a double bond and to N¹ with a single bond, N² is carrying only the substituent R², which is hydrogen and not the substituent R³, can be prepared, for example, by reacting a cyanopyridine-sulfonylhalide (II) with ammonia or a primary amine (III) (see Scheme 1), similarly to a process described in EP-A1 33984 (see p.11-12) for corresponding phenyl sulfonylhalides.

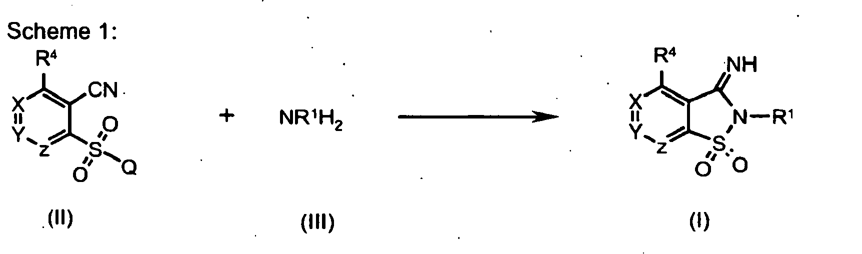

In Scheme 1 the variables X, Y, Z and R¹ to R⁷ are as defined above and Q is halogen, especially chlorine or bromine. The reaction of the sulfonylhalide II, especially a sulfonylchloride, with the amine III can be carried out similarly to standard methods of reacting sulfonylhalides with primary amines as described in J. March, 4^{th} edition 1992, p. 499 and the literature cited therein.

In general, the amine III is employed in an at least equimolar amount, preferably at least 2-fold molar excess, based on the sulfonylhalide II, to bind the hydrogen halide formed. It may be advantageous to employ the primary amine III in an up to 6-fold molar excess, based on the sulfonylhalide II.

It may be advantageous to carry out the reaction in the presence of an auxiliary base. Suitable auxiliary bases include organic bases, for example tertiary amines, such as aliphatic tertiary amines, such as trimethylamine, triethylamine or diisopropylamine, cycloaliphatic tertiary amines such as N-methylpiperidine or aromatic amines such pyridine, substituted pyridines such as 2,3,5-collidine, 2,4,6-collidine, 2,4-lutidine, 3,5-lutidine or 2,6-lutidine and inorganic bases for example alkali metal hydrides such as sodium hydride and potassium hydride, alkali metal carbonates and alkaline earth metal carbonates such as lithium carbonate, potassium carbonate and sodium carbonate, calcium carbonate and alkaline metal hydrogencarbonates such as sodium hydrogen carbonate. The molar ratio of auxiliary base to sulfonylhalide II is preferably in the range of from 1:1 to 4:1, preferably 1:1 to 2:1. If the reaction is carried out in the presence of an auxiliary base, the molar ratio of primary amine III to sulfonylhalide II usually is 1:1 to 1.5:1.

The reaction of II and III is usually carried out in the presence of a solvent. Suitable solvents are polar solvents which are inert under the reaction conditions, for example C₁-C₄-alkanois such as methanol, ethanol, n-propanol or isopropanol, dialkyl ethers such as diethyl ether, diisopropyl ether or methyl tert-butyl ether, cyclic ethers such as dioxane or tetrahydrofuran, acetonitrile, carboxamides such as N,N-dimethyl formamide, N,N-dimethyl acetamide or N-methylpyrrolidinone, water, (provided the sulfonylhalide II is sufficiently resistant to hydrolysis under the reaction conditions used) or a mixture thereof.

The reaction is usually carried out at a reaction temperature ranging from 0°C to the boiling point of the solvent, preferably from 0 to 30°C.

If not commercially available, the amines III can be prepared by standard methods for preparing primary or secondary amines.

Sulfonylhalides II can be prepared by the methods as described in US serial-number 60/703753 filed July 2005 and hereinafter.

Sulfonylchloride compounds II wherein Z is nitrogen can be prepared, for example, according to the reaction sequence shown in Scheme 2 where the variables R⁴, R⁵ and R⁶ are as defined above: 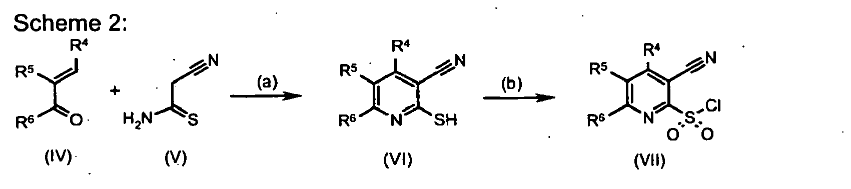
a) condensation of cyanothioacetamide V with a 3,4-unsaturated carbonyl compound IV to the thiopyridine VI in analogy to a process described in Liebigs Annalen der Chemie, No. 1, (1986), pp. 210-19 and the Journal of the Chinese Chemical Society (Taiwan), Vol. 49, No. 4, (2002), pp. 561-569 and subsequent
b) oxidation of the thiol VI to the sulfonylchloride VII, for example, by reacting the thiol VI with chlorine in water or a water-solvent mixture, e. g. a mixture of water and acetic acid or water and dichloromethane, in analogy to a process described in Jerry March, 3rd edition, 1985, reaction 9-27, p. 1087.

Sulfonylchloride compounds II wherein Y is nitrogen can be prepared, for example, according to the reaction sequence shown in Scheme 3 where the variables R⁴, R⁵ and R⁷ are as defined above and R is alkyl, preferably C₁-C₆-alkyl, more preferably C₁-C₄-alkyl, like e.g. n-propyl: 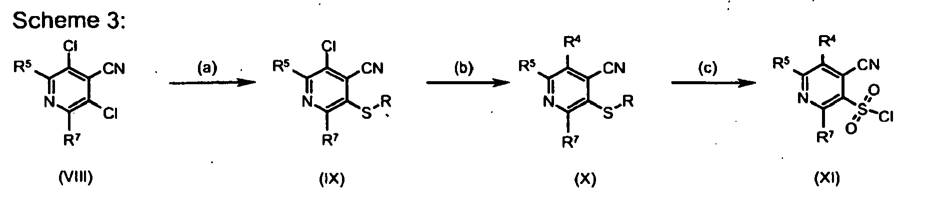 conversion of a dichloroisonicotinonitrile VIII into a thiopropylether IX, for example, in analogy to a process described in Heterocycles Vol. 43, No. 9, (1996), pp. 1893-1899, by reacting VIII with an alkaline thiopropylate reagent formed from propanethiol and a base such as for example alkali metal hydrides such as sodium hydride and potassium hydride, alkali metal carbonates and alkaline earth metal carbonates such as lithium carbonate, potassium carbonate and sodium carbonate, calcium carbonate, alkaline metal hydrogencarbonates such as sodium hydrogen carbonate, or an organo alkyl metal reagent like butyllithium. The reaction can be performed in an inert organic solvent, for example dialkyl ethers such as diethyl ether, diisopropyl ether or methyl tert-butyl ether, cyclic ethers such as dioxane or tetrahydrofuran, acetonitrile, carboxamides such as N,N-dimethyl formamide, N,N-dimethyl acetamide or N-methylpyrrolidinone. The reaction is usually carried out at a reaction temperature ranging from 0 °C to the boiling point of the solvent. The molar ratio of the alkaline thiopropylate to dichloroisonicotinonitrile VIII is preferably in the range from 1:1 to 1.5:1. The dichloroisonicotinonitrile VIII can be prepared in analogy to a process described in Heterocycles Vol. 43, No. 9, (1996), pp. 1893-1899; or a process described in Journal of Medicinal Chemistry, Vol. 44, No.6, (2001), pp. 988-100; and subsequent
b) Introduction of an alkyl or alkoxide substituent, if R⁴ is an alkyl or alkoxide group, for example, in analogy to a process described in Heterocycles Vol. 43, No. 9, (1996), pp. 1893-1899, by reacting IX with an alkali metal alkylate or alkali metal alkoxide like, for example, sodium methylate in an inert organic solvent, for example dialkyl ethers such as diethyl ether, diisopropyl ether or methyl tert-butyl ether, cyclic ethers such as dioxane or tetrahydrofuran, acetonitrile, carboxamides such as N,N-dimethyl formamide, N,N-dimethyl acetamide or N-methylpyrrolidinone. The reaction is usually carried out at a reaction temperature ranging from 0 °C to the boiling point of the solvent. The molar ratio of the alkali metal alkylate or alkali metal alkoxide to chloronicotononitrile IX is preferably in the range from 1:1 to 10:1; and subsequent
c) oxidative cleavage of the thioether X to obtain the sulfonylchloride XI for example, by reacting the thioether X with chlorine in water or a water-solvent mixture, e. g. a mixture of water and acetic acid or water and dichloromethane, in analogy to a process described in Canadian Journal of Chemistry, Vol. 74, No. 9, (1996), pp. 1638-1648.

The preparation of the sulfonylchloride compound II wherein Y is nitrogen and R⁴ is CH₃ can also be carried out, for example, according to the reaction sequence shown in Scheme 4 where the variables R⁵ and R⁷ are as defined above: 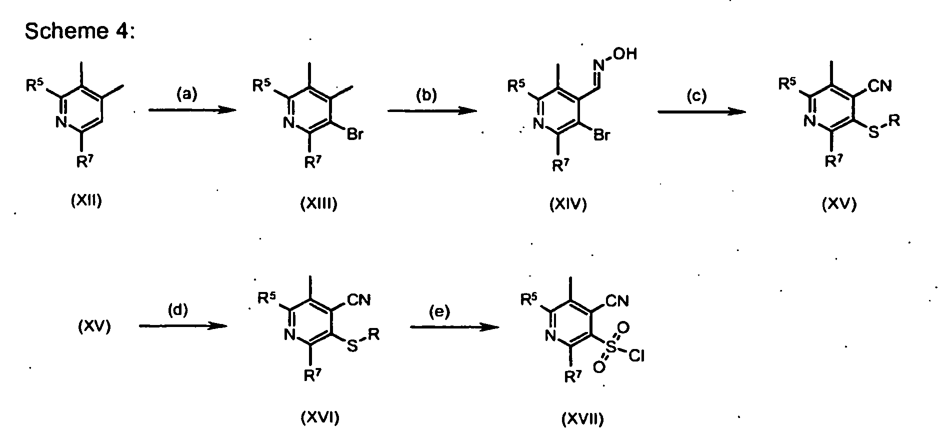
a) conversion of a 3,4-lutidine XII into a bromide XIII by reaction with a bromination agent like bromine in a polar solvent like sulfuric acid in analogy to a process described in Zeitschrift für Chemie, Vol. 28, No. 2, (1988), pp. 59-60 and subsequent
b) conversion of XIII into the oxime XIV by reacting XIII with an alkali amide base like lithiumdiisopropylamide or an alkali alkoxide base like potassium tertbutoxide and an nitrosation reagent like alkyl nitrite, for example n-butyl nitrite, in analogy to a process described in Heterocycles, Vol. 6, No. 9-10, (1977), pp. 1616-21 and subsequent
c) dehydration of oxime XIV to nitrile XV by reaction of XIV with refluxing acetic anhydride according to a process described in Heterocycles, Vol. 5, No. 1, (1987), pp. 343-5 and subsequent
d) conversion of the bromonitrile XV into a thioalkylether XVI, by reacting XV with an alkaline thioalkylate reagent formed from alkylmercaptan and a base in analogy to step a) of scheme 3, and subsequent
e) oxidative cleavage of the thioether XVI to obtain the sulfonylchloride XVII for example, by reacting the thioether XVI with chlorine in water or a water-solvent mixture in analogy to step c) of scheme 3.

Isothiazolopyridin-3-ylenamine compounds of the formula la, i.e. compounds I, where, n = 2, A is carbon, which is connected to N² with a double bond and to N¹ with a single bond, N² is carrying only the substituent R², which is hydrogen, and not the substituent R³, and X is nitrogen can be prepared, for example, according to the reaction sequence shown in Scheme 5 where the variables R¹, R⁴, R⁶ and R⁷ are as defined above: 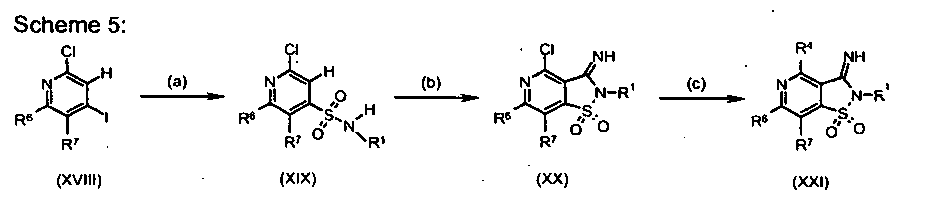
a) conversion of a 4-iodopyridine XVIII into a sulfonamide XIX in analogy to a process described in Journal of Organic Chemistry, Vol. 68, No. 21, (2003), pp. 8274-8276. The iodopyridine XVIII is treated with isopropylmagnesium chloride to generate the corresponding Grignard reagent, followed by treatment with SO₂ and subsequent treatment with sulfuryl chloride. The sulfonylchloride is isolated and subsequentially converted into a sulfonamide XIX by reacting the intermediate sulfonylchloride with a primary or secondary amine similarly to the process described in scheme 1.
b) conversion of XIX into the iminosaccharine XX by reaction with a strong organometallic base like lithiumdiisopropylamide or t-Butyllithium followed by treatment with a cyanide source like p-toluene sulfonyl cyanide in a inert organic solvent like tetrahydrofuran followed by acidic cyclization in analogy to a process described in Journal of Heterocyclic Chemistry 1992, Vol. 29, No. 1, pp. 61-64 and Journal of Organic Chemistry 1987, Vol. 52, No. 6, pp. 1133-6, and subsequent
c) Introduction of an alkyl or alkoxide substituent, if R⁴ is an alkyl or alkoxide group, for example, in analogy to the process described in step b) of scheme 3.

Isothiazolopyridin-3-ylenamine compounds of the formula Ia, i.e. compounds I, where, n = 2, A is carbon, which is connected to N² with a double bond and to N¹ with a single bond, N² is carrying only the substituent R², which is hydrogen, and not the substituent R³, and X is nitrogen can also be prepared, for example, according to the reaction sequence shown in Scheme 6 where the variables R¹, R⁴, R⁶ and R⁷ are as defined above: 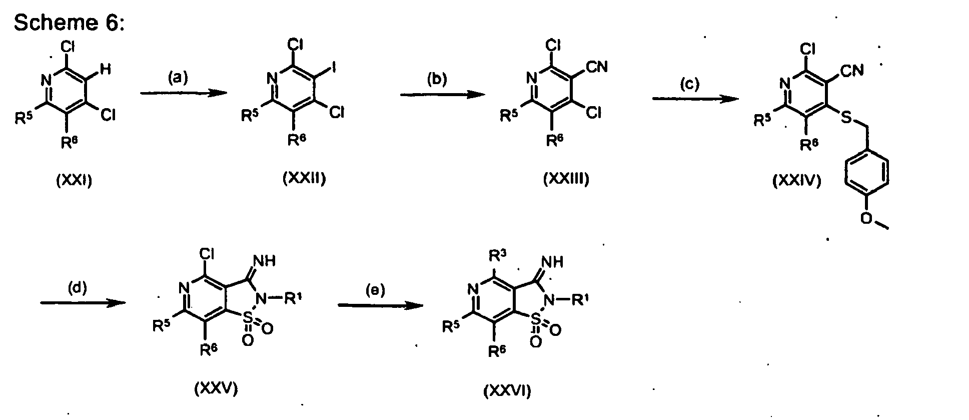
a) conversion of XXI into a iodide XXII by reaction with a strong organometallic base like lithiumdiisopropylamide or t-butyllithium followed by treatment with iodide in an inert organic solvent like tetrahydrofuran in analogy to a process described in European Journal of Organic Chemistry, Vol. 7, (2001), pp. 1371-1376. The starting compound XXI can be prepared according to a procedure described in Synthetic Communications, Vol. 22, No.19, (1992), pp. 2829-2837.
b) conversion of iodide XXII into a nitrile XXIII by reaction with Copper(I)cyanide in refluxing N,N-Dimethylformamide under an inter gas atmosphere in analogy to a process described in Journal of Medicinal Chemistry Vol. 47, No. 14, (2004), pp. 3658-3664; and subsequent
c) conversion of Chloride XXIII into a p-methoxybenzylthioether XXIV by reaction with, for example, in analogy to a process described in Bioorganic & Medicinal Chemistry Letters, Vo. 11, No. 14, (2001), pp. 1951-1954 by reacting VIII with an alkaline 4-methoxybenzylthiolate reagent formed from 4-methoxybenzylmercaptane and a base such as for example alkali metal hydrides, alkali metal carbonates and alkaline earth metal carbonates, or an organo alkyl metal reagent like butyllithium. The reaction can be performed in an inert organic solvent, for example dialkyl ethers cyclic ethers such as tetrahydrofuran, carboxamides such as N,N-dimethyl formamide, N,N-dimethyl acetamide or N-methylpyrrolidinone. The reaction is usually carried out at a reaction temperature ranging from 0 °C to the boiling point of the solvent. The molar ratio of the alkaline thiopropylate to dichloronicotononitrile VIII is preferably in the range from 1:1 to 1.5:1; and subsequent
   oxidative cleavage of the thioether XXIV to obtain the isothiazolopyridine-3-ylidenamine XXV for example, by reacting XXIV with chlorine in water or a water-solvent mixture in analogy to step c) of scheme 3, directly followed by reaction of the intermediate sulfonyl chloride with amines in analogy to scheme 1; and subsequent
e) Introduction of an alkyl or alkoxide substituent, if R³ is an alkyl or alkoxide group, for example, in analogy to the process described in step b) of scheme 3.

Isothiazolopyridin-3-ylenamine compounds of the formula Ia, i.e. compounds I, where, n = 2, A is carbon, which is connected to N² with a single bond and to N¹ with a double bond, N¹ is not carrying the substituent R¹ and N² is carrying the substituents R² and R³, can be prepared, for example, by reacting a 3-Halo-isothiazolopyridine (XXVII) with a primary or secondary amine (XXVIII) (see Scheme 7), similarly to a process described in Indian Journal of Chemistry, Section B 1977, Vol. 15, No.8, pp. 720-726 and Journal of Chemical and Engineering Data 1987, Vol 32, No. 3, pp. 391-393 for corresponding pseudosaccharin chlorides.

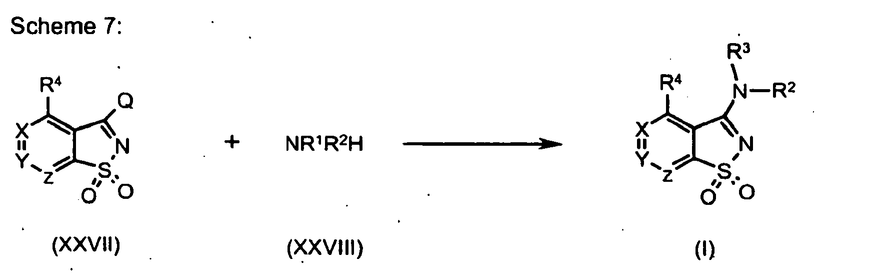

In Scheme 7 the variables X, Y, Z and R² to R⁷ are as defined above and Q is halogen, especially chlorine or bromine. The reaction of the 3-Halo-isothiazolopyridine XXVII, especially a sulfonylchloride, with the amine XXVIII can be carried out similarly to standart methods of reacting 3-halo-benzisothiazoles with primary or secondary amines as described in Journal of the Chemical Society, Perkin Transactions 2, (8), 1315-1324; 2001.

In general, the amine XXVIII is employed in an at least equimolar amount, preferably at least 2-fold molar excess, based on the 3-Halo-isothiazolopyridine XXVII, to bind the hydrogen halide formed. It may be advantageous to employ the primary amine XXVIII in an up to 6-fold molar excess, based on the 3-Halo-isothiazolopyridine XXVII.

It may be advantageous to carry out the reaction in the presence of an auxiliary base. Suitable auxiliary bases include organic bases, for example tertiary amines, such as aliphatic tertiary amines, such as trimethylamine, triethylamine or diisopropylamine, cycloaliphatic tertiary amines such as N-methylpiperidine or aromatic amines such pyridine, substituted pyridines such as 2,3,5-collidine, 2,4,6-collidine, 2,4-lutidine, 3,5-lutidine or 2,6-lutidine and inorganic bases for example alkali metal hydrides such as sodium hydride and potassium hydride, alkali metal carbonates and alkaline earth metal carbonates such as lithium carbonate, potassium carbonate and sodium carbonate, calcium carbonate and alkaline metal hydrogencarbonates such as sodium hydrogen carbonate. The molar ratio of auxiliary base to 3-Halo-isothiazolopyridine XXVII is preferably in the range of from 1:1 to 4:1, preferably 1:1 to 2:1. If the reaction is carried out in the presence of an auxiliary base, the molar ratio of primary amine XXVIII to 3-Halo-isothiazolopyridine XXVII usually is 1:1 to 1.5:1.

The reaction of XXVII and XXVIII is usually carried out in the presence of a solvent. Suitable solvents are polar solvents which are inert under the reaction conditions, for example C₁-C₄-alkanois such as methanol, ethanol, n-propanol or isopropanol, dialkyl ethers such as diethyl ether, diisopropyl ether or methyl tert-butyl ether, cyclic ethers such as dioxane or tetrahydrofuran, acetonitrile, carboxamides such as N,N-dimethyl formamide, N,N-dimethyl acetamide or N-methylpyrrolidinone, water, (provided the 3-Halo-isothiazolopyridine XXVII is sufficiently resistant to hydrolysis under the reaction conditions used) or a mixture thereof.

The reaction is usually carried out at a reaction temperature ranging from 0°C to the boiling point of the solvent, preferably from 0 to 30°C.

If not commercially available, the amines XXVIII can be prepared by standard methods for preparing primary or secondary amines.

3-Halo-isothiazolopyridines XXVII are new and can be prepared by the method described hereinafter. 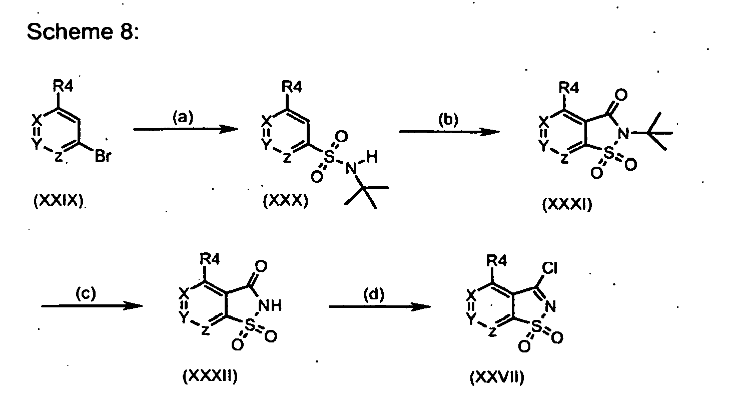 conversion of a bromo pyridine XXIX into a tertbutyl sulphonamide XXX bromine lithium exchange via treatment of XXIX with a metallation reagent like n-butyllithium, sequential reaction of the metallated pyridine with SO₂ and sulfuryl chloride and reaction of the derived sulfonyl chloride with tertbutyl amine; directed ortho metallation of the pyridine sulphonamide XXX using an organometallic base like lithium diisopropyl amide and reaction of the metallated intermediate with CO₂ followed by acid catalyzed cyclization of the intermediate to pyridine saccharine XXXI; acid induced cleavage of the tertbutyl group towards pyridine saccharine XXXII using an acidic reagent like trifluoro acetic acid; conversion of XXXII into the pyridine pseudosaccharine halide XXVII using a halogenation reagent like thionyl chloride.

Isothiazolopyridin-N-oxide-3-ylenamine compounds of the formula (XXXV), i.e. compounds I, where, n = 2, X is an NO, Y and Z are carbon, and the variables A, R¹, R², R³, R⁴, R⁶ and R⁷ are as defined above, can be prepared, for example, by reacting the corresponding Isothiazolopyridin-3-ylenamine compound (XXXIII) and an oxidation reagent, for example a peroxide reagent (XXXIV) like 3-chloroperbenzoic acid (see Scheme 9), similarly to a process described in Synthetic Communications 1977, Vol. 7, No. 8, pp. 509-14.

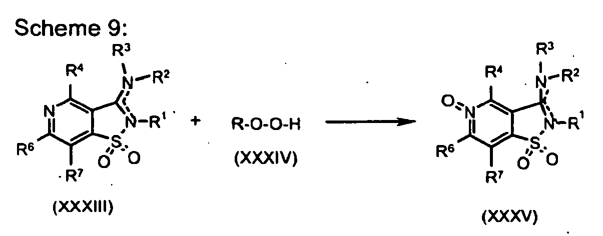

Isothiazolopyridin-N-oxide-3-ylenamine compounds of the formula (XXXVII), i.e. compounds I, where, n = 2, Y is an NO, X and Z are carbon, and the variables A, R¹, R², R³, R⁴, R⁵ and R⁷ are as defined above, can be prepared, for example, by reacting the corresponding Isothiazolopyridin-3-ylenamine compound (XXXVI) and an oxidation reagent, for example a peroxide reagent (XXXIV) like 3-chloroperbenzoic acid (see Scheme 10), similarly to a process described in Synthetic Communications 1977, Vol. 7, No. 8, pp. 509-14.

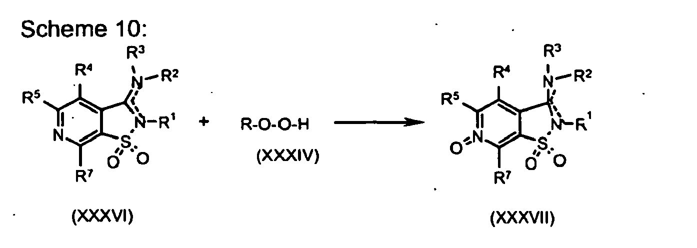

Isothiazolopyridin-N-oxide-3-ylenamine compounds of the formula (XXXIX), i.e. compounds I, where, n = 2, Z is an NO, X and Z are carbon, and the variables A, R¹, R², R³, R⁴, R⁵ and R⁷ are as defined above, can be prepared, for example, by reacting the corresponding Isothiazolopyridin-3-ylenamine compound (XXXVIII) and an oxidation reagent, for example a peroxide reagent (XXXIV), preferably halogenated perbenzoic acids, e.g. like 3-chloroperbenzoic acid (see Scheme 11) or hydrogen peroxide, similarly to a process described in Synthetic Communications 1977, Vol. 7, No. 8, pp. 509-14.

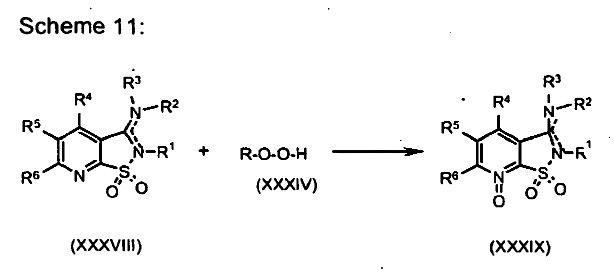

Isothiazolopyridin-N-oxide-3-ylenamine compounds of the formula (Ib), i.e. compounds I, where, n = 0 and the variables A, R¹, R², R³, R⁴, X, Y and Z are as defined above, can be prepared, for example, by
a) Reduction of a Isothiazolopyridin-N-oxide-3-ylenamine (Ia) to the corresponding compound (Ib) using a suitable reducing agent. Suitable reducing agent include (1) tris(2-carboxyethyl)phosphine in a mixture of dioxane and water as described in Synthetic Communications 2003, Vol. 33, No. 20, pp. 3503-3511; or (2) triphenylphosphine with or without the addition of iodine in an aromatic solvent like benzine or toluene as described in Bulletin of the Chemical Society of Japan 1983, Vol. 56, No. 12, pp. 3802-3812; or (3) zinc in combination with dichlorodimethylsilane, dimethylacetamide in a chlorinated hydrocarbon like dichloroethane as described in Tetrahedron Letters 1999, Vol. 40, pp. 3179-3182. (see scheme 12)

Isothiazolopyridin-N-oxide-3-ylenamine compounds of the formula (Ic), i.e. compounds I, where n = 1 and the variables A, R¹, R², R³, R⁴, X, Y and Z are as defined above, can be prepared, for example, by
b) Oxidation of a Isothiazolopyridin-N-oxide-3-ylenamine (Ib) to the corresponding compound (Ic) using a suitable oxidating agent. Suitable oxidating agents include (1) H2O2 in acetic acid as described in Journal of Organic Chemistry (2000), vol. 65, pp. 8439-8443 for corresponding phenyl derivatives; (2) N-bromsuccinimid or (3) gaseous Cl₂; both as described in Synthesis (2001), vol.11, pp. 1659-1664 for corresponding phenyl derivatives.

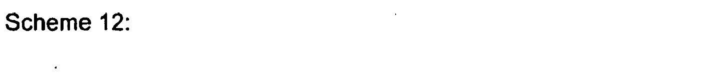

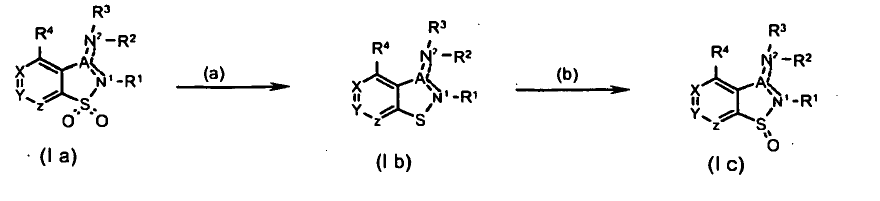

If individual compounds cannot be prepared via the above-described routes, they can be prepared by derivatization of other compounds I or by customary modifications of the synthesis routes described.

The reaction mixtures are worked up in the customary manner, for example by mixing with water, separating the phases, and, if appropriate, purifying the crude products by chromatography, for example on alumina or silica gel may be employed. Some of the intermediates and end products may be obtained in the form of colorless or pale brown viscous oils, which are freed or purified from volatile components under reduced pressure and at moderately elevated temperature. If the intermediates and end products are obtained as solids, they may be purified by recrystallization or digestion.

The compounds of the formula I and their salts are in particular suitable for efficiently controlling arthropodal pests such as arachnids, myriapedes and insects as well as nematodes.

In particular, they are suitable for controlling insect pests, such as insects from the order of

Lepidoptera: for example Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera eridania, Spodoptera frugiperda, Spodoptera littoralis, Spodoptera litura, Thaumatopoea pityocampa, Tortrix viridana, Trichoplusia ni and Zeiraphera canadensis,

Coleoptera (beetles), for example Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus and Sitophilus granaria,

Diptera, for example Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea and Tipula paludosa,

Thysanoptera (thrips), e.g. Dichromothrips spp., Frankliniella fusca, Frankliniella occidentalis, Frankliniella tritici, Scirtothrips citri, Thrips oryzae, Thrips palmi and Thrips tabaci,

Hymenoptera e.g. Athalia rosae, Atta cephalotes, Atta sexdens, Atta texana, Hoplocampa minuta, Hoplocampa testudinea, Monomorium pharaonis, Solenopsis geminata and Solenopsis invicta,

Heteroptera, e.g. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis and Thyanta perditor,

Homoptera (in particular aphids), e.g. Acyrthosiphon onobrychis, Adelges laricis, Aphidula nasturtii, Aphis craccivora, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Bemisa tabaci, Bemisa argentifolii, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzodes persicae, Myzus ascalonicus, Myzus cerasi, Myzus varians, Nasonovia ribis-nigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiand, and Viteus vitifolii,

Isoptera (termites), e.g. Calotermes flavicollis, Leucotermes flavipes, Reticulitermes lucifugus und Termes natalensis,

Orthoptera, e.g. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus and Tachycines asynamorus, and

Collembola (springtails), e.g. Onychiurus ssp..

They are also suitable for controlling Nematodes : plant parasitic nematodes such as root knot nematodes, Meloidogyne hapla, Meloidogyne incognita, Meloidogyne javanica, and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species.

The compounds of the formula I and their salts are also useful for controlling arachnids (Arachnoidea), such as acarians (Acarina), e.g. of the families Argasidae, Ixodidae and Sarcoptidae, such as Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Dermanyssus gallinae, Psoroptes ovis, Rhipicephalus appendiculatus, Rhipicephalus evertsi, Sarcoptes scabiei, and Eriophyidae spp. such as Aculus schlechtendali, Phyllocoptrata oleivora and Eriophyes sheldoni; Tarsonemidae spp. such as Phytonemus pallidus and Polyphagotarsonemus latus; Tenuipalpidae spp. such as Brevipalpus phoenicis; Tetranychidae spp. such as Tetranychus cinnabarinus, Tetranychus kanzawai, Tetranychus pacificus, Tetranychus telarius and Tetranychus urticae, Panonychus ulmi, Panonychus citri, and oligonychus pratensis.

Compounds of the formula I are particularly useful for controlling insects, preferably sucking or piercing insects such as insects from the genera Thysanoptera, Hymenoptera, Orthoptera and Homptera, in particular the following species:

Thysanoptera (thrips): Frankliniella fusca, Frankliniella occidentalis, Frankliniella tritici, Scirtothrips citri, Thrips oryzae, Thrips palmi and Thrips tabaci,

Hymenoptera: Athalia rosae, Atta cephalotes, Atta sexdens, Atta texana, Hoplocampa minuta, Hoplocampa testudinea, Monomorium pharaonis, Solenopsis geminata and Solenopsis invicta,

Orthoptera: Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus and Tachycines asynamorus ;

Homoptera, in particular aphids: Acyrthosiphon onobrychis, Adelges laricis, Aphidula nasturtii, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzodes persicae, Myzus ascalonicus, Myzus cerasi, Myzus varians, Nasonovia ribis-nigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiand, and Viteus vitifolii;

Compounds of the formula I are particularly useful for controlling insects of the orders Homoptera and Thysanoptera and more preferably for controlling aphids.

### Formulations

For use in a method according to the present invention, the compounds I can be converted into the customary formulations, e.g. solutions, emulsions, suspensions, dusts, powders, pastes, granules and directly sprayable solutions. The use form depends on the particular purpose and application method. Formulations and application methods are chosen to ensure in each case a fine and uniform distribution of the compound of the formula I according to the present invention.

The formulations are prepared in a known manner (see e.g. for review US 3,060,084, EP-A 707 445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 and Mollet, H., Grubemann, A., Formulation technology, Wiley VCH Verlag GmbH, Weinheim (Germany), 2001, 2. D. A. Knowles, Chemistry and Technology of Agrochemical Formulations, Kluwer Academic Publishers, Dordrecht, 1998 (ISBN 0-7514-0443-8), for example by extending the active compound with auxiliaries suitable for the formulation of agrochemicals, such as solvents and/or carriers, if desired emulsifiers, surfactants and dispersants, preservatives, antifoaming agents, anti-freezing agents, for seed treatment formulation also optionally colorants and/or binders and/or gelling agents.

Solvents/carriers, which are suitable, are e.g.:
- solvents such as water, aromatic solvents (for example Solvesso products, xylene and the like), paraffins (for example mineral fractions), alcohols (for example methanol, butanol, pentanol, benzyl alcohol), ketones (for example cyclohexanone, gamma-butyrolactone), pyrrolidones (N-metyhl-pyrrolidone (NMP),N-octylpyrrolidone NOP), acetates (glycol diacetate), alkyl lactates, lactones such as g-butyrolactone, glycols, fatty acid dimethylamides, fatty acids and fatty acid esters, triglycerides, oils of vegetable or animal origin and modified oils such as alkylated plant oils. In principle, solvent mixtures may also be used.
- carriers such as ground natural minerals and ground synthetic minerals, such as silica gels, finely divided silicic acid, silicates, talc, kaolin, attaclay, limestone, lime, chalk, bole, loess, clay, dolomite, diatomaceous earth, calcium sulfate and magnesium sulfate, magnesium oxide, ground synthetic materials, fertilizers, such as, for example, ammonium sulfate, ammonium phosphate, ammonium nitrate, ureas and products of vegetable origin, such as cereal meal, tree bark meal, wood meal and nutshell meal, cellulose powders and other solid carriers.

Suitable emulsifiers are nonionic and anionic emulsifiers (for example polyoxyethylene fatty alcohol ethers, alkylsulfonates and arylsulfonates).

Examples of dispersants are lignin-sulfite waste liquors and methylcellulose.

Suitable surfactants are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenyl ether, ethoxylated isooctylphenol, octylphenol, nonylphenol, alkylphenyl polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters,

Also anti-freezing agents such as glycerin, ethylene glycol, propylene glycol and bactericides such as can be added to the formulation.

Suitable antifoaming agents are for example antifoaming agents based on silicon or magnesium stearate.

Suitable preservatives are for example dichlorophen und benzyl alcohol hemiformal

Suitable thickeners are compounds, which confer a pseudoplastic flow behavior to the formulation, i.e. high viscosity at rest and low viscosity in the agitated stage. Mention may be made, in this context, for example, of commercial thickeners based on polysaccharides, such as Xanthan Gum® (Kelzan® from Kelco), Rhodopol® 23 (Rhone Poulenc) or Veegum® (from R.T. Vanderbilt), or organic phyllosilicates, such as Attaclay® (from Engelhardt). Antifoam agents suitable for the dispersions according to the invention are, for example, silicone emulsions (such as, for example, Silikon® SRE, Wacker or Rhodorsil® from Rhodia), long-chain alcohols, fatty acids, organofluorine compounds and mixtures thereof. Biocides can be added to stabilize the compositions according to the invention against attack by microorganisms. Suitable biocides are, for example, based on isothiazolones such as the compounds marketed under the trademarks Proxel® from Avecia (or Arch) or Acticide® RS from Thor Chemie and Kathon® MK from Rohm & Haas. Suitable antifreeze agents are organic polyols, for example ethylene glycol, propylene glycol or glycerol. These are usually employed in amounts of not more than 10% by weight, based on the total weight of the active compound composition. If appropriate, the active compound compositions according to the invention may comprise 1 to 5% by weight of buffer, based on the total amount of the formulation prepared, to regulate the pH, the amount and type of the buffer used depending on the chemical properties of the active compound or the active compounds. Examples of buffers are alkali metal salts of weak inorganic or organic acids, such as, for example, phosphoric acid, boronic acid, acetic acid, propionic acid, citric acid, fumaric acid, tartaric acid, oxalic acid and succinic acid.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, strongly polar solvents, for example dimethyl sulfoxide, N-methylpyrrolidone and water.

Powders, materials for spreading and dusts can be prepared by mixing or concomitantly grinding the active substances with a solid carrier.

Granules, for example coated granules, impregnated granules and homogeneous granules, can be prepared by binding the active ingredients to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the active ingredient. The active ingredients are employed in a purity of from 90% to 100%, preferably 95% to 100% (according to NMR spectrum).

For seed treatment purposes, respective formulations can be diluted 2-10 fold leading to concentrations in the ready to use preparations of 0,01 to 60% by weight active compound by weight, preferably 0,1 to 40% by weight.

The compound of formula I can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the active compounds according to the invention.

The following are examples of formulations:

### 1. Products for dilution with water. For seed treatment purposes, such products may be applied to the seed diluted or undiluted.

### A) Water-soluble concentrates (SL, LS)

10 parts by weight of the active compound is dissolved in 90 parts by weight of water or a water-soluble solvent. As an alternative, wetters or other auxiliaries are added. The active compound dissolves upon dilution with water, whereby a formulation with 10 % (w/w) of active compound is obtained.

### B) Dispersible concentrates (DC)

20 parts by weight of the active compound is dissolved in 70 parts by weight of cyclohexanone with addition of 10 parts by weight of a dispersant, for example polyvinylpyrrolidone. Dilution with water gives a dispersion, whereby a formulation with 20% (w/w) of active compounds is obtained.

### C) Emulsifiable concentrates (EC)

15 parts by weight of the active compounds is dissolved in 7 parts by weight of xylene with addition of calcium dodecylbenzenesulfonate and castor oil ethoxylate (in each case 5 parts by weight). Dilution with water gives an emulsion, whereby a formulation with 15% (w/w) of active compounds is obtained.

### D) Emulsions (EW, EO, ES)

25 parts by weight of the active compound is dissolved in 35 parts by weight of xylene with addition of calcium dodecylbenzenesulfonate and castor oil ethoxylate (in each case 5 parts by weight). This mixture is introduced into 30 parts by weight of water by means of an emulsifier machine (e.g. Ultraturrax) and made into a homogeneous emulsion. Dilution with water gives an emulsion, whereby a formulation with 25% (w/w) of active compound is obtained.

### E) Suspensions (SC, OD, FS)

In an agitated ball mill, 20 parts by weight of the active compound is comminuted with addition of 10 parts by weight of dispersants, wetters and 70 parts by weight of water or of an organic solvent to give a fine active compound suspension. Dilution with water gives a stable suspension of the active compound, whereby a formulation with 20% (w/w) of active compound is obtained.

### F) Water-dispersible granules and water-soluble granules (WG, SG)

50 parts by weight of the active compound is ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the active compound, whereby a formulation with 50% (w/w) of active compound is obtained.

### G) Water-dispersible powders and water-soluble powders (WP, SP, SS, WS)

75 parts by weight of the active compound are ground in a rotor-stator mill with addition of 25 parts by weight of dispersants, wetters and silica gel. Dilution with water gives a stable dispersion or solution of the active compound, whereby a formulation with 75% (w/w) of active compound is obtained.

### H) Gel-Formulation (GF)

In an agitated ball mill, 20 parts by weight of the active compound is comminuted with addition of 10 parts by weight of dispersants, 1 part by weight of a gelling agent wetters and 70 parts by weight of water or of an organic solvent to give a fine active compound suspension. Dilution with water gives a stable suspension of the active compound, whereby a formulation with 20% (w/w) of active compound is obtained.

### 2. Products to be applied undiluted for foliar applications. For seed treatment purposes, such products may be applied to the seed diluted or undiluted.

### I) Dustable powders (DP, DS)

5 parts by weight of the active compound are ground finely and mixed intimately with 95 parts by weight of finely divided kaolin. This gives a dustable product having 5% (w/w) of active compound.

### J) Granules (GR, FG, GG, MG)

0.5 part by weight of the active compound is ground finely and associated with 95.5 parts by weight of carriers, whereby a formulation with 0.5% (w/w) of active compound is obtained. Current methods are extrusion, spray-drying or the fluidized bed. This gives granules to be applied undiluted for foliar use.

### K) ULV solutions (UL)

10 parts by weight of the active compound is dissolved in 90 parts by weight of an organic solvent, for example xylene. This gives a product having 10% (w/w) of active compound, which is applied undiluted for foliar use.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active ingredient concentrations in the ready-to-use products can be varied within relatively wide ranges. In general, they are from 0.0001 to 10%, preferably from 0.01 to 1%.

The active ingredients may also be used successfully in the ultra-low-volume process (ULV), it being possible to apply formulations comprising over 95% by weight of active ingredient, or even to apply the active ingredient without additives.

In the method of this invention compounds I may be applied with other active ingredients, for example with other pesticides, insecticides, herbicides, fertilizers such as ammonium nitrate, urea, potash, and superphosphate, phytotoxicants and plant growth regulators, safeners and nematicides. These additional ingredients may be used sequentially or in combination with the above-described compositions, if appropriate also added only immediately prior to use (tank mix). For example, the plant(s) may be sprayed with a composition of this invention either before or after being treated with other active ingredient.

The following list M of pesticides together with which the compounds according to the invention can be used and with which potential synergistic effects might be produced, is intended to illustrate the possible combination, but not to impose any limitation:

M.1. Organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;

M.2. Carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;

M.3. Pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;

M.4. Growth regulators: a) chitin synthesis inhibitors: benzoylureas: chlorfluazuron, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramat;

M.5. Nicotinic receptor agonists/antagonists compounds: clothianidin, dinotefuran, imidacloprid, thiamethoxam, nitenpyram, acetamiprid, thiacloprid;
the thiazol compound of formula (Γ¹)

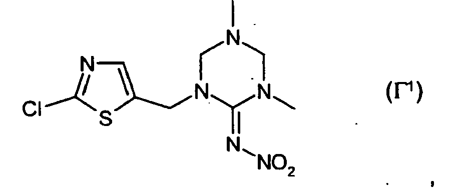

M.6. GABA antagonist compounds: acetoprole, endosulfan, ethiprole, fipronil, vaniliprole, pyrafluprole, pyriprole, the phenylpyrazole compound of formula Γ²

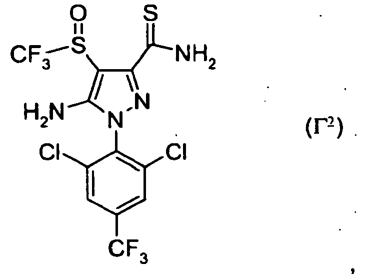

M.7. Macrocyclic lactone insecticides: abamectin, emamectin, milbemectin, lepimectin, spinosad

M.8. METI I compounds: fenazaquin, pyridaben, tebufenpyrad, tolfenpyrad, flufenerim;

M.9. METI II and III compounds: acequinocyl, fluacyprim, hydramethylnon;

M.10. Uncoupler compounds: chlorfenapyr;

M.11. Oxidative phosphorylation inhibitor compounds: cyhexatin, diafenthiuron, fenbutatin oxide, propargite;

M.12. Moulting disruptor compounds: cyromazine;

M.13. Mixed Function Oxidase inhibitor compounds: piperonyl butoxide;

M.14. Sodium channel blocker compounds: indoxacarb, metaflumizone,

M.15. Various: benclothiaz, bifenazate, cartap, flonicamid, pyridalyl, pymetrozine, sulfur, thiocyclam, flubendiamide, cyenopyrafen, flupyrazofos, cyflumetofen, amidoflumet, N-R'-2,2-dihalo-1-R''cyclo-propanecarboxamide-2-(2,6-dichloro- α ,α ,α , -tri-fluoro-p-tolyl)hydrazone or N-R'-2,2-di(R'")propionamide-2-(2,6-dichloro- α ,α ,α - trifluoro-p-tolyl)-hydrazone, wherein R' is methyl or ethyl, halo is chloro or bromo, R" is hydrogen or methyl and R''' is methyl or ethyl, anthranilamide compounds of formula Γ⁵ 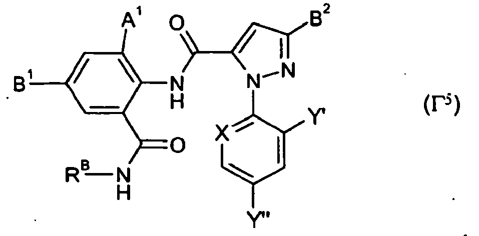 wherein A¹ is CH₃, Cl, Br, I, X is C-H, C-Cl, C-F or N, Y' is F, Cl, or Br, Y" is F, Cl, CF₃, B¹ is hydrogen, Cl, Br, I, CN, B² is Cl, Br, CF₃, OCH₂CF₃, OCF₂H, and R^{B} is hydrogen, CH₃ or CH(CH₃)₂, and malononitrile compounds as described in JP2002-284608, WO02/89579, WO02/90320, WO02/90321, WO04/06677, WO04/20399, JP2004-99597, WO2005068423, WO2005068432 and W02005063694.

The commercially available compounds of the group M may be found in The Pesticide Manual, 13th Edition, British Crop Protection Council (2003) among other publications. Thiamides of formula Γ² and their preparation have been described in WO 98/28279. Lepimection is known from Agro Project, PJB Publications Ltd, November 2004. Benclothiaz and its preparation have been described in EP-A1 454621. Methidathion and Paraoxon and their preparation have been described in Farm Chemicals Handbook, Volume 88, Meister Publishing Company, 2001. Acetoprole and its preparation have been described in WO 98/28277. Metaflumizone and its preparation have been described in EP-A1 462 456. Flupyrazofos has been described in Pesticide Science 54, 1988, p.237-243 and in US 4822779. Pyrafluprole and its preparation have been described in JP-A 2002-193709 and in WO 01/00614. Pyriprole and its preparation have been described in WO 98/45274 and in US 6335357. Amidoflumet and its preparation have been described in US 6221890 and in JP-A 21010907. Flufenerim and its preparation have been described in WO 03/007717 and in WO 03/007718. Cyflumetofen and its preparation have been described in WO 04/080180. Anthranilamides of formula Γ⁵ and their preparation have been described in WO 01/70671; WO 02/48137; WO 03/24222, WO 03/15518, WO 04/67528; WO 04/33468; and WO 05/118552.

In the methods according to the invention the pests are controlled by contacting the target parasite/pest, its food supply, habitat, breeding ground or its locus with a pesticidally effective amount of compounds of formula I or with a salt thereof or with a composition, containing a pesticidally effective amount of a compound of formula I or a salt thereof.

"Locus" means a habitat, breeding ground, plant, seed, soil, area, material or environment in which a pest or parasite is growing or may grow.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

The compounds of the invention can also be applied preventively to places at which occurrence of the pests is expected.

The compounds of formula I may be also used to protect growing plants from attack or infestation by pests by contacting the plant with a pesticidally effective amount of compounds of formula I. As such, "contacting" includes both direct contact (applying the compounds/compositions directly on the pest and/or plant - typically to the foliage, stem or roots of the plant) and indirect contact (applying the compounds/compositions to the locus of the pest and/or plant).

The aforementioned compositions are particularly useful for protecting crop plants against infestation of said pests or for combating these pests in infested plants.

For use in treating crop plants, the rate of application of the active ingredients of this invention may be in the range of 0.1 g to 4000 g per hectare, desirably from 25 g to 600 g per hectare, more desirably from 50 g to 500 g per hectare.

In the case of soil treatment or of application to the pests dwelling place or nest, the quantity of active ingredient ranges from 0.0001 to 500 g per 100 m², preferably from 0.001 to 20 g per 100 m².

The compounds of formula I are also suitable for the treatment of seeds in order to protect the seed from insect pest, in particular from soil-living insect pests and the resulting plant's roots and shoots against soil pests and foliar insects.

The compounds of formula I are particularly useful for the protection of the seed from soil pests and the resulting plant's roots and shoots against soil pests and foliar insects. The protection of the resulting plant's roots and shoots is preferred. More preferred is the protection of resulting plant's shoots from piercing and sucking insects, wherein the protection from aphids is most preferred.

The present invention therefore comprises a method for the protection of seeds from insects, in particular from soil insects and of the seedlings' roots and shoots from insects, in particular from soil and foliar insects, said method comprising contacting the seeds before sowing and/or after pregermination with a compound of the general formula I or a salt thereof. Particularly preferred is a method, wherein the plant's roots and shoots are protected, more preferably a method, wherein the plants shoots are protected form piercing and sucking insects, most preferably aa method, wherein the plants shoots are protected from aphids.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

The term seed treatment comprises all suitable seed treatment techniques known in the art, such as seed dressing, seed coating, seed dusting, seed soaking and seed pelleting.
The present invention also comprises seeds coated with or containing the active compound.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is seed of cereals, root crops, oil crops, vegetables, spices, ornamentals, for example seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

In addition, the active compound may also be used for the treatment seeds from plants, which tolerate the action of herbicides or fungicides or insecticides owing to breeding, including genetic engineering methods.

For example, the active compound can be employed in treatment of seeds from plants, which are resistant to herbicides from the group consisting of the sulfonylureas, imidazolinones, glufosinate-ammonium or glyphosate-isopropylammonium and analogous active substances (see for example, EP-A-0242236, EP-A-242246) (WO 92/00377) (EP-A-0257993, U.S. Pat. No. 5,013,659) or in transgenic crop plants, for example cotton, with the capability of producing Bacillus thuringiensis toxins (Bt toxins) which make the plants resistant to certain pests (EP-A-0142924, EP-A-0193259),

Furthermore, the active compound can be used also for the treatment of seeds from plants, which have modified characteristics in comparison with existing plants consist, which can be generated for example by traditional breeding methods and/or the generation of mutants, or by recombinant procedures). For example, a number of cases have been described of recombinant modifications of crop plants for the purpose of modifying the starch synthesized in the plants (e.g. WO 92/11376, WO 92/14827, WO 91/19806) or of transgenic crop plants having a modified fatty acid composition (WO 91/13972).

The seed treatment application of the active compound is carried but by spraying or by dusting the seeds before sowing of the plants and before emergence of the plants.

In the treatment of seeds the corresponding formulations are applied by treating the seeds with an effective amount of the active compound. Herein, the application rates of the active compound are generally from 0,1 g to 10 kg per 100 kg of seed, preferably from 1 g to 5 kg per 100 kg of seed, in particular from 1 g to 2,5 kg per 100 kg of seed. For specific crops such as lettuce the rate can be higher.

Compositions which are especially useful for seed treatment are e.g.:
- A: Soluble concentrates (SL, LS)
- D: Emulsions (EW, EO, ES)
- E: Suspensions (SC, OD, FS)
- F: Water-dispersible granules and water-soluble granules (WG, SG)
- G: Water-dispersible powders and water-soluble powders (WP, SP, WS)
- H: Gel-Formulations (GF)
- I: Dustable powders (DP, DS)

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter

In a preferred embodiment a FS formulation is used for seed treatment. Typcially, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

Preferred FS formulations of compounds of formula I for seed treatment usually comprise from 0.1 to 80% by weight (1 to 800 g/L) of the active ingredient, from 0.1 to 20 % by weight (1 to 200 g/L) of at least one surfactant, e.g. 0.05 to 5 % by weight of a wetter and from 0.5 to 15 % by weight of a dispersing agent, up to 20 % by weight, e.g. from 5 to 20 % of an anti-freeze agent, from 0 to 15 % by weight, e.g. 1 to 15 % by weight of a pigment and/or a dye, from 0 to 40 % by weight, e.g. 1 to 40 % by weight of a binder (sticker /adhesion agent), optionally up to 5 % by weight, e.g. from 0.1 to 5 % by weight of a thickener, optionally from 0.1 to 2 % of an anti-foam agent, and optionally a preservative such as a biocide, antioxidant or the like, e.g. in an amount from 0.01 to 1 % by weight and a filler/vehicle up to 100 % by weight.

Seed Treatment formulations may additionally also comprise binders and optionally colorants.

Binders can be added to improve the adhesion of the active materials on the seeds after treatment Suitable binders are block copolymers EO/PO surfactants but also polyvinylalcoholsl, polyvinylpyrrolidones, polyacrylates, polymethacrylates, polybutenes, polyisobutylenes, polystyrene, polyethyleneamines, polyethyleneamides, polyethyleneimines (Lupasol®, Polymin®), polyethers, polyurethans, polyvinylacetate, tylose and copolymers derived from these polymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

### Examples of a gelling agent is carrageen (Satiagel®)

In the treatment of seed, the application rates of the compounds I are generally from 0.1 g to 10 kg per 100 kg of seed, preferably from 1 g to 5 kg per 100 kg of seed, in particular from 1 g to 1000 g per 100 kg of seed.

The invention therefore also relates to seed comprising a compound of the formula I, or an agriculturally useful salt of I, as defined herein. The amount of the compound I or the agriculturally useful salt thereof will in general vary from 0.1 g to 10 kg per 100 kg of seed, preferably from 1 g to 5 kg per 100 kg of seed, in particular from 1 g to 1000 g per 100 kg of seed.

The compounds of the invention may also be applied against non-crop insect pests, such as ants, termites, wasps, flies, mosquitos, crickets, or cockroaches. For use against said non-crop pests, compounds of formula I are preferably used in a bait composition.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickyness, moisture retention or aging characteristics.

The bait employed in the composition is a product, which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature and are known to those skilled in the art.

For use in bait compositions, the typical content of active ingredient is from 0.001 weight % to 15 weight %, desirably from 0.001 weight % to 5% weight % of active compound.

Formulations of compounds of formula I as aerosols (e.g in spray cans), oil sprays or pump sprays are highly suitable for the non-professional user for controlling pests such as flies, fleas, ticks, mosquitos or cockroaches. Aerosol recipes are preferably composed of the active compound, solvents such as lower alcohols (e.g. methanol, ethanol, propanol, butanol), ketones (e.g. acetone, methyl ethyl ketone), paraffin hydrocarbons (e.g. kerosenes) having boiling ranges of approximately 50 to 250 °C, dimethylformamide, N-methylpyrrolidone, dimethyl sulfoxide, aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate siabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

For use in spray compositions, the content of active ingredient is from 0.001 to 80 weights %, preferably from 0.01 to 50 weight % and most preferably from 0.01 to 15 weight %.

The compounds of formula I and its respective compositions can also be used in mosquito and fumigating coils, smoke cartridges, vaporizer plates or long-term vaporizers and also in moth papers, moth pads or other heat-independent vaporizer systems.

The present invention is now illustrated in further details by the following examples.

### S. Synthesis Examples

### Example S.1: 2-Benzyl-4-methyl-1,1-dioxo-1,2-dihydro-1λ⁶-isothiazolo[5,4-b]pyridine-3-ylidenamine (1)

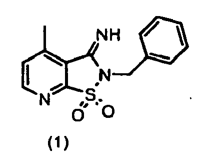

S.1.1 2-thio-3-cyano-4-methylpyridine: 2.00 g (19.6 mmol) of cyanothioacetamide and 1.39 g (19.6 mmol) of crotonaldehyde were dissolved in 100 ml of dry ethanol. At room temperature, 160 mg (1.96 mmol) of sodium ethoxide was added. The resulting solution was refluxed under an atmosphere of nitrogen for 7 h. After being cooled to room temperature, the reaction mixture was stirred at 0 °C for 90 min. The precipitate was collected by filtration and dried to afford to afford 0.60 g (3.79 mmol) of 2-thio-3-cyano-4-methylpyridine.

S.1.2 3-cyano-4-methyl-pyridirie-2-sulfonylchloride: 1.00 of 2-thio-3-cyano-4-methylpyridine was dissolved in 15 ml of a 1 N aqueous solution of HCl. At 0-5 °C Cl₂ gas was passed through the reaction mixture for 1 h. After that the reaction mixture was stirred at 5 °C for 30 minutes.
The precipitate was filtered off; it washed with ice water and dried to afford 2.0 g (∼6.6 mmol) of 3-cyano-4-methyl-pyridine-2-sulfonylchloride which was used without further purification.

S.1.3 2-Benzyl-4-methyl-1,1-dioxo-1,2-dihydro-1λ⁶-isothiazolo[5,4-b]pyridine-3-ylidene-amine (1): A solution of 1.0 g (3.3 mmol) of 3-cyano-4-methyl-pyridine-sulfonyl-chloride in 10 ml of tetrahydrofuran was added to a solution of 0.83 g (7.7 mmol) of benzylamine in 20 ml of tetrahydrofuran at - 5 °C. The reaction mixture was stirred at 0 °C for 2 hours before water was added. The THF was removed from the reaction mixture by evaporation. The aqueous phase was extracted three times with dichloromethane. The combined organic extracts were dried over sodium sulfate and filtered. The filtrate was concentrated in vacuo and purified by chromatography to afford 0.50 g (1.6 mmol) of the title compound.

The compounds nos. 1 to 5 of the formula I with Z = N, n = 2 and R₂ = H listed in the following table 1 were prepared analogously.

**Table 1:**

| | | | | | |
|---|---|---|---|---|---|
| 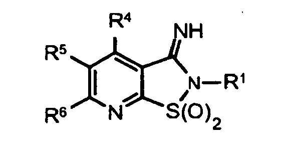 | | | | | |

| Example no. | R¹ | R⁴ | R⁵ | R⁶ | Physical property [m.p. [ °C] RT (HPLC/MS) or chemical shift (¹H-NMR)] |
|---|---|---|---|---|---|
| 1. | CH₂-C₆H₅ | CH₃ | H | H | ¹H-NMR |
| 2. | CH₂-CH₃ | CF₃ | H | CH₃ | 137-139 |
| 3. | CH₂-C₆H₅ | CF₃ | H | CH₃ | 126-129 |
| 4. | CH₂-C₆H₅ | CF₃ | H | C₆H₅ | ¹H-NMR |
| 5. | CH₂-CH₃ | CF₃ | H | C₆H₅ | ¹H-NMR |

Some compounds were characterized by ¹H-NMR. The signals are characterized by chemical shift (ppm) vs. tetramethylsilane, by their multiplicity and by their integral (relative number of hydrogen atoms given). The following abbreviations are used to characterize the multiplicity of the signals: m = multiplett, q = quartett, t = triplett, d = doublet and s = singulett.
- Example 1:: 2.45 (s, 3 H9, 4.25 (s, 2 H), 7.15-7.25 (m, 5 H), 7.75 (d, 1 H), 8.75 (d, 1 H), 8.9 (br. s, 1 H), d₆-DMSO
- Example 4:: 4.35 (s, 2 H), 7.15-7.2 (m, 3 H), 7.25 (m, 2 H), 7.55-7.65 (m, 3 H), 8.4 (dd, 2 H), 8.65 (s, 1 H), 9.3 (br. s, 1 H), d₆-DMSO
- Example 5:: 1.45 (t, 3 H), 3.95 (br. s, 2 H), 7.5-7.55 (m, 3 H), 8.15-8.2 (m, 2 H), 8.3 (s, 1 H), 8.9 (br. s, 1 H), CDCl₃

### Example S.2: 2-Benzyl-4-methyl-1,1-dioxo-1,2-dihydro-1λ⁶-isothiazolo[5,4-c]pyridine-3-ylideneamine (6)

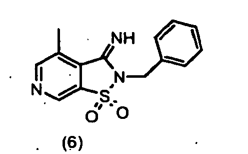

S.2.1. 5-Bromo-3,4-dimethylpyridine: 50 ml of concentrated sulfuric acid where cooled to 0 °C. 10.0 g (91.5 mmol) of 3,4-lutidin where added dropwise. The mixture was heated to 60 °C, and bromine was added slowly over a period of 30 min. After completion of the addition, the mixture was stirred at 60 °C for 90 min. After being cooled to room temperature, the reaction mixture was carefully poured on crushed ice. The aqueous solution was washed with dichloromethane. Then the aqueous solution was cooled to 0 °C, basified with a 50 % aqueous NaOH solution and extracted with dichloromethane (3ẋ). The combined organic layer was washed with water, dried over Na₂SO₄ and evaporated under reduced pressure. The crude product was purified by recrystallization to yield 11.4 g 52.1 mmol) of the title compound.

S.2.2. 3-Bromo-5-methyl-pyridine-4-carbaldehyde-O-methyloxime: 2.00 g (10.75 mmol) 5-bromo-3,4-dimethylpyridine and 1.52 g (14 mmol) n-butyl nitrite where dissolved in 10 ml of DMF and cooled to - 78 °C. 2.83 g (24.7 mmol) of potassium tertbutoxide dissolved in 10 ml of DMF where added dropwise over 10 minutes. The reaction mixture was stirred at - 50 °C for 30 minutes. Then a mixture of 3 ml of glacial acetic acid and 5 ml water was added at - 50 °C. The reaction mixture was warmed to room temperature, and 50 ml water was added. The precipitate was collected and dried to give 1.60 g (7.3 mmol) of the title compound.

S.2.3. 3-Bromo-5-methyl-isonicotinonitrile: 0.70 g (3.26 mmol) of 3-Bromo-5-methylpyridine-4-carbaldehyde-O-methyloxime where dissolved in 3 ml of acetic anhydride and refluxed for 4 hours. After completion the solvent was evaporated under reduced pressure. The solid residue was dissolved in dichloromethane, washed with water and dried over Na₂SO₄. The solvent was evaporated under reduced pressure to give 0.56 g (2.8 mmol) of the title compound as a yellow solid.

S.2.4. 3-methyl-5-propylsulfanyl-isonicotinonitrile: 1.00 g (5.1 mmol) of 3-bromo-5-rriethyl-isonicotinonitrile and 0.60 g (7.6 mmol) of propane-1-thiol where dissolved in 5 ml of DMF. At 0 °C, a solution of 0.67 g (10.2 mmol) KOH in 2 ml of water was added drop wise. The reaction mixture was stirred at 0 °C for 2 hours before 20 ml of water was added. The resulting precipitate was collected. The solid was washed with water and dried to give 0.95 g (4.7 mmol) of the title compound as a colorless solid having a melting point of 37-39 °C.

S.2.5 4-cyano-5-methyl-pyridin-2-sulfonylchloride: 0.9 g (4.7 mmol) of 2-thio-3-cyano-4-methyl-pyridine was dissolved in 15 ml of a 1 N aqueous solution of HCl. At 0-5 °C Cl₂ gas was passed through the reaction mixture for 1 h. After that the reaction mixture was stirred at 5 °C for 30 minutes. The emerging precipitate was filtered off; it washed with ice water and dried to afford 1.0 g (∼4.6 mmol) of the title compound, which was used without further purification.

S.2.6 2-Benzyl-4-methyl-1,1-dioxo-1,2-dihydro-1λ⁶-isothiazolo[5,4-c]pyridine-3-ylidene-amine (6): A solution of 1.6 g (7.4 mmol) of 4-cyano-5-methyl-pyridin-2-sulfonylchloride in 10 ml of tetrahydrofuran was added to a solution of 1.95 g (18 mmol) of benzylamine in 20 ml of tetrahydrofuran at -5 °C. The reaction mixture was stirred at 0 °C for 2 hours before water was added. The THF was removed from the reaction mixture by evaporation. The remaining aqueous mixture was extracted three times with dichloromethane. The combined organic extracts were dried over Na₂SO₄ and filtered. The filtrate was concentrated in vacuo. The residue was purified by flash chromatography to yield 0.2 g (0.66 mmol) of the title compound having a melting point of 157-159 °C.

### Example S.3: 2-Ethyl-4-methoxy-1,1-dioxo-1,2-dihydro-1λ⁶-isothiazolo[5,4-c]pyridine-3-ylideneamine (9)

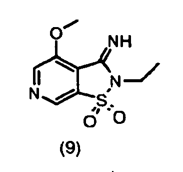

S.3.1. 3,5-dichloro-isonicotinonitrile: 30.0 g (170.5 mmol) 3,5-dichloro-4-pyridine carbaldehyde was dissolved in 200 ml of formic acid. 15.4 g (221.6 mmol) of hydroxylamine hydrochloride and 2 drops of concentrate sulfuric acid where added, and the resulting mixture was refluxed for 3.5 hours. The being cooled to room temperature, the solvent was evaporated under reduced pressure and the residue was redissolved in methyl tert-butyl ether. The resulting solution was washed with water, aqueous saturated NaHCO₃ solution and again with water. The mixture was dried over Na₂SO₄ and the solvent was evaporated under reduced pressure to give 28.3 g (163.6 mmol) of the title compound without further purification.

S.3.2 3-chloro-5-propylsulfanyl-isonicotinonitrile: At -78 °C, 6.47 g (85 mmol) of propane-1-thiol dissolved in 100 ml of dry THF was treated with 53.1 ml (85 mmol) of a 1.6 molar solution of nBuLi in hexane. The resulting milky white suspension was brought to room temperature and stirred for additional 15 minutes. In a separate flask, 14.0 g (80.9 mmol) of 3,5-dichloro-isonicotinonitrile where dissolved in 100 ml of dry THF. At room temperature, the lithium thiopropylate solution was slowly added over a period of 30 minutes. After the addition was completed, the resulting solution was further stirred for 1 hour, before the reaction was quenched by addition of 200 ml of saturated aqueous NH₄Cl solution. After addition of ethyl acetate, the phases where separated, and the aqueous layer was extracted with ethyl acetate (3x): The combined organic layers where washed with saturated NaCl solution, dried over Na₂SO₄ and evaporated. The residue slowly crystallized to give 16.95 g (79.7 mmol) of the title compound without further purification.

S.3.3. 3-methoxy-5-propylsulfanyl-isonicotinonitrile: 15.2 g (84.3 mmol) of a 30 % sodium methylate solution in methanol was dissolved in 50 ml of dry THF. 17.9 g (84.3 mmol) of 3-chloro-5-propylsulfanyl-isonicotinonitrile dissolved in 150 ml of dry THF was added at room temperature. The resulting mixture was refluxed for 2.5 hours. After the mixture was cooled to room temperature, water was added. The aqueous mixture was extracted with ethyl acetate (3x). The combined organic layers where washed with saturated NaCl solution, dried over Na₂SO₄ and evaporated. The residue was purified by flash chromatography to give 7.0 g (33.6 mmol) of the title compound as a colourless solid.

S.3.4. 4-cyano-5-methoxy-pyridine-3-sulfonylchloride: 1.80 g (7.4 mmol) of 3-methoxy-5-propylsulfanyl-isonicotinonitrile where dissolved in 30 ml of dichloromethane and 3 ml of water. At 0 °C Cl₂ gas was passed through the reaction mixture for 1 h. After that the reaction mixture was stirred at 5 °C for 30 minutes. The reaction was quenched by addition of water. After phase separation, the aqueous layer was extracted with dichloromethane (3x). The combined organic layer was washed with water and dried over Na₂SO₄ to afford 2.0 g (∼6.6 mmol) of the title compound, which was used without further purification.

S.3.5. 2-Ethyl-4-methoxy-1,1-dioxo-1,2-dihydro-1λ⁶-isothiazolo[5,4-c]pyridine-3-ylidene-amine (9): A solution of 10.0 g (31.2 mmol) of 4-cyano-5-difluormethoxy-pyridin-3-sulfonylchloride in 100 ml of tetrahydrofuran was added to a solution of 5.0 g (78.0 mmol) of ammoniak solution in 100 ml of tetrahydrofuran at 0 °C. The reaction mixture was stirred at 0 °C for 2 hours before water was added. The THF was removed from the reaction mixture by evaporation. The remaining aqueous mixture was extracted three times with dichloromethane. The combined organic extracts where dried over Na₂SO₄ and filtered. The filtrate was concentrated in vacuo. The residue was purified by flash chromatography to give 3.30 g (13.7 mmol) of the title compound.

### Example S.4: 4-Difluoromethoxy-1,1-dioxo-1,2-dihydro-1λ⁶-isothiazolo[5,4-c]pyridin-3-ylideneamine (13)

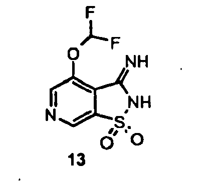

S.4.1. 3-hydroxy-5-propylsulfanyl-isonicotinonitrile: 9.4 g (45.1 mmol) of 3-methoxy-5-propytsulfanyl-isonicotinonitrile and 90.0 g of pyridinium chloride where placed in a flask. The mixture was heated until reflux. After the mixture was refluxed for 10 minutes, it was cooled to room temperature, and 1 I of water was added. The aqueous mixture was extracted with dichloromethane (3x). The combined organic layers where washed with water, dried over Na₂SO₄ and evaporated to give 6.2 g (31.9 mmol of the title compound, which was used without further purification.

S.4.2. 3-difluormethoxy-5-propylsulfanyl-isonicotinonitrile: 1.0 g (5.2 mmol) of the crude 3-hydroxy-5-propylsulfanyl-isonicotinonitrile was dissolved in 20 ml of DMF. 3.56 g (25.7 mmol) of K₂CO₃ was added. 0.81 g (6.2 mmol) of gaseous bromodifluoromethane was added, and the resulting mixture was stirred at room temperature for 5 h. Another 0.81 g (6.2 mmol) of gaseous bromodifluoromethane was added, and stirring was continued overnight. The mixture was poured into water and extracted with methyl tert-butyl ether (3x). The combined organic layers where washed with brine, dried over Na₂SO₄ and evaporated. The residue was purified by flash chromatography to give 0.46 g (1.89 mmol) of the title compound.

S.4.3. 4-cyano-5-difluormethoxy-pyridine-3-sulfonylchloride: 1.80 g (7.4 mmol) of 3-difluormethoxy-5-propylsulfanyl-isonicotinonitrile where dissolved in 30 ml of dichloromethane and 3 ml of water. At 0 °C Cl₂ gas was passed through the reaction mixture for 1 h. After that the reaction mixture was stirred at 5 °C for 30 minutes. The reaction was quenched by addition of water. After phase separation, the aqueous layer was extracted with dichloromethane (3x). The combined organic layer was washed with water and dried over Na₂SO₄ to afford 2.0 g (∼6.6 mmol) of the title compound, which was used without further purification.

S.4.4. 4-Difluoromethoxy-1,1-dioxo-1,2-dihydro-1λ⁶-isothiazolo[5,4-c]pyridin-3-ylidene-amine (13): A solution of 2.0 g (7.5 mmol) of 4-cyano-5-difluormethoxy-pyridin-3-sulfonylchloride in 10 ml of tetrahydrofuran was added to a solution of 1.3 g (18.8 mmol) of a 25% aqueous ammoniak solution in 20 ml of tetrahydrofuran at 0 °C. The reaction mixture was stirred at 0 °C for 2 hours before water was added. The THF was removed from the reaction mixture by evaporation. The remaining aqueous mixture was extracted three times with dichloromethane. The combined organic extracts where dried over Na₂SO₄ and filtered. The filtrate was concentrated to give a solid, which was recrystallized to afford 0.36 g (1.37 mmol) of the title compound.

The compounds nos. 6 to 14 of the formula I with Y = N, n = 2 and R₂ = H listed in the following table 2 were prepared analogously.

**Table 2:**

| | | | | | |
|---|---|---|---|---|---|
| 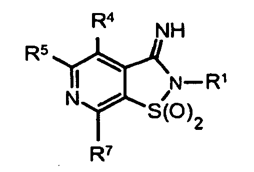 | | | | | |

| Example no. | R¹ | R⁴ | R⁵ | R⁷ | Physical property [m.p. [ °C] RT (HPLC/MS) or chemical shift (¹H-NMR)] |
|---|---|---|---|---|---|
| 6. | CH₂-C₆H₅ | CH₃ | H | H | 157-159 |
| 7. | H | OCH₃ | H | H | ¹H-NMR |
| 8. | CH₃ | OCH₃ | H | H | 129-136 |
| 9. | CH₂-CH₃ | OCH₃ | H | H | 96-100 |
| 10. | C(CH₃)₃ | OCH₃ | H | H | ¹H-NMR |
| 11. | Cyclopropyl | OCH₃ | H | H | ¹H-NMR |
| 12. | C(=O)CH₂-CH₃ | OCH₃ | H | H | ¹H-NMR |
| 13. | H | OCHF₂ | H | H | ¹H-NMR |
| 14. | CH₃ | OCHF₂ | H | H | ¹H-NMR |
| 15. | CH₂-CH₃ | OCHF₂ | H | H | ¹H-NMR |
| 16. | NHCH₃ | NHCH₃ | H | H | ¹H-NMR |
| 17. | NHCH₂CH₃ | NHCH₂CH₃ | H | H | ¹H-NMR |

Some compounds were characterized by ¹H-NMR. The signals are characterized by chemical shift (ppm) vs. tetramethylsilane, by their multiplicity and by their integral (relative number of hydrogen atoms given). The following abbreviations are used to characterize the multiplicity of the signals: m = multiplett, q = quartett, t = triplett, d = doublet and s = singulett.
- Example 7:: 4.15 (s, 3 H), 8.1 (br. s, 1 H), 8.8 (s, 1 H), 8.85 (s, 1 H), 9.35 (br. s, 1 H), d₆-DMSO
- Example 10:: 1.15 (s, 9 H), 4.15 (s, 3 H), 8.25 (br. s, 1 H), 8.75 (s, 1 H), 8.95 (s, 1 H), d₆-DMSO
- Example 11:: 0.55-0.65 (m, 4 H), 2.4 (m, 1 H), 4.15 (s, 3 H), 7.6 (br. s, 1 H), 8.7 (s, 1 H), 8.95 (s, 1 H), CDCl₃
- Example 12:: 1.2 (t, 3 H), 3.1 (q, 2 H), 4.3 (s, 3 H), 8.75 (s, 1 H), 8.85 (s, 1 H), 9.15 (br. s, 1 H), CDCl₃
- Example 13:: 7.45 (t, 1 H), 8.1 (br. s, 1 H), 8.9 (s, 1 H), 9.15 (s, 1 H), 9.5 (br. s, 1 H), d₆-DMSO
- Example 14:: 2.60 (s, 3 H), 7.55 (t, 1 H), 8.35 (br. s, 1 H), 9.0 (s, 1 H), 9.05 (s, 1 H), d₆-DMSO
- Example 15:: 1.05 (t, 3 H), 3.0 (q, 2 H), 7.55 (t, 1 H), 8.45 (br. s, 1 H), 9.0 (s, 1 H), 9.05 (s, 1 H), d₆-DMSO
- Example 16:: 3.1 (d, 3 H), 3.15 (s, 3 H), 7.65 (br. s, 1 H), 8.35 (s, 1 H), 8.35 (s, 1 H), CDCl₃
- Example 17:: 1.35 (t, 3 H), 1.35 (t, 3 H), 3.4 (m, 2 H), 3.65 (q, 2 H), 7.7 (br. s, 1 H), 8.35 (s, 1 H9, 8.4 (s, 1 H), CDCl₃

### Example S.5: Diethyl-(4-methoxy-1,1-dioxo-1H-λ⁶-isothiazolo[5,4-c]pyridine-3-yl)-amine (20)

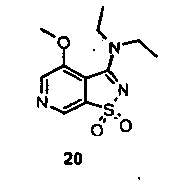

S.5.1. 5-Methoxy-pyridine-3-sulfonic acid tertbutylamide: 8.65 g (50 mmol) 3-bromo-5-methoxypyridine was dissolved in 200 ml of dry THF under an argon athomosphere. The solution was cooled to -78 °C, and a solution of 2.5 M N-butyllithium (52 mmol) in hexane was added. The resulting mixture was stirred at -78 °C for 30 min. In a separate flask gaseous SO₂ (around 25 g) was introduced into 50 ml of dry THF. The resulting SO₂-solution was added to the lithiated starting material dropwise at -78 °C. The resulting mixture was allowed to come to 0 °C and was further stirred at 0 °C for 1 h. Then sulfuryl chloride (100 mmol) was added dropwise at 0 °C, and the resulting mixture was further stirred at 0 °C for 30 min, before the reaction was allowed to come to room temperature. After 1 h, the reaction was quenched by addition of water. The aqueous solution was extracted with dichloromethane. The organic layer was dried over Na₂SO₄; it was filtered arid evaporated. The residue, the crude sulfonyl chloride, was dissolved in 100 ml of THF and added to a solution of tert-butyl amine (200 mmol) in 200 ml THFat 0 °C. The resulting mixture was stirred at 0 °C for 3 h before the reaction was quenched by addition of water. The aqueous solution was extracted with dichloromethane. The organic layer was dried over Na₂SO₄; it was filtered and evaporated. The residue was purified by flash chromatography to give 7.82 g (32.0 mmol) of the title compound.

S.5.2. 2-tert-butyl-4-methoxy-1,1-dioxo-1,2dihydro-λ⁶-isothiazolo-[5,4-c]pyridin-3-one: 7.33 g (30 mmol) 5-methoxy-pyridine-3-sulfonic acid tertbutylamide was dissolved in 150 ml of dry THF. The solution was cooled to -78 °C, and a solution of 2.5 M lithium diisopropyl amide (LDA) (75 mmol) in THF was added. The resulting mixture was stirred at - 78 °C for 2 h. Then the mixture was poured on freshly crudes dry ice in 100 ml of dry THF. The resulting mixture was stirred for 30 min before the reactuion was quenched by addition of aqueous saturated NH₄Cl solution. After addition of ethyl acetate the phases were separated. The aqueous layer was extracted with ethyl acetate. The combined organic layer was dried over Na₂SO₄; it was filtered and evaporated. The residue was dissolved in 100 ml of dry THF and cooled to 0 °C. POCl₃ (50 mmol) was added, and the resulting mixture was stirred at rt for 1 h. Then the reaction mixture was poured on crushed ice. After addition of ethyl acetate the phases were separated. The aqueous layer was extracted with ethyl acetate. The combined organic layer was dried over Na₂SO₄; it was filtered and evaporated. The residue was purified by flash chromatography to give 5.14 g (19.0 mmol) of the title compound.

S.5.3. 4-Methoxy-1,1-dioxo-1,2dihydro-λ⁶-isothiazolo-[5,4-c]pyridin-3-one: 6.0 g (22.0 mmol) 2-tert-butyl-4-methoxy-1,1-dioxo-1,2dihydro-λ⁶-isothiazolo-[5,4-c]pyridin-3-one were dissolved in 50 ml CF₃COOH and heated to reflux for 44 h. Upon cooling to rt, a precipitate formed that was collected by filtration to afford after drying 3.0 g (14.3 mmol) of the title compound.

S.5.4. 3-Chloro-4-methoxy-isothiazolo[5,a-c]pyridine-1,1-dioxide: 3.0 g (14.0 mmol) 4-methoxy-1,1-dioxo-1,2dihydro-λ⁶-isothiazolo-[5,4-c]pyridin-3-one were dissolved in 50 ml 1,4-dioxane at rt. 0.11 ml (1.4 mmol) DMF was added, followed by 4.0 ml (56.0 mmol) SOCl₂. The solution was heated to reflux for 44 h. After cooling to room temperature, all volatiles were removed by distillation and the obtained title compound was used without further purification.

S.5.5. Diethyl-(4-methoxy-1,1-dioxo-1H-λ⁶-isothiazolo[5,4-c]pyridine-3-yl)-amine (20): 430 mg (1.84 mmol) 3-Chloro-4-methoxy-isothiazolo[5,a-c]pyridine-1,1-dioxide were dissolved in 15 ml THF and cooled to 0 °C. 0.29 ml (2.76 mmol) diethyl amine were added and stirring was continued for 1 h at 0 °C and 18 h at room temperture. Saturated NH₄Cl-solution was added and the layers were separated. The aqueous layer was extracted three times with dichloro-methane. The combined organic layers were dried over MgSO₄ and concentrated. The residue was dissolved in 15ml ethyl acetate and cyclohexane was added until a precipitate was formed. The precipitate was collected by suction and purified on silica (cyclohexane/ethyl acetate 1:2) to yield 77 mg (0.29 mmol) of the title compound.

The compounds nos. 18 to 21 of the formula I with Y = N, n = 2 and R₁ = H listed in the following table 2 were prepared analogously.

**Table 3:**

| 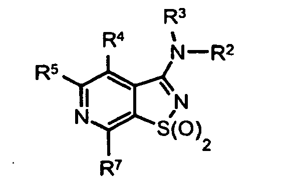 | | | | | | |
|---|---|---|---|---|---|---|
| Example no. | R² | R³ | R⁴ | R⁵ | R⁷ | Physical property [m.p. [ °C]; RT (HPLC/MS) or chemical shift (¹H-NMR)] |
| 18. | H | CH₂-CH₃ | OCH₃ | H | H | 172-175 |
| 19. | CH₃ | CH₂-CH₃ | OCH₃ | H | H | 162 |
| 20. | CH₂-CH₃ | CH₂-CH₃ | OCH₃ | H | H | 176-177 |
| 21. | CH₃ | C(=O)CH₃ | OCH₃ | H | H | 180-181 |

### B. Examples of action against pests

The active conmpounds were formulated in a mixture of 50 vol.-% acetone:50 vol.-% water. A nonionic surfactant (Kinetic®) was included in the solution at a volume of 0.01 % v/v.

In the following tests, the formulated solutions of the active compounds were diluted to an active ingredient concentration of 300 ppm and the diluted solutions were applied in the below mentioned tests.

The action of the compounds of the formula I against pests was demonstrated by the following experiments:

### B.1 Cotton aphid (aphis gossypii), mixed life stages

Cotton plants at the cotyledon stage were infested prior to treatment by placing a heavily infested leaf from the main aphid colony on top of each cotyledon. The aphids were allowed to transfer overnight and the host leaf was removed. The infested cotyledons were then dipped and agitated in the test solution for 3 seconds and allowed to dry in a fume hood. Test plants were maintained under fluorescent lighting in a 24-hr photoperiod at 25°C and 20-40% relative humidity. Aphid mortality on the treated plants, relative to mortality on untreated check plants, was determined after 5 days.

In this test compounds each of examples 6-15 and 18-21 at 300 ppm provided at least 90 % mortality of cotton aphid (Aphis gossypii, mixed life stages) in comparison with untreated controls.

### B.2 Green Peach Aphid (Myzus persicae), mixed life stages

Bell pepper plants at the first true-leaf stage were infested prior to treatment by placing heavily infested leaves from the main aphid colony on top of the treatment plants. The aphids were allowed to transfer overnight to accomplish an infestation of 30-40 aphids per plant and the host leaves were removed. The infested leaves of the test plants were then dipped and agitated in the test solution for 3 seconds and allowed to dry in a fume hood. Test plants were maintained under fluorescent lighting in a 24-hr photoperiod at 25°C and 20-40% relative humidity. Aphid mortality on the treated plants, relative to mortality on untreated check plants, was determined after 5 days.

In this test compounds each of examples 6-15 and 18-21 at 300 ppm provided at least 90 % mortality of green peach aphidin comparison with untreated controls.

### B.3 Bean Aphid (Aphis fabae)

Nasturtium plants grown in Metro mix in the 1^{st} leaf-pair stage (variety'Mixed Jewel') were infested with approximately 2-30 laboratory-reared aphids by placing infested cut plants on top of the test plants. The cut plants were removed after 24 hr. Each plant was dipped into the test solution to provide complete coverage of the foliage, stem, protruding seed surface and surrounding cube surface and allowed to dry in the fume hood. The treated plants were kept at about 25°C with continuous fluorescent light. Aphid mortality is determined after 3 days.
In this test compounds each of examples 6-15 and 18-21 at 300 ppm provided at least 90 % mortality of bean aphid in comparison with untreated controls.

## Claims

1. A Isothiazolopyridin-3-ylenamine compound of the formula I 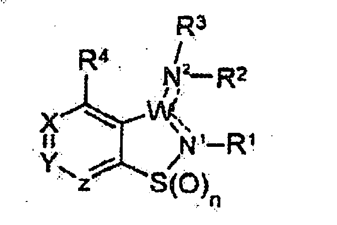 where
n is 2;
X is N, N-O or C-R⁵;
Y is N, N-O or C-R⁶;
Z is C-R⁷;
with the proviso that one of the variables X or Y, at least one and not more than one, is N or NO;
W is C, which is either connected to N¹ with a double bond and to N² with a single bond or connected to N² with a double bond and to N¹ with a single bond
If W is connected to N¹ with a single bond and to N² with a double bond, then N² is carrying only the substituent R² and not the substituent R³. In this case, R² is hydrogen, and R¹ is selected from the group consisting of selected from the group consisting of hydrogen, C(=O)-R⁸, C₁-C₁₀-alkyl, C₂-C₆-alkenyl, C₂-C₁₀-alkinyl, C₁-C₁₀-alkoxy or C₃-C₁₀-cycloalkyl, wherein the five last-mentioned radicals may be unsubstituted, partially or fully halogenated and/or may carry 1, 2 or 3 radicals, independently of one another each selected from the group consisting of cyano, nitro, amino, C₁-C₁₀-alkoxy, C₁-C₁₀-alkylthio, C₁-C₁₀-alkylsulfinyl, C₁-C₁₀-alkylsulfonyl, C₁-C₁₀-haloalkoxy_{,} C₁-C₁₀-haloalkylthio, C₁-C₁₀-alkoxycarbonyl, (C₁-C₁₀-alkyl)amino, di-(C₁-C₁₀-alkyl)amino, C₃-C₁₀-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy;
If W is connected to N² with a single bond and to N¹ with a double bond, then N¹ is not carrying R¹, In this case, R² and R³ are, of one another, selected from the group consisting of hydrogen, C(=O)-R⁸, C₁-C₁₀-alkyl, C₂-C₆-alkenyl, C₂-C₁₀-alkinyl, C₁-C₁₀-alkoxy or C₃-C₁₀-cycloalkyl, wherein the five last-mentioned radicals may be unsubstituted, partially or fully halogenated and/or may carry 1,2 or 3 radicals, independently of one another each selected from the group consisting of cyano, nitro, amino, C₁-C₁₀-alkoxy, C₁-C₁₀-alkythio, C₁-C₁₀-alkylsulfinyl, C₁-C₁₀-alkylsulfonyl, C₁-C₁₀-haloalkoxy, C₁-C₁₀-haloalkylthio, C₁-C₁₀-alkoxycarbonyl, (C₁-C₁₀-alkyl)amino, di-(C₁-C₁₀-alkyl)amino, C₃-C₁₀-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy; or
R² and R³ together with the adjacent nitrogen form a 3 to 10-membered ring, optionally substituted by 1, 2 or 3 C₁-C₅-alkyl radicals, wherein the ring may contain, in addition to the nitrogen and carbon ring members, 1, 2 or 3 heteroatoms as ring members selected from the group consisting of nitrogen, oxygen, sulfur, a group SO, SO₂ or N-R⁹;
R⁴ is hydrogen, nitro, cyano, azido, amino, halogen, sulfonylamino, sulfenylamino, sulfinylamino, C(=O)R⁹,
C₁-C₆-alkyl, C₂-C₆-alkenyl, C₂-C₆-alkinyl, C₃-C₆-cycloalkyl, C₁-C₆-alkoxy, C₁-C₆-alylthio, (C₁-C₆-alkyl)amino, di(C₁-C₈-alkyl)amino, C₂-C₆-alkylsulfinyl. C₁-C₆-alkylsulfenyl or C₁-C₆-alkylsulfonyl, wherein the eleven last-mentioned radicals may be unsubstituted, partially or fully halogenated and/or may carry 1, 2 or 3 radicals, selected from the group consisting of cyano, nitro, amino, C₁-C₄-alkoxy, C₁-C₄-alkylthio, C₁-C₄-alkylsulfinyl, C₁-C₄-alkylsulfonyl, C₁-C₄-haloalkoxy, C₁-C₄-haloalkylthio, (C₁-C₄-alkoxy)carbonyl, (C₁-C₄-alkyl)amino, di(C₁-C₄-alkyl)amino, C₃-C₈-cycloalkyl and phenyl, it being possible for phenyl to be unsubstituted, partially or fully halogenated and/or to carry 1, 2 or 3 substituents, independently of one another selected from the group consisting of C₁-C₄-alkyl, C₁-C₄-haloalkyl, C₁-C₄-alkoxy and C₁-C₄-haloalkoxy;
R⁵, R⁶ and R⁷ are independently of one another selected from the group consisting of hydrogen, halogen, cyano, azido, nitro, C₁-C₆-alkyl, C₃-C₆-cycloalkyl, C₁-C₄-haloalkyl, C₁-C₄-alkoxy, C₁-C₄-alkylthio, C₁-C₄-alkylsufinyl, C₁-C₄-alkylsutfonyl, C₁-C₄-haloalkoxy, C₁-C₄-haloalkylthio, C₂-C₆-alkenyl, C₂-C₆-alkinyl, (C₁-C₄-alkoxy)carbonyl, amino. (C₁-C₄-alkyl)amino, di(C₁-C₄-alkyl)amino, aminocarbonyl, (C₁-C₄-alkyl)aminocarbonyl, di(C₁-C₄-alkyl)aminocarbonyl, sulfonyl, sulfonylamino, sulfenylamino, sulfanylamino and C(=O)-R¹¹;
R⁸ is C₁-C₆-alkyl, aryl, aryl-C₁-C₄-alkyl, 3- to 7-membered heteroaryl or heteroaryl-C₁-C₄-alkyl, wherein the heteroaryl ring contains as ring members 1,2 or 3 heteroatoms, selected from the group consisting of nitrogen, oxygen, sulfur, a group SO, SO₂ or N-R¹², wherein R¹² is hydrogen or C₁-C₄-alkyl;
3- to 7-membered heterocyclyl or heterocyclyl-C₁-C₄-alkyl, wherein the heterocyclic ring contains as ring members 1,2 or 3 heteroatoms, selected from the group consisting of nitrogen, oxygen, sulfur a group SO, SO₂ or N-R¹³, wherein. R¹³ is hydrogen or C₁-C₄-alkyl;
and wherein the carbon atoms of the heterocyclic rings may by unsubstituted or substituted by 1 or 2 C₁-C₄-alkyl groups;
R⁹ is hydrogen or C₁-C₄-alkyl
R¹⁰ and R¹¹, independently of one another, are hydrogen, hydroxy, C₁-C₁₀-alkoxy amino, C₁-C₄-alkyl, aryl, aryl-C₁-C₄-alkyl,
3- to 7-membered heteroaryl or heteroaryl-C₁-C₄-alkyl, wherein the heteroaryl ring contains as ring members 1, 2 or 3 heteroatoms, selected from the group consisting of nitrogen, oxygen, sulfur a group SO, SO₂ or N-R¹³, wherein R¹³ is hydrogen or C₁-C₄-alkyl;
3- to 7-membered heterocyclyl or heterocyclyl C₁-C₄-alkyl, wherein the heterocyclic ring contains 1, 2 or 3 heteroatoms, selected from the group to consisting of nitrogen, oxygen, sulfur, a group SO, SO₂ or N-R¹⁴, wherein R¹⁴ is hydrogen or C₁-C₄-alkyl; and wherein the carbon atoms of the heterocyclic rings may by unsubstituted or substituted by 1 or 2 C₁-C₄-alkyl groups;
and/or the agriculturally acceptable salts thereof.

2. A compound of the formula I as defined in claim 1, wherein X is C-R⁵ and Y is N or NO.

3. A compound of the formulae I as defined in claim 1, wherein X is C-R⁵ and Y is N.

4. A compound according to claim 1,2 or 3, wherein either R⁵ or R⁷ is hydrogen and the other radical R⁵ or R⁷ is selected from halogen, C₁-C₄-alkyl or C₁-C₄-haloalkyl

5. A compound according to claim 1.2 or 3, wherein either R⁵ or R⁷ is hydrogen and the other radical R⁵ or R⁷ is selected from halogen, C₁-C₂-alkyl, or C₁-haloalkyl

6. A compound according to claim 1,2 or 3, wherein either R⁵ or R⁷ is hydrogen and the other radical R⁶ or R⁷ is selected from F, Cl, Br, 1, CH₃ or CF₃

7. A compound according to any of the precedding claims, wherein R⁴ is hydrogen.

8. A compound according to arty of the preceeding claims, wherein both R⁵ and R⁷ are hydrogen.

9. An agricultural composition comprising such an amount of at least one compound of the formula I and/or at least one agriculturally useful salt of I, as defined in any of claims 1 to 8, and at least one inert liquid and/or solid agronomically acceptable, carrier and, if desired, at least one surfactant

10. The use of an Isothiazolopyridin-3-ylenamine compound of the formula I or a salt thereof as defined in any of claims 1 to 8 for combating animal pests.

11. A method of combating animal pests excluding a therapeutic method which comprises contacting the animal pests, their habit, breeding ground, food supply, plant, seed, soil, area, material or environment in which the animal pests are growing or may grow, or the materials, plants, seeds, soils, surfaces: or spaces to be protected from animal attack or infestation with a pesticidally effective amount of at least one compound of the formula I and/or at least one agriculturally acceptable salt thereof, as defined in any of claims 1 to 8.

12. A method as defined in claim 11, where the animal pest is from the order Homoptera or Thysanoptera.

13. A method for protecting crops from attack or infestation by animal pests, which comprises contacting a crop with a pesticidally effective amount of at least one compound of the formula I and/or at least one salt thereof, as defined in any of claims 1 to 8.

14. A method for the protection of seeds from soil insects and of the seedlings' roots and shoots fro m insects comprising contacting the seeds before sowing and/or after pregermination with a compound of the formula I as defined in any of claims 1 to 8 and/or at least one agriculturally acceptable salt thereof, in pesticidally effective amounts.

15. The method according claim 14, wherein the compound of formula I is applied in an amount of from 0.1 g to 10 kg per 100 kg of seeds.

16. A method according to claim 14 or 15 wherein of the resulting plant's roots and shoots are protected.

17. A method according to claim 14 or 15, wherein the resulting plant's shoots are protected from aphids.

18. Seed comprising a compounds of the formula or an agriculturally useful salt of 1, as defined in any of claims 1 to 8, in an amount of from 0.1 g to 10 kg per 100 kg of seed.

## Patentansprüche

1. Isothiazolopyridin-3-ylenamin-Verbindung nach Formel I, 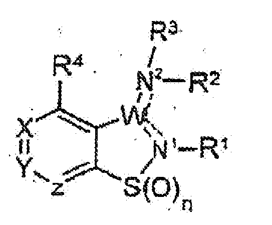 mit den Bedeutungen:
n bedeutet 2;
X bedeutet N, N-O oder C-R⁵;
Y bedeutet N, N-O oder C-R⁶;
Z bedeutet C-R⁷;
mit der Maßgabe, daß eine der Variablen X oder Y, mindestens eine und nicht mehr als eine, N oder NO bedeutet;
W bedeutet C, das entweder an N¹ über eine Doppelbindung und an N² mit einer einfachen Bindung gebunden ist oder an N² über eine Doppelbindung und an N¹ über eine einfache Bindung gebunden ist
wenn W an N¹ über eine einfache Bindung und an N² über eine Doppelbindung gebunden ist, dann trägt N² nur den Substituenten R² und nicht den Substituenten R³; in diesem Fall bedeutet R² Wasserstoff und R¹ stammt aus der Gruppe bestehend aus Wasserstoff, C(=O)-R⁶, C₁-C₁₀-Alkyl, C₂-C₆-Alkenyl, C₂-C₁₀-Alkinyl, C₁-C₁₀-Alkoxy oder C₃-C₁₀-Cycloalkyl, wobei die letztgenannten fünf Reste unsubstituiert, teilweise oder ganz halogeniert sein können und/oder 1, 2 oder 3 Reste tragen können, die unabhängig voneinander jeweils aus der Gruppe bestehend aus Cyano, Nitro, Amino, C₁-C₁₀-Alkoxy, C₁-C₁₀-Alkylthio, C₁-C₁₀-Alkylsulfinyl, C₁-C₁₀-Alkylsulfonyl, C₁-C₁₀-Halogenalkoxy, C₁-C₁₀-Halogenalkylthio, C₁-C₁₀-Alkoxycarbonyl, (C₁-C₁₀-Alkyl)amino, Di-(C₁-C₁₀-alkyl)amino, C₃-C₁₀-Cycloalkyl und Phenyl stammen, wobei das Phenyl unsubstituiert, teilweise oder ganz halogeniert sein kann und/oder 1, 2 oder 3 Substituenten tragen kann, die unabhängig voneinander aus der Gruppe bestehend aus C₁-C₄-Alkyl, C₁-C₄-Halogen-alkyl, C₁-C₄-Alkoxy und C₁-C₄-Halogenalkoxy stammen;
wenn W an N² über eine einfache Bindung und an N¹ über eine Doppelbindung gebunden ist, dann trägt N¹ nicht R¹; in diesem Fall stammen R² und R³ unabhängig voneinander aus der Gruppe bestehend aus Wasserstoff, C(=O)-R⁶, C₁-C₁₀-Alkyl, C₂-C₆-Alkenyl, C₂-C₁₀-Alkinyl, C₁-C₁₀-Alkoxy oder C₃-C₁₀-Cycloalkyl, wobei die letztgenannten fünf Reste unsubstituiert, R⁴ teilweise oder ganz halogeniert sein könnten und/oder 1, 2 oder 3 Reste tragen können, die unabhangig voneinander jeweils aus der Gruppe bestehend aus Cyano, Nitro, Amine, C₁-C₁₀-Alkoxy, C₁-C₁₀-Alkylthio, C₁-C₁₀-Alkylsulfinyl, C₁-C₁₀-Alkylsulfonyl, C₁-C₁₀-Halogenalkoxy, C₁-C₁₀-Halogenalkylthio, C₁-C₁₀-Alkoxycarbonyl, (C₁-C₁₀-Alkyl) amino, Di-(C₁-C₁₀-alkyl)amino, C₃-C₁₀-Cycloalkyl und Phenyl stammen, wobei das Phenyl unsubstituiert, teilsweise oder ganz halogeniert sein kann und/oder 1, 2 oder 3 Substituenten tragen kann die unabgängig voneinander aus der Gruppe bestehend aus C₁-C₄-Alkyl, C₁-C₄-Halogen-alkyl, C₁-C₄-Alkoxy und C₁-C₄-Halogenalkoxy stammen oder
R² und R³ bilden gemeinsam mit dem benachbarten Stickstoff einen 3- bis 10-gliedrige Ring, der gegebenenfalls durch 1, 2 oder 3 C₁-C₅-Alkyl-reste substituiert ist, wobei der Ring zusätzlich zu den Ringgliedern Stickstoff und Kohlenstoff 1, 2 oder 3 Heteroatome aus der Gruppe bestehend aus Stickstoff, Sauerstoff, Schwefel, SO-Gruppe, SO₂-Gruppe oder N-R⁹-Gruppe als Ringglieder enthalten kann;
R⁴ bedeutet Wasserstoff, Nitro, Cyano, Azido, Amino, Halogen, Sulfonylamino, Sulfenylamino, Sulfinylamino, C(=O)R⁹;
G₁-C₆-Alkyl, C₂-C₆-Alkenyl, C₂-C₆-Alkinyl, C₃-C₆-Cycloalkyl, C₁-C₆-Alkoxy, C₁-C₆-Alkylthio, (C₁-C₆-Alkyl) amino, Di-(C₁-C₆-alkyl)amino, C₁-C₆-Alkylsulfinyl, C₁-C₆-Alkylsulfenyl oder C₁-C₆-Alkylsulfonyl, wobei die letztgenannte elf Reste unsubstituiert, teilweise oder ganz halogeniert sein können und/oder 1, 2 oder 3 Reste aus der Gruppe bestehend aus Cyano, Nitro, Amino, C₁-C₄-Alkoxy, C₁-C₄-Alkylthio, C₁-C₄-Alkylsulfinyl, C₁-C₄Alkylsulfonyl C₁-C₄-Halogenalkoxy, C₁-C₄-Halogenalkylthio, (C₁-C₄-Alkoxy)carbonyl, (C₁-C₄-Alkyl)amino, Di(C₁-C₄-Alkyl)amino, C₃-C₈-Cycloalkyl und Phenyl tragen können, wobei das Phenyl unsubstituiert, teilweise oder ganz halogeniert sein kann und/oder 1, 2 oder 3 Substituenten tragen kann, die unabhängig voneinander aus der Gruppe bestehend aus C₁-C₄-Alkyl, C₁-C₄-Halogenalkyl, C₁-C₄-Alkoxy und C₁-C₄-Halogenalkoxy stammen;
R⁵, R⁶ und R⁷ stammen unabhängig voneinander aus der Gruppe bestehend aus Wasserstoff, Halogen, Cyano, Azido, Nitro, C₁-C₆-Alkyl, C₃-C₈-Cycloalkyl, C₁-C₄-Halogenalkyl, C₁-C₄-Alkoxy, C₁-C₄-Alkylthio, C₁-C₄-Alkylsulfinyl, C₁-C₄-Alkylsulfonyl, C₁-C₄-Halogenalkoxy, C₁-C₄-Halogen-alkylthio, C₂-C₆-Alkenyl, C₂-C₆-Alkinyl, (C₁-C₄-Alkoxy)carbonyl, Amino, (C₁-C₄-Alkyl)amino, Di(C₁-C₄-Alkyl)amino, Aminocarbonyl, (C₁-C₄-Alkyl)aminocarbonyl, Di(C₁-C₄-Alkyl)aminocarbonyl, Sulfonyl, Sulfonylamino, Sulfenylamino, Sulfanylamino und C(=O)-R¹¹_{;}
R⁸ bedeutet C₁-C₆-Alkyl, Aryl, Aryl-C₁-C₄-alkyl, 3- bis 7-gliedriges Heteroaryl oder Heteroaryl-C₁-C₄-alkyl, wobei der Hetercarylring 1, 2 oder 3 Heteroatome aus der Gruppe bestehend aus Stickstoff, sauerstoff, Schwefel, SO-Gruppe, SO₂-Gruppe oder N-R¹²-Gruppe, wobei R¹² Wasserstoff oder C₁-C₄-Alkyl bedeutet, als Ringglieder enthält;
3- bis 7-gliedrige Heteroaryl oder Heterocyclyl-C₁-C₄-alkyl, wobei der Heterocyclylring 1, 2 oder 3 Heteroatome aus der Gruppe bestehend aus Stickstoff, Sauerstoff, Schwefel, SO-Gruppe, SO₂-Gruppe oder N-R¹³-Gruppe, wobei R¹³ Wasserstoff oder C₁-C₄-Alkyl bedeutet, als Ringglieder enthält; und wobei die Kohlenstoffatome der heterocyclischen Ringe unsubstituiert oder durch 1 oder 2 C₁-C₄-Alkylgruppen substituiert sein können;
R⁹ bedeutet Wasserstoff oder C₁-C₄-Alkyl
R¹⁰ und R¹¹ bedeuten unabhangig voreinander Wasserstoff, Hydroxy, C₁-C₆-Alkoxy, Amino, C₁-C₄-Alkyl, Aryl, Aryl-C₁-C₄-alkyl,
3- bis 7-gliedrige Heteroaryl oder Heteroaryl-C₁-C₄-alkyl, wobei der Heteroarylring 1, 2 oder 3 Heteroatome aus der Gruppe bestehend aus Stickstoff, Sauerstoff, Schwefel, SO-Gruppe, SO₂-Gruppe oder N-R¹³-Gruppe, wobei R¹³ Wasserstoff oder C₁-C₄-Alkyl bedeutet, als Ringglieder enthält;
3- bis 7-gliedriges Heteroaryl oder Heterocyclyl-C₁-C₄-alkyl, wobei der Heterocyclylring 1, 2 oder 3 Heteroatome aus der Gruppe bestehend aus Stickstoff, Sauerstoff, Schwefel, SO-Gruppe, SO₂-Gruppe oder N-R¹⁴-Gruppe, wobei R¹⁴ Wasserstoff oder C₁-C₄-Akyl bedeutet, als Ringglieder enthält;
und wobei die Kohlenstoffatome der heterocyclischen Ringe unsubstituiert oder durch 1 oder 2 C₁-C₄-Alkylgruppen substituiert sein können;
und/oder ihre landwirtschaftlich annehmbaren Salze.

2. Verbindung der Formel 1 wie in Anspruch 1 definiert, wobei X C-R⁵ bedeutet und Y N oder NO bedeutet.

3. Verbindung der Formel I wie in Anspruch 1 definiert, wobei X C-R⁵ bedeutet und Y N bedeutet.

4. Verbindung nach Anspruch 1, 2 oder 3, wobei einer der Reste R⁵ oder R⁷ Wasserstoff bedeutet und der andere Rest R⁵ oder R⁷ aus der Reihe Halogen, C₁-C₄-Alkyl oder C₃-C₄-Halogenalkyl stammt.

5. Verbindung nach Anspruch 1, 2 order 3, wobei einer der Reste R⁵ oder R⁷ Wasserstoff bedeutet und der andere Rest R⁵ ader R⁷ aus der Reihe Halogen, C₁-C₂-Alkyl oder C₁₋Halogenalkyl stammt.

6. Verbindung noch Anspruch 1, 2 oder 3 wobei einer der Reste R⁵ oder R⁷ Wasserstoff bedeutet und der andere Rest R⁵ oder R⁷ aus der Reiche F, Cl, Br, I, CH₃ oder CF₃ stammt.

7. Verbindung nach einem der vorhergehenden Ansprüche, wobei R⁴ Wasserstoff bedeutet.

8. Verbindung nach einem der vorhergehenden Ansprüche, wobei sowohl R⁵ als auch R⁷ Wasserstoff bedeuten.

9. Landwirtschaftliche Zusammensetzung, umfassend solch eine Menge an mindestens einer Verbindung der Formel I und/oder mindestens einem landwirtschaftlich nützlichen Satz von I, wie in einem der Ansprüche 1 bis 8 definiert, und mindestens einen inerten flüssigen und/oder festen agronomisch annehmbaren Träger und gegebenenfalls mindestens ein Tensid.

10. Verwendung einer Isothiazolopyridin-3-ylenamin-verbindung der Formel I oder eines ihrer Salze, wie in einem der Ansprüche 1 bis 8 definiert, für die Bekämpfung von tierischen Schädlinge.

11. Verfahren für die Bekämpfung von tierischen Schädlingen mit Ausnahme eines therapeutischen Verfahrens, bei dem man die tierischen Schädlinge, ihr/e/n Lebensraum, Brutstätte, Nahrungsgrundlage, Pflanze, Samen, Boden, Areal, Material oder Umwelt, worin die tierischen Schädlinge wachsen oder wachsen können, oder die Materialien, Pflanzen, Samen, Böden, Oberflächen oder Räume, die es gegenüber Befall oder Verseuchung mit Tieren zu schützen gilt, mit einer pestizidwirksamen Menge an mindestens einer Verbindung der Formel I und/oder mindestens einem ihrer landwirtschaftlich annehmbaren Salze, wie in einem der Ansprüche 1 bis 8 definiert, in Kontakt bringt.

12. Verfahren nach Anspruch 11, wobei der tierische Schädling aus der Ordnung Homoptera oder Thysanoptera stammt.

13. Verfahren zum Schützen von Kulturen gegen Befall oder Verseuchung mit tierischen Schädlingen, bei dem man eine Kultur mit einer pestizidwirksamen Menge an mindestens einer Verbindung der Formel I und/oder mindestens einem ihrer Salze, wie in einem der Ansprüche 1 bis 8 definiert, in Kontakt bringt.

14. Verfahren zum Schützen von Samen gegenüber Bodeninsekten und zum Schützen der Keimlingswurzeln und Sprosse gegenüber Insekten, wobei man die Samen vor dem Aussäen und/oder nach dem Vorkeimen mit einer Verbindung der Formel I, wie in einem der Ansprüche 1 bis 8 definiert, und/oder mindestens einem ihrer landwirtschaftlich annehmbaren Salze in pestizidwirksamen Mengen in Kontakt bringt.

15. Verfahren nach Anspruch 14, wobei die Verbindung der Formel I in einer Menge von 0,1 g bis 10 kg pro 100 kg Samen ausgebracht wird.

16. Verfahren nach Anspruch 14 oder 15, wobei die Wurzeln und Schoße der entstandenen Pflanze geschützt werden.

17. Verfahren nach Anspruch 14 oder 15, wobei die Schoße der entstandenen Pflanze gegen Blattläuse geschützt werden.

18. Samen, umfassend eine Verbindung der Formel I oder ein landwirtschaftlich nützliches Salz von I nach einem der Ansprüche 1 bis 8 in einer Menge von 0,1 g bis 10 kg pro 100 kg Samen.

## Revendications

1. Composé d'isothiasolopyridin-3-ylénamine de formule I 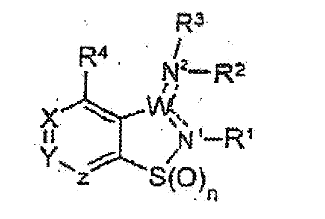 dans laquelle
n est 2 ;
X est N, N-O ou C-R⁵ ;
Y est N, N-O ou C-R⁶ ;
Z est C-R⁷ ;
à condition que l'une des variables X ou Y, au moins une et pas plus d'une, soit N ou NO ;
W est C, qui est soit lié à N¹ par une double liaison et à N² par une simple liaison soit lié à N² par une double liaison et à N¹ par une simple liaison si W est lié à N¹ par une simule liaison et à N² par une double liaison, alors N² ne porte que le substituant R² et pas le substituant R³ ; dans ce cas, R² est hydrogène, et R¹ est choisi dans le groupe constitué d'hydrogène, C(=O)-R⁶, C₁-C₁₀-alkyle, C₂-C₆-alcényle, C₂-C₁₀-alcynyle, C₁-C₁₀-alcoxy ou C₃-C₁₀-cycloalkyle, les cinq derniers radicaux mentionnés prouvant être non substitués, partiellement ou totalement halogénés et/ou pouvant porter 1, 2 ou 3 radicaux, indépendamment les uns des autres, choisis chacun dans le groupe constitué de cyano, nitro, amino, C₁-C₁₀-alcoxy, C₁-C₁₀-alkylthio, C₁-C₁₀-alkylsulfinyle, C₁-C₁₀-alkylsulfonyle, C₁-C₁₀-halogénoalcoxy, C₁-C₁₀-halogénoalkylthio, C₁-C₁₀-alcoxycarbonyle, (C₁-C₁₀-alkyl)amino, di-(C₁-C₁₀-alkyl)amino, C₃-C₁₀-cycloalkyle et phényle, le phényle pouvant être non substitué, partiellement ou totalement halogéné et/ou prouvant porter 1, 2 ou 3 substituants, indépendamment les uns des autres, choisis dans le groupe constitué de C₁-C₄-alkyle, C₁-C₄-halogénoalkyle, C₁-C₄-alcoxy et C₁-C₄-halogénoalcoxy ;
si W est lié à N² par une simple liaison et à N¹ par une double liaison, alors N¹ ne porte pas R¹ ; dans ce cas, R² et R³ sont, indépendamment l'un de l'autre, choisis dons le groupe constitué d'hydrogène, C(=O)-R⁶, C₁-C₁₀-alkyle, C₂-C₆-alcényle, C₂-C₁₀-alcynyle, C₁-C₁₀-alcoxy ou C₃-C₁₀-cycloalkyle, les cinq derniers radicaux mentionnés prouvant être non substitués, partiellement ou totalement halogénés et/ou pouvant porter 1, 2 ou 3 radicaux, indépendamment les uns des autres, choisais chacun dans le groupe constitué de cyano, nitro, amino, C₁-C₁₀-alcoxy, C₁-C₁₀-alkylthio, C₁-C₁₀-alkylsulfinyle, C₁-C₁₀-alkylsulfonyle, C₁-C₁₀-halogénoalcoxy, C₁-C₁₀-halogénoalkylthio, C₁-C₁₀-alcoxycarbonyle, (C₁-C₁₀-alkyl)amino, di-(C₁-C₁₀-alkyl)amino, C₃-C₁₀-cycloalkyle et phényle, le phényle pouvant être non substitué, partiellement ou totalement halogéné et/ou pouvant porter 1, 2 ou 3 substituants, indépendamment les uns des autres, choisis dans le groupe constitué de C₁-C₄-alkyle, C₁-C₄-halogénoalkyle, C₁-C₄-alcoxy et C₁-C₄-halogénoalcoxy ; ou
R² et R³ ensemble avec l'azote adjacent forment un cycle à 3 à 10 chaînons, éventuellement substitué par 1, 2 ou 3 radicaux C₁-C₅-alkyle, le cycle pouvant contenir, outre les membres azote et carbone du cycle, 1, 2 ou 3 hétéroatomes en tant que membres du cycle choisis dans le groupe constitué d'azote, d'oxygène, de soufre, d'un groupement SO, SO₂ ou N-R⁹ ;
R⁴ est hydrogène, nitro, cyano, azido, amino, halogène, sulfonylamino, sulfénylamino, sulfinylamino, C(=O)R⁹,
C₁-C₆-alkyle, C₂-C₆-alcényle, C₂-C₆-alcynyle, C₃-C₆-cycloalkyle, C₁-C₆-alcoxy, C₁-C₆-alkylthio, (C₁-C₆-alkyl)amino, di(C₁-C₆-alkyl)amino, C₁-C₆-alkylsulfinyle, C₁-C₆-alkylsulfényle ou C₁-C₆-alkylsulfonyle, les onze derniers radicaux mentionnés pouvant être non substitués, partiellement ou totalement halogénés et/ou pouvant porter 1, 2 ou 3 radicaux, choisis dans le groupe constitué de cyano, nitro, amino, C₁-C₄-alcoxy C₁-C₄-alkylthio, C₁-C₄-alkylsulfinyle, C₁-C₄-alkylsulfonyle, C₁-C₄-halogénoalcoxy, C₁-C₄-halogénoalkylthio, (C₁-C₄-alcoxy)carbonyle, (C₁-C₄-alkyl)amino, di(C₁-C₄-alkyl)amino, C₃-C₈-cycloalkyle et phényle, le phényle pouvant être non substitué, partiellement ou totalement halogéné et/ou porter 1, 2 ou 3 substituants, indépendamment les uns des autres choisis dans le groupe constitué de C₁-C₄-alkyle, C₁-C₄-halogénoalkyle, C₁-C₄-alcoxy et C₁-C₄-halogénoalcoxy;
R⁵, R⁶ et R⁷ sont indépendamment les uns des autres choisis dons le groupé constitué d'hydrogène, halogène, cyano, azido, nitro, C₁-C₆-alkyle, C₃-C₈-cycloalkyle, C₁-C₄-halogénoalkyle, C₁-C₄-alcoxy, C₁-C₄-alkylthio, C₁-C₄-alkylsulfinyle, C₁-C₄-alkylsulfonyle, C₁-C₄-halogénoalcoxy, C₁-C₄-halogénoalkylthio, C₂-C₆-alcényle; C₂-C₆-alcynyle, (C₁-C₄-alcoxy)carbonyle, amino, (C₁-C₄-alkyl)amino, di(C₁-C₄-alkyl)amino, aminocarbonyle, (C₁-C₄-alkyl)aminocarbonyle, di(C₁-C₄alkyl)aminocarbonyle, sulfonyle, sulfonylamino, sulfénylamino, sulfanylamino et C(=O)-R¹¹ ;
R⁸ est C₁-C₆-alkyle, aryle, aryl-C₁-C₄-alkyle, hétéroaryle ou hétéroaryl-C₁-C₄-alkyle à 3 à 7 chaînons, le cycle hétéroaryle contentant en tant que membres du cycle 1, 2 ou 3 hétéroatomes, choisis dans le groupe constitué d'azote, d'oxygène, de soufre, d'un groupement SO, SO₂ ou N-R¹², où R¹² est hydrogène ou C₁-C₄-alkyle ;
hétérocyclyle ou hétérocyclyl-C₁-C₄-alkyle à 3 à 7 chaînons, le cycle hétérocyclique contenant en tant que membres du cycle 1, 2 ou 3 hétéroatomes, choisis dans le groupe constitué d'azote, d'oxygène, de soufre, d'un groupement SO, SO₂ ou N-R¹³, où R¹³ est hydrogène ou C₁-C₄-alkyle ; et les atomes de carbone des cycles hétérocycliques pouvant être non substitués ou substitués par 1 ou 2 groupements C₁-C₄-alkyle ;
R⁹ est hydrogène ou C₁-C₄-alkyle
R¹⁰ et R¹¹, indépendamment l'un de l'autre, sont hydrogène, hydroxy, C₁-C₆-alcoxy, amino, C₁-C₄-alkyle, aryle, aryl-C₁-C₄-alkyle,
hétéroaryle ou hétéroaryl-C₁-C₄-alkyle à 3 à 7 chaînons, le cycle hétéroaryle contenant en tant que membres du cycle 1, 2 ou 3 hétéroatomes, choisis dans le groupe constitué d'azote, oxygène, de soufre, d'un groupement SO, SO₂ ou N-R¹³, où R¹³ est hydrogène ou C₁-C₄-alkyle ;
hétérocyclyle ou hétérocyclyl-C₁-C₄-alkyle à 3 à 7 chaînons, le cycle hétérocyclique contenant 1, 2 ou 3 hétéroatomes, choisis dans le groupe constitué d'azote, d'oxygène, de soufre, d'un groupement SO, SO₂ ou N-R¹⁴, où R¹⁴ est hydrogène ou C₁-C₄-alkyle ;
et les atomes de carbone des cycles hétérocycliques pouvant être non substitués ou substitués par 1 ou 2 groupements C₁-C₄-alkyle ;
et/ou les sels de celui-ci acceptables en agriculture.

2. Composé de formule I tel que défini dans la revendication 1, **caractérisé en ce que** X est C-R⁵ et Y est N ou NO.

3. Composé de formule I tel que défini dans la revendication 1, **caractérisé en ce que** X est C-R⁵ et Y est N.

4. Composé selon la revendication 1, 2 ou 3,
**caractérisé en ce que** soit R⁵ soit R⁷ est hydrogène et l'autre radical R⁵ ou R⁷ est choisi parmi halogène, C₁-C₄-alkyle ou C₁-C₄-halogénoalkyle.

5. Composé selon la revendication 1, 2 ou 3,
**caractérisé en ce que** soit R⁵ soit R⁷ est hydrogène et l'autre radical ou R⁷ est choisi parmi halogène, C₁-C₂-alkyle ou C₁-halogénoalkyle.

6. Composé selon la revendication 1, 2 ou 3,
**caractérisé en ce que** soit R⁵ soit R⁷ est hydrogène et l'autre radical R⁵ ou R⁷ est choisi parmi F, Cl, Br, I, CH₃ ou CF₃.

7. Composé selon l'une quelconque des revendications précédentes, **caractérisé en ce que** R⁴ est hydrogène.

8. Composé selon l'une quelconque des revendications précédentes, **caractérisé en ce que** R⁵ et R⁷ sont tous deux hydrogène.

9. Composition agricole comprenant une quantité d'au moins un composé de formule I et/ou d'au moins un sel de I utile en agriculture, tel que défini dans l'une quelconque des revendications 1 à 8, et au moins un support liquide et/ou solide inerte agronomiquement acceptable et, si on le souhaite, au moins un tensioactif.

10. Utilisation d'un composé d'isothiazolopyridin-3-ylénamine de formule I ou d'un sel de celui-ci tel que défini dans l'une quelconque des revendications 1 à 8, pour combattre les animaux nuisibles.

11. Méthode pour combattre les animaux nuisibles, à l'exclusion d'une méthode thérapeutique, **caractérisée en ce qu'**elle comprend la mise en contact desdits animaux nuisibles, leur habitat, lieu de reproduction, source d'alimentation, d'un(e) plainte, semence, sol, surface, matériau ou environnement dans lequel les animaux nuisibles se développent ou peuvent se développer, ou des matériaux, plantes, semences, sols, surfaces ou espaces à protéger contre une attaque ou une infestation par les animaux, avec une quantité efficace du point de vue pesticide d'au moins un composé de formule I et/ou d'au moins un sel de celui-ci acceptable en agriculture, tel que défini dans l'une quelconque des revendications 1 à 8.

12. Méthode telle que définie dans la revendication 11, **caractérisée en ce que** l'animal nuisible provient de l'ordre Homoptera ou Thysanoptera.

13. Méthode de protection de cultures contre une attaque ou une infestation par des animaux nuisibles, **caractérisée en ce qu'**elle comprend la mise en contact d'une culture avec une quantité efficace du point de vue pesticide d'au moins un composé de formule I et/ou d'au moins un sel de celui-ci, tel que défini dans l'une quelconque des revendications 1 à 8.

14. Méthode de protection des semences contre les insectes du sol, et des racines et des pousses des semis contre les insectes, comprenant la mise en contact des semences avant le semi et/ou après la prégermination avec un composé de formule I tel que défini dans l'une quelconque des revendications 1 à 8 et/ou au moins un sel acceptable en agriculture de celui-ci dans des quantités efficaces du point de vue pesticide.

15. Méthode selon la revendication 14, **caractérisée en ce que** le composé de formule I est appliqué dans une quantité de 0,1 g à 10 kg par 100 kg de semences.

16. Méthode selon la revendication 14 ou 15, **caractérisée en ce que** les racines et les pousses de la plante résultante sont protégées.

17. Méthode selon la revendication 14 ou 15, **caractérisée en ce que** les pousses de la plante résultante sont protégées contre les pucerons.

18. Semence comprenant un composé de formule I ou un sel utile en agriculture de I, tel que défini dans l'une quelconque des revendications 1 à 8, dans une quantité de 0,1 g à 10 kg par 100 kg de semences.
